# EUROPEAN PATENT APPLICATION

(11) **EP 4 023 642 A1**
(43) Date of publication of application: **06.07.2022**
(21) Application number: 20872098.7
(22) Date of filing: 21.09.2020
(51) Int. Cl.: C07D 239/48, C07D 239/47, C07D 239/38, A61K 31/505, A61P 1/16, A61P 35/00, A61P 3/00, C07D 401/04, C07D 403/12, C07D 401/12, C07D 405/02, C07D 405/14

(54) **JNK INHIBITOR, AND PHARMACEUTICAL COMPOSITION AND USE THEREOF**

(30) Priority: 30.09.2019 CN 201910942775
(71) Applicant: Wuhan LL Science and Technology Development Co., Ltd., Wuhan, Hubei 430075 (CN)
(72) Inventor: LI, Jinping, Wuhan, Hubei 430075 (CN); LOU, Jun, Wuhan, Hubei 430075 (CN); CHEN, Yongkai, Wuhan, Hubei 430075 (CN); GUO, Xiaodan, Wuhan, Hubei 430075 (CN); ZHANG, Yihan, Wuhan, Hubei 430075 (CN); ZENG, Xian, Wuhan, Hubei 430075 (CN); QIAN, Lina, Wuhan, Hubei 430075 (CN); WANG, Chaodong, Wuhan, Hubei 430075 (CN)
(74) Representative: Gille Hrabal Partnerschaftsgesellschaft mbB Patentanwälte
(86) International application number: PCT/CN2020/116617
(87) International publication number: WO 2021/063207

(57) **Abstract**

Provided are a compound as shown in formula (I), which formula is as follows, and racemates, stereoisomers, tautomers, isotopic markers, solvates, polymorphs and oxynitrides of the compound, or a pharmaceutically acceptable salt thereof, wherein same can be used as JNK inhibitors. Also provided are a method for preparing the compound shown in formula (I), a pharmaceutical composition comprising the compound shown in formula (I), and the use of the compound shown in formula (I) for preparing drugs, wherein the drugs are used for treating diseases which can be treated by inhibiting JNK activity.

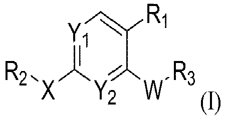

## Description

The present application claims priority to Chinese Patent Application No. 201910942775.3 filed with China National Intellectual Property Administration on Sep. 30, 2019 and entitled "JNK INHIBITOR, AND PHARMACEUTICAL COMPOSITION AND USE THEREOF", which is incorporated herein by reference in its entirety.

### TECHNICAL FIELD

The present disclosure relates to the field of medicines, and in particular to a compound capable of inhibiting JNK activity and a pharmaceutical composition and use thereof.

### BACKGROUND

Mitogen-activated protein kinase (MAPK) is one of the important pathways in the eukaryotic signaling network. MAPK chain consists of 3 types of protein kinases and is expressed as MAP3K-MAP2K-MAPK, and upstream signals are conveyed to downstream response molecules by successive phosphorylation of the protein kinases. There are three major MAPK signal transduction pathways were comprised in mammalian organisms, namely ERK (extracellular signal-regulated kinase) 1/2, JNK (c-Jun N-terminal kinase) and p38 signal pathways, wherein the ERK1/2 signal transduction pathway regulates cell growth and differentiation, and the JNK and p38 MAPK signal transduction pathways play an important role in stress reactions such as inflammation and apoptosis. JNK is an important member of the MAPK family, and it can be activated by stress signals and is therefore also called stress-activated protein kinase (SAPK). JNK is involved in a wide range of biological processes, including embryonic development, apoptosis/cell survival, transformation of oncogene expressing cells, angiogenesis, T cell activation, B cell proliferation, cytokine production and occurance of inflammation.

Currently, three distinct genes have been identified (JNK1, JNK2 and JNK3) and they encode 10 splice variants. JNK1 and JNK2 are expressed in a wide variety of tissues, while JNK3 is mainly expressed in neurons and, to a lesser extent, in the heart and testis. Members of the JNK family are activated by proinflammatory cytokines such as tumor necrosis factor α (TNF-α) and interleukin-1β (IL-1β) and environmental stress. Activation of JNK is mediated by its upstream kinases, MKK4 and MKK7, via dual phosphorylation of Thr-183 and Tyr-185. It has been shown that MKK4 and MKK7 can be activated by different upstream kinases, including MEKK1 and MEKK4, which depends on external stimulus and cellular environment. Specificity of JNK signaling is achieved by using scaffold proteins called JNK-interacting proteins to form JNK-specific signaling complexes that contain multiple components of the kinase cascade. JNK has been shown to play an important role in inflammation, T cell function, apoptosis and cell survival through phosphorylation of specific substrates, including transcription factors such as c-Jun, members of activator protein-1 (API) family, ATF2, and non-transcription factors such as IRS-1 and Bcl-2. Over-activation of JNK is considered an important mechanism in autoimmune, inflammation, metabolism, neurological diseases and cancer. Some studies have shown that the JNK signal transduction pathway is activated in pulmonary fibrosis and hepatic fibrosis and regulates inflammation, proliferation, differentiation, apoptosis, etc., of cells, and it can be induced by TGF- β1.

Idiopathic interstitial pulmonary fibrosis (IPF) is a chronic and diffuse pulmonary interstitial disease with unknown cause and its characteristic pathological change is common interstitial pneumonia, and the disease mainly shows manifestation of common interstitial pneumonia according to histopathology and imageology examinations. The disease condition progresses irreversibly due to the complex pathogenesis of the disease, and early diagnosis thereof is difficult; the survival rate of patients diagnosed with the disease is remarkably decreased over time, and the 3-year survival rate is 50%, and the 5-year survival rate is only 20%, which is lower than that of most cancers (such as leukemia, breast cancer, colon cancer, uterine tumor and renal cancer), and therefore the disease is called as "cancer that is not cancer".

Hepatic fibrosis is a shared step in the progression of all chronic liver diseases to cirrhosis. Previous related studies have been limited to avoiding fibrosis and delaying or even blocking the progression of fibrosis. In recent years, however, several experiments suggest that whether pathogenic factors are eliminated or effective anti-fibrosis drugs are applied, the hepatic fibrosis of patients and model animals can be partially or completely reversed, namely, the hepatic fibrosis is reversible to a certain extent. However, the reversal process of hepatic fibrosis is very complex, and many signaling pathways, such as the known MAPK signaling pathways like MAPK/EKR, SAPK/JNK and MEK5, and non-MAPK signaling pathways like PI3K/Akt and Notch, play important roles in this process.

Currently, no drugs that treat numerous disorders including fibrosis through inhibition of JNK are available on the market. Therefore, the development of new JNK inhibitor compounds is of great significance for treating the above-mentioned diseases.

### SUMMARY

In order to solve the problems in the prior art, the present disclosure provides the following technical solution:
a compound of formula (I) and a racemate, a stereoisomer, a tautomer, an isotopically labeled compound, a solvate, a polymorph and a nitrogen oxide thereof, or pharmaceutically acceptable salts thereof: 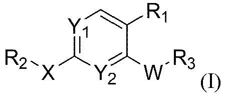 wherein,
Y₁ and Y₂ are independently CR₄ or N;
X and W are independently selected from CR₅R₆, O and NR₇;
R₁ is selected from H, hydroxy, halogen, cyano, nitro, NR₁₁R₁₂, C(O)NR₁₁R₁₂, C(=S)NR₁₁R₁₂, S(0)₂NR₁₁R₁₂, C(=NR₁₃)NR₁₁R₁₂, NHC(O)NR₁₁R₁₂, P(O)₂NR₁₁R₁₂, P(O)R₁₃NR₁₁R₁₂, C(O)R₁₄, NHC(O)R₁₄, C(O)OR₁₅, and the following groups unsubstituted or optionally substituted with one, two or more Rₐ: C₁₋₄₀ alkyl, C₂₋₄₀ alkenyl, C₂₋₄₀ alkynyl, C₁₋₄₀ alkoxy, C₃₋₂₀ cycloalkyl, 3-20 membered heterocyclyl, C₆₋₂₀ aryl and 5-20 membered heteroaryl;
R₂ is selected from H and the following groups unsubstituted or optionally substituted with one, two or more R_{b}: C₁₋₄₀ alkyl, C₂₋₄₀ alkenyl, C₂₋₄₀ alkynyl, C₁₋₄₀ alkoxy, C₃₋₂₀ cycloalkyl, 3-20 membered heterocyclyl, C₆₋₂₀ aryl, C₆₋₂₀ aryl-C₁₋₄₀ alkyl, 5-20 membered heteroaryl and 5-20 membered heteroaryl-C₁₋₄₀ alkyl; or
R₂, together with X, forms the following groups unsubstituted or optionally substituted with one, two or more R_{b}: NHC(O)NR₁₁R₁₂, C₃₋₂₀ cycloalkyl, 3-20 membered heterocyclyl, C₆₋₂₀ aryl and 5-20 membered heteroaryl;
R₃ is selected from H and the following groups unsubstituted or optionally substituted with one, two or more R_{c}: NR₁₁R₁₂, C(O)NR₁₁R₁₂, C(O)R₁₄, C₁₋₄₀ alkyl, C₂₋₄₀ alkenyl, C₂₋₄₀ alkynyl, C₁₋₄₀ alkoxy, C₃₋₂₀ cycloalkyl, 3-20 membered heterocyclyl, C₆₋₂₀ aryl and 5-20 membered heteroaryl; or
R₃, together with W, forms the following groups unsubstituted or optionally substituted with one, two or more R_{c}: NHC(0)NR₁₁R₁₂, C₃₋₂₀ cycloalkyl, 3-20 membered heterocyclyl, C₆₋₂₀ aryl and 5-20 membered heteroaryl;
R₄ is selected from H, hydroxy, halogen, cyano, nitro, amino, and the following groups unsubstituted or optionally substituted with one, two or more R_{d}: C₁₋₄₀ alkyl, C₂₋₄₀ alkenyl, C₂₋₄₀ alkynyl and C₁₋₄₀ alkoxy;
R₅ and R₆ are each independently selected from H, hydroxy, halogen, cyano, and the following groups unsubstituted or optionally substituted with one, two or more R_{d}: C₁₋₄₀ alkyl, C₂₋₄₀ alkenyl, C₂₋₄₀ alkynyl and C₁₋₄₀ alkoxy, with the proviso that R₅ and R₆ are not both hydroxy, cyano or C₁₋₄₀ alkoxy simultaneously; or, R₅ and R₆, together with the carbon atom attached thereto, form carbonyl, i.e., CR₅R₆ is C(=O);
R₇ is selected from H and the following groups unsubstituted or optionally substituted with one, two or more Rₑ: C₁₋₄₀ alkyl, C₂₋₄₀ alkenyl, C₂₋₄₀ alkynyl, C₁₋₄₀ alkoxy, C₃₋₂₀ cycloalkyl, 3-20 membered heterocyclyl, C₆₋₂₀ aryl and 5-20 membered heteroaryl;
R₁₁ and R₁₂ are each independently selected from H and the following groups unsubstituted or optionally substituted with one, two or more Rₑ: C(O)R₁₄, C₁₋₄₀ alkyl, C₂₋₄₀ alkenyl, C₂₋₄₀ alkynyl, C₁₋₄₀ alkoxy, C₃₋₂₀ cycloalkyl, 3-20 membered heterocyclyl, C₆₋₂₀ aryl and 5-20 membered heteroaryl; or
R₁₁ and R₁₂, together with the nitrogen atom attached thereto, form the following groups unsubstituted or optionally substituted with one, two or more Rₑ: 3-20 membered heterocyclyl and 5-20 membered heteroaryl;
R₁₃ is selected from H, hydroxy, cyano, and the following groups unsubstituted or optionally substituted with one, two or more Rₑ: C₁₋₄₀ alkyl and C₁₋₄₀ alkoxy;
R₁₄ is selected from H, hydroxy, halogen, and the following groups unsubstituted or optionally substituted with one, two or more R_{f}: C₁₋₄₀ alkyl, C₂₋₄₀ alkenyl, C₂₋₄₀ alkynyl, C₁₋₄₀ alkoxy, C₃₋₂₀ cycloalkyl, 3-20 membered heterocyclyl, C₆₋₂₀ aryl and 5-20 membered heteroaryl;
R₁₅ is selected from H and the following groups unsubstituted or optionally substituted with one, two or more R_{f}: C₁₋₄₀ alkyl, C₂₋₄₀ alkenyl, C₂₋₄₀ alkynyl, C₃₋₂₀ cycloalkyl, 3-20 membered heterocyclyl, C₆₋₂₀ aryl and 5-20 membered heteroaryl;
each Rₐ is independently selected from CN, halogen, OH, NH₂, oxo, and the following groups unsubstituted or optionally substituted with one, two or more R_{f}: C₁₋₄₀ alkyl and C₁₋₄₀ alkoxy;
each R_{b} is independently selected from CN, halogen, OH, NH₂, COOH, NO₂, oxo, S(O)₂CH₃, C(O)NHCH₂CH₃, and the following groups unsubstituted or optionally substituted with one, two or more R_{f}: C₁₋₄₀ alkyl, C₁₋₄₀ alkoxy, C₃₋₂₀ cycloalkyl, 3-20 membered heterocyclyl, C₆₋₂₀ aryl and 5-20 membered heteroaryl;
each R_{c} is independently selected from CN, halogen, OH, NH₂, COOH, NO₂, C(O)NH₂, C(O)NHOH, C(O)N(OH)CH₃, oxo, C(O)CH₂COOH, C(O)CH₂CN, C(O)CH₂Cl, C(O)CH₂F, C(O)CH₂Br, and the following groups unsubstituted or optionally substituted with one, two or more R_{f}: C₁₋₄₀ alkyl, C₁₋₄₀ alkoxy, C₃₋₂₀ cycloalkyl, 3-20 membered heterocyclyl, C₆₋₂₀ aryl and 5-20 membered heteroaryl;
R_{d}, Rₑ and R_{f} are each the same or different, and are each independently selected from CN, halogen, OH, NH₂, oxo, S(O)₂CH₃, C₁₋₄₀ alkyl, C₁₋₄₀ haloalkyl, C₁₋₄₀ alkoxy and C₁₋₄₀ haloalkoxy.

According to an embodiment of the present disclosure, in the compound of formula (I), 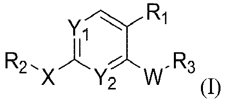 wherein,
Y₁ and Y₂ are independently CR₄ or N;
X and W are independently selected from CR₅R₆, O and NR₇;
R₁ is selected from H, hydroxy, halogen, cyano, nitro, NR₁₁R₁₂, C(O)NR₁₁R₁₂, C(=S)NR₁₁R₁₂, S(0)₂NR₁₁R₁₂, C(=NR₁₃)NR₁₁R₁₂, NHC(O)NR₁₁R₁₂, P(O)₂NR₁₁R₁₂, P(O)R₁₃NR₁₁R₁₂, C(O)R₁₄, NHC(O)R₁₄, C(O)OR₁₅, and the following groups unsubstituted or optionally substituted with one, two or more Rₐ: C₁₋₁₀ alkyl, C₂₋₁₀ alkenyl, C₂₋₁₀ alkynyl, C₁₋₁₀ alkoxy, C₃₋₁₀ cycloalkyl, 3-10 membered heterocyclyl, C₆₋₁₄ aryl and 5-14 membered heteroaryl;
R₂ is selected from H and the following groups unsubstituted or optionally substituted with one, two or more R_{b}: C₁₋₁₀ alkyl, C₂₋₁₀ alkenyl, C₂₋₁₀ alkynyl, C₁₋₁₀ alkoxy, C₃₋₁₀ cycloalkyl, 3-10 membered heterocyclyl, C₆₋₁₄ aryl, C₆₋₁₄ aryl-Ci-io alkyl, 5-14 membered heteroaryl and 5-14 membered heteroaryl-Ci-io alkyl; or
R₂, together with X, forms the following groups unsubstituted or optionally substituted with one, two or more R_{b}: NHC(0)NR₁₁R₁₂, C₃₋₁₀ cycloalkyl, 3-10 membered heterocyclyl, C₆₋₁₄ aryl and 5-14 membered heteroaryl;
R₃ is selected from H and the following groups unsubstituted or optionally substituted with one, two or more R_{c}: NR₁₁R₁₂, C(O)NR₁₁R₁₂, C(O)R₁₄, C₁₋₁₀ alkyl, C₂₋₁₀ alkenyl, C₂₋₁₀ alkynyl, C₁₋₁₀ alkoxy, C₃₋₁₀ cycloalkyl, 3-10 membered heterocyclyl, C₆₋₁₄ aryl and 5-14 membered heteroaryl; or
R₃, together with W, forms the following groups unsubstituted or optionally substituted with one, two or more R_{c}: NHC(O)NR₁₁R₁₂, C₃₋₁₀ cycloalkyl, 3-10 membered heterocyclyl, C₆₋₁₄ aryl and 5-14 membered heteroaryl;
R₄ is selected from H, hydroxy, halogen, cyano, nitro, amino, and the following groups unsubstituted or optionally substituted with one, two or more R_{d}: C₁₋₁₀ alkyl, C₂₋₁₀ alkenyl, C₂₋₁₀ alkynyl and C₁₋₁₀ alkoxy;
R₅ and R₆ are each independently selected from H, hydroxy, halogen, cyano, and the following groups unsubstituted or optionally substituted with one, two or more R_{d}: C₁₋₁₀ alkyl, C₂₋₁₀ alkenyl, C₂₋₁₀ alkynyl and C₁₋₁₀ alkoxy, with the proviso that R₅ and R₆ are not both hydroxy, cyano or C₁₋₁₀ alkoxy simultaneously; or, R₅ and R₆, together with the carbon atom attached thereto, form carbonyl, i.e., CR₅R₆ is C(=O);
R₇ is selected from H and the following groups unsubstituted or optionally substituted with one, two or more Rₑ: C₁₋₁₀ alkyl, C₂₋₁₀ alkenyl, C₂₋₁₀ alkynyl, C₁₋₁₀ alkoxy, C₃₋₁₀ cycloalkyl, 3-10 membered heterocyclyl, C₆₋₁₄ aryl and 5-14 membered heteroaryl;
R₁₁ and R₁₂ are each independently selected from H and the following groups unsubstituted or optionally substituted with one, two or more Rₑ: C(O)R₁₄, C₁₋₁₀ alkyl, C₂₋₁₀ alkenyl, C₂₋₁₀ alkynyl, C₁₋₁₀ alkoxy, C₃₋₁₀ cycloalkyl, 3-10 membered heterocyclyl, C₆₋₁₄ aryl and 5-14 membered heteroaryl; or
R₁₁ and R₁₂, together with the nitrogen atom attached thereto, form the following groups unsubstituted or optionally substituted with one, two or more Rₑ: 3-10 membered heterocyclyl and 5-14 membered heteroaryl;
R₁₃ is selected from H, hydroxy, cyano, and the following groups unsubstituted or optionally substituted with one, two or more Rₑ: C₁₋₁₀ alkyl and C₁₋₁₀ alkoxy;
R₁₄ is selected from H, hydroxy, halogen, and the following groups unsubstituted or optionally substituted with one, two or more R_{f}: C₁₋₁₀ alkyl, C₂₋₁₀ alkenyl, C₂₋₁₀ alkynyl, C₁₋₁₀ alkoxy, C₃₋₁₀ cycloalkyl, 3-10 membered heterocyclyl, C₆₋₁₄ aryl and 5-14 membered heteroaryl;
R₁₅ is selected from H and the following groups unsubstituted or optionally substituted with one, two or more R_{f}: C₁₋₁₀ alkyl, C₂₋₁₀ alkenyl, C₂₋₁₀ alkynyl, C₃₋₁₀ cycloalkyl, 3-10 membered heterocyclyl, C₆₋₁₄ aryl and 5-14 membered heteroaryl;
each Rₐ is independently selected from CN, halogen, OH, NH₂, oxo, and the following groups unsubstituted or optionally substituted with one, two or more R_{f}: C₁₋₁₀ alkyl and C₁₋₁₀ alkoxy;
each R_{b} is independently selected from CN, halogen, OH, NH₂, COOH, NO₂, oxo, S(O)₂CH₃, C(O)NHCH₂CH₃, and the following groups unsubstituted or optionally substituted with one, two or more R_{f}: C₁₋₁₀ alkyl, C₁₋₁₀ alkoxy, C₃₋₁₀ cycloalkyl, 3-10 membered heterocyclyl, C₆₋₁₄ aryl and 5-14 membered heteroaryl;
each R_{c} is independently selected from CN, halogen, OH, NH₂, COOH, NO₂, C(O)NH₂, C(O)NHOH, C(O)N(OH)CH₃, oxo, C(O)CH₂COOH, C(O)CH₂CN, C(O)CH₂Cl, C(O)CH₂F, C(O)CH₂Br, and the following groups unsubstituted or optionally substituted with one, two or more R_{f}: C₁₋₁₀ alkyl, C₁₋₁₀ alkoxy, C₃₋₁₀ cycloalkyl, 3-10 membered heterocyclyl, C₆₋₁₄ aryl and 5-14 membered heteroaryl;
R_{d}, Rₑ and R_{f} are each the same or different, and are each independently selected from CN, halogen, OH, NH₂, oxo, S(O)₂CH₃, C₁₋₁₀ alkyl, C₁₋₁₀ haloalkyl, C₁₋₁₀ alkoxy and C₁₋₁₀ haloalkoxy.

According to an embodiment of the present disclosure, in the compound of formula (I), 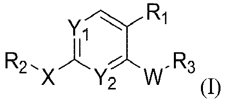 wherein,
Y₁ and Y₂ are independently CR₄ or N;
X and W are independently selected from CR₅R₆, O and NR₇;
R₁ is selected from H, hydroxy, halogen, cyano, nitro, NR₁₁R₁₂, C(O)NR₁₁R₁₂, C(=S)NR₁₁R₁₂, S(O)₂NR₁₁R₁₂, C(=NR₁₃)NR₁₁R₁₂, NHC(O)NR₁₁R₁₂, P(O)₂NR₁₁R₁₂, P(O)R₁₃NR₁₁R₁₂, C(O)R₁₄, NHC(O)R₁₄, C(O)OR₁₅, and the following groups unsubstituted or optionally substituted with one, two or more Rₐ: C₁₋₆ alkyl, C₂₋₆ alkenyl, C₂₋₆ alkynyl, C₁₋₆ alkoxy, C₃₋₁₀ cycloalkyl, 3-10 membered heterocyclyl, C₆₋₁₄ aryl and 5-14 membered heteroaryl;
R₂ is selected from H and the following groups unsubstituted or optionally substituted with one, two or more R_{b}: C₁₋₆ alkyl, C₂₋₆ alkenyl, C₂₋₆ alkynyl, C₁₋₆ alkoxy, C₃₋₁₀ cycloalkyl, 3-10 membered heterocyclyl, C₆₋₁₄ aryl, C₆₋₁₄ aryl-C₁₋₆ alkyl, 5-14 membered heteroaryl and 5-14 membered heteroaryl-C₁₋₆ alkyl; or
R₂, together with X, forms the following groups unsubstituted or optionally substituted with one, two or more R_{b}: NHC(O)NR₁₁R₁₂, C₃₋₁₀ cycloalkyl, 3-10 membered heterocyclyl, C₆₋₁₄ aryl and 5-14 membered heteroaryl;
R₃ is selected from H and the following groups unsubstituted or optionally substituted with one, two or more R_{c}: NR₁₁R₁₂, C(O)NR₁₁R₁₂, C(O)R₁₄, C₁₋₆ alkyl, C₂₋₆ alkenyl, C₂₋₆ alkynyl, C₁₋₆ alkoxy, C₃₋₁₀ cycloalkyl, 3-10 membered heterocyclyl, C₆₋₁₄ aryl and 5-14 membered heteroaryl; or
R₃, together with W, forms the following groups unsubstituted or optionally substituted with one, two or more R_{c}: NHC(O)NR₁₁R₁₂, C₃₋₁₀ cycloalkyl, 3-10 membered heterocyclyl, C₆₋₁₄ aryl and 5-14 membered heteroaryl;
R₄ is selected from H, hydroxy, halogen, cyano, nitro, amino, and the following groups unsubstituted or optionally substituted with one, two or more R_{d}: C₁₋₆ alkyl, C₂₋₆ alkenyl, C₂₋₆ alkynyl and C₁₋₆ alkoxy;
R₅ and R₆ are each independently selected from H, hydroxy, halogen, cyano, and the following groups unsubstituted or optionally substituted with one, two or more R_{d}: C₁₋₆ alkyl, C₂₋₆ alkenyl, C₂₋₆ alkynyl and C₁₋₆ alkoxy, with the proviso that R₅ and R₆ are not both hydroxy, cyano or C₁₋₆ alkoxy simultaneously; or, R₅ and R₆, together with the carbon atom attached thereto, form carbonyl, i.e., CR₅R₆ is C(=O);
R₇ is selected from H and the following groups unsubstituted or optionally substituted with one, two or more Rₑ: C₁₋₆ alkyl, C₂₋₆ alkenyl, C₂₋₆ alkynyl, C₁₋₆ alkoxy, C₃₋₁₀ cycloalkyl, 3-10 membered heterocyclyl, C₆₋₁₄ aryl and 5-14 membered heteroaryl;
R₁₁ and R₁₂ are each independently selected from H and the following groups unsubstituted or optionally substituted with one, two or more Rₑ: C(O)R₁₄, C₁₋₆ alkyl, C₂₋₆ alkenyl, C₂₋₆ alkynyl, C₁₋₆ alkoxy, C₃₋₁₀ cycloalkyl, 3-10 membered heterocyclyl, C₆₋₁₄ aryl and 5-14 membered heteroaryl; or R₁₁ and R₁₂, together with the nitrogen atom attached thereto, form the following groups unsubstituted or optionally substituted with one, two or more Rₑ: 3-10 membered heterocyclyl and 5-14 membered heteroaryl;
R₁₃ is selected from H, hydroxy, cyano, and the following groups unsubstituted or optionally substituted with one, two or more Rₑ: C₁₋₆ alkyl and C₁₋₆ alkoxy;
R₁₄ is selected from H, hydroxy, halogen, and the following groups unsubstituted or optionally substituted with one, two or more R_{f}: C₁₋₆ alkyl, C₂₋₆ alkenyl, C₂₋₆ alkynyl, C₁₋₆ alkoxy, C₃₋₁₀ cycloalkyl, 3-10 membered heterocyclyl, C₆₋₁₄ aryl and 5-14 membered heteroaryl;
R₁₅ is selected from H and the following groups unsubstituted or optionally substituted with one, two or more R_{f}: C₁₋₆ alkyl, C₂₋₆ alkenyl, C₂₋₆ alkynyl, C₃₋₁₀ cycloalkyl, 3-10 membered heterocyclyl, C₆₋₁₄ aryl and 5-14 membered heteroaryl;
each Rₐ is independently selected from CN, halogen, OH, NH₂, oxo, and the following groups unsubstituted or optionally substituted with one, two or more R_{f}: C₁₋₆ alkyl and C₁₋₆ alkoxy;
each R_{b} is independently selected from CN, halogen, OH, NH₂, COOH, NO₂, oxo, S(O)₂CH₃, C(O)NHCH₂CH₃, and the following groups unsubstituted or optionally substituted with one, two or more R_{f}: C₁₋₆ alkyl, C₁₋₆ alkoxy, C₃₋₁₀ cycloalkyl, 3-10 membered heterocyclyl, C₆₋₁₄ aryl and 5-14 membered heteroaryl;
each R_{c} is independently selected from CN, halogen, OH, NH₂, COOH, NO₂, C(O)NH₂, C(O)NHOH, C(O)N(OH)CH₃, oxo, C(O)CH₂COOH, C(O)CH₂CN, C(O)CH₂Cl, C(O)CH₂F, C(O)CH₂Br, and the following groups unsubstituted or optionally substituted with one, two or more R_{f}: C₁₋₆ alkyl, C₁₋₆ alkoxy, C₃₋₁₀ cycloalkyl, 3-10 membered heterocyclyl, C₆₋₁₄ aryl and 5-14 membered heteroaryl;
R_{d}, Rₑ and R_{f} are each the same or different, and are each independently selected from CN, halogen, OH, NH₂, oxo, S(O)₂CH₃, C₁₋₆ alkyl, C₁₋₆ haloalkyl, C₁₋₆ alkoxy and C₁₋₆ haloalkoxy.

According to an embodiment of the present disclosure, in the compound of formula (I), 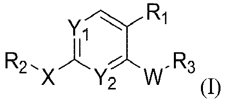 wherein,
Y₁ and Y₂ are independently CR₄ or N;
X and W are independently O or NR₇;
R₁ is selected from H, hydroxy, halogen, cyano, nitro, NR₁₁R₁₂, C(O)NR₁₁R₁₂, C(=S)NR₁₁R₁₂, S(O)₂NR₁₁R₁₂, C(=NR₁₃)NR₁₁R₁₂, NHC(O)NR₁₁R₁₂, P(O)₂NR₁₁R₁₂, P(O)R₁₃NR₁₁R₁₂, C(O)R₁₄, NHC(O)R₁₄, C(O)OR₁₅, and the following groups unsubstituted or optionally substituted with one, two or more Rₐ: C₁₋₆ alkyl, C₂₋₆ alkenyl, C₂₋₆ alkynyl, C₁₋₆ alkoxy, C₃₋₆ cycloalkyl, 3-7 membered heterocyclyl and 5-6 membered heteroaryl;
R₂ is selected from H and the following groups unsubstituted or optionally substituted with one, two or more R_{b}: C₁₋₆ alkyl, C₂₋₆ alkenyl, C₂₋₆ alkynyl, C₁₋₆ alkoxy, C₃₋₆ cycloalkyl, 3-7 membered heterocyclyl, phenyl, benzyl, 5-6 membered heteroaryl and 5-6 membered heteroaryl-C₁₋₆ alkyl; or R₂, together with X, forms the following groups unsubstituted or optionally substituted with one, two or more R_{b}: NHC(O)NR₁₁R₁₂, 3-7 membered heterocyclyl and 5-6 membered heteroaryl;
R₃ is selected from H and the following groups unsubstituted or optionally substituted with one, two or more R_{c}: NR₁₁R₁₂, C(O)NR₁₁R₁₂, C(O)R₁₄, C₁₋₆ alkyl, C₃₋₆ cycloalkyl, 3-7 membered heterocyclyl, phenyl and 5-6 membered heteroaryl; or
R₃, together with W, forms the following groups unsubstituted or optionally substituted with one, two or more R_{c}: NHC(O)NR₁₁R₁₂, 3-7 membered heterocyclyl and 5-6 membered heteroaryl;
R₄ is selected from H, hydroxy and halogen;
R₇ is H;
R₁₁ and R₁₂ are each independently selected from H and the following groups unsubstituted or optionally substituted with one, two or more Rₑ: C₁₋₆ alkyl, C₂₋₆ alkenyl and C₂₋₆ alkynyl; or
R₁₁ and R₁₂, together with the nitrogen atom attached thereto, form the following groups unsubstituted or optionally substituted with one, two or more Rₑ: 3-7 membered heterocyclyl and 5-6 membered heteroaryl;
R₁₃ is selected from H, cyano, methyl and hydroxy;
R₁₄ is selected from H, hydroxy, halogen, and the following groups unsubstituted or optionally substituted with one, two or more R_{f}: C₁₋₆ alkyl, C₂₋₆ alkenyl, C₂₋₆ alkynyl. C₁₋₆ alkoxy and C₃₋₆ cycloalkyl;
R₁₅ is selected from H and C₁₋₆ alkyl unsubstituted or optionally substituted with one, two or more R_{f};
each Rₐ is independently selected from CN, halogen, OH, NH₂, oxo, and the following groups unsubstituted or optionally substituted with one, two or more R_{f}: C₁₋₆ alkyl and C₁₋₆ alkoxy;
each R_{b} is independently selected from CN, halogen, OH, NH₂, COOH, NO₂, oxo, S(O)₂CH₃, C(O)NHCH₂CH₃, and the following groups unsubstituted or optionally substituted with one, two or more R_{f}: C₁₋₆ alkyl, C₁₋₆ alkoxy, C₃₋₆ cycloalkyl, 3-7 membered heterocyclyl, phenyl and 5-6 membered heteroaryl;
each R_{c} is independently selected from CN, halogen, OH, NH₂, COOH, NO₂, C(O)NH₂, C(O)NHOH, C(O)N(OH)CH₃, oxo, C(O)CH₂COOH, C(O)CH₂CN, C(O)CH₂Cl, C(O)CH₂F, C(O)CH₂Br, and the following groups unsubstituted or optionally substituted with one, two or more R_{f}: C₁₋₆ alkyl, C₁₋₆ alkoxy, 3-7 membered heterocyclyl and 5-6 membered heteroaryl;
Rₑ and R_{f} are each the same or different, and are each independently selected from CN, halogen, OH, NH₂, oxo, S(O)₂CH₃, C₁₋₆ alkyl, C₁₋₆ haloalkyl, C₁₋₆ alkoxy and C₁₋₆ haloalkoxy.

According to an embodiment of the present disclosure, in the compound of formula (I), 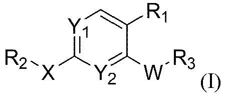 wherein,
Y₁ and Y₂ are independently CR₄ or N;
X and W are independently O or NR₇;
R₁ is selected from H, hydroxy, halogen, cyano, nitro, NR₁₁R₁₂, C(O)NR₁₁R₁₂, C(=S)NR₁₁R₁₂, S(O)₂NR₁₁R₁₂, C(=NR₁₃)NR₁₁R₁₂, NHC(O)NR₁₁R₁₂, P(O)₂NR₁₁R₁₂, P(O)R₁₃NR₁₁R₁₂, C(O)R₁₄, NHC(O)R₁₄, C(O)OR₁₅, and the following groups unsubstituted or optionally substituted with one, two or more Rₐ: C₁₋₆ alkyl, C₂₋₆ alkenyl, C₂₋₆ alkynyl, C₁₋₆ alkoxy, oxetanyl, azetidinyl, imidazolyl, pyrazolyl, triazolyl, oxadiazolyl, isoxadiazolyl, thiadiazolyl, isothiadiazolyl, tetrazolyl, pyrrolyl, furanyl, thiazolyl, isothiazolyl, oxazolyl, isoxazolyl, pyridinyl, pyrimidinyl, pyridazinyl, pyrazinyl, pyranyl and thienyl;
R₂ is selected from H and the following groups unsubstituted or optionally substituted with one, two or more R_{b}: C₁₋₆ alkyl, C₂₋₆ alkenyl, C₂₋₆ alkynyl, C₁₋₆ alkoxy, C₃₋₆ cycloalkyl, oxetanyl, piperidinyl, piperazinyl, morpholinyl, pyrrolidinyl, phenyl, benzyl, imidazolyl, pyrazolyl, triazolyl, oxadiazolyl, isoxadiazolyl, thiadiazolyl, isothiadiazolyl, tetrazolyl, pyrrolyl, furanyl, thiazolyl, isothiazolyl, oxazolyl, isoxazolyl, pyridinyl, pyrimidinyl, pyridazinyl, pyrazinyl, pyranyl, thienyl and 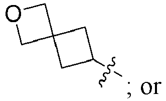
R₂, together with X, forms the following groups unsubstituted or optionally substituted with one, two or more R_{b}: NHC(O)NR₁₁R₁₂, azetidinyl, oxetanyl, piperidinyl, piperazinyl, morpholinyl, pyrrolidinyl, imidazolidinyl, pyrrolinyl, pyranyl, imidazolyl, pyrazolyl, triazolyl, oxadiazolyl, isoxadiazolyl, thiadiazolyl, isothiadiazolyl, tetrazolyl, pyrrolyl, furanyl, thiazolyl, isothiazolyl, oxazolyl, isoxazolyl, pyridinyl, pyrimidinyl, pyridazinyl and pyrazinyl;
R₃ is selected from H and the following groups unsubstituted or optionally substituted with one, two or more R_{c}: C(O)NR₁₁R₁₂, C(O)R₁₄, C₁₋₆ alkyl, cyclopentyl, cyclohexyl, piperidinyl, pyrrolidinyl, phenyl, imidazolyl, pyrazolyl, triazolyl, pyrrolyl, furanyl, piperazinyl, morpholinyl, pyranyl, oxadiazolyl, isoxadiazolyl, thiadiazolyl, isothiadiazolyl, tetrazolyl, isothiazolyl, oxazolyl, isoxazolyl, pyridinyl, pyrimidinyl, pyridazinyl, pyrazinyl, thiazolyl and thienyl; or
R₃, together with W, forms the following groups unsubstituted or optionally substituted with one, two or more R_{c}: NHC(O)NR₁₁R₁₂, piperidinyl, piperazinyl, morpholinyl, pyrrolidinyl, imidazolidinyl, pyrrolinyl, azetidinyl, oxetanyl, pyranyl, imidazolyl, pyrazolyl, triazolyl, oxadiazolyl, isoxadiazolyl, thiadiazolyl, isothiadiazolyl, tetrazolyl, pyrrolyl, furanyl, thiazolyl, isothiazolyl, oxazolyl, isoxazolyl, pyridinyl, pyrimidinyl, pyridazinyl and pyrazinyl;
R₄ is selected from H, hydroxy and halogen;
R₇ is H;
R₁₁ and R₁₂ are each independently selected from H and C₁₋₆ alkyl unsubstituted or optionally substituted with one, two or more Rₑ; or
R₁₁ and R₁₂, together with the nitrogen atom attached thereto, form 3-7 membered azacycloalkyl unsubstituted or optionally substituted with one, two or more Rₑ;
R₁₃ is selected from H, cyano, methyl and hydroxy;
R₁₄ is selected from H, hydroxy, halogen, and the following groups unsubstituted or optionally substituted with one, two or more R_{f}: C₁₋₆ alkyl, C₂₋₆ alkenyl, C₂₋₆ alkynyl, C₁₋₆ alkoxy and C₃₋₆ cycloalkyl;
R₁₅ is selected from H and C₁₋₆ alkyl unsubstituted or optionally substituted with one, two or more R_{f};
each Rₐ is independently selected from CN, halogen, OH, NH₂, oxo, C₁₋₆ alkyl, C₁₋₆ haloalkyl, C₁₋₆ alkoxy, C₁₋₆ haloalkoxy and CH₂CH₂S(O)₂CH₃;
each R_{b} is independently selected from CN, halogen, OH, NH₂, COOH, NO₂, oxo, S(O)₂CH₃, C(O)NHCH₂CH₃, and the following groups unsubstituted or optionally substituted with one, two or more R_{f}: C₁₋₆ alkyl, C₁₋₆ alkoxy, phenyl and piperidinyl;
each R_{c} is independently selected from CN, halogen, OH, NH₂, COOH, NO₂, C(O)NH₂, C(O)NHOH, C(O)N(OH)CH₃, oxo, C(O)CH₂COOH, C(O)CH₂CN, C(O)CH₂Cl, C(O)CH₂F, C(O)CH₂Br, and the following groups unsubstituted or optionally substituted with one, two or more R_{f}: C₁₋₆ alkyl, C₁₋₆ alkoxy, 5 membered heterocyclyl and 5 membered heteroaryl;
Rₑ and R_{f} are each the same or different, and are each independently selected from CN, halogen, OH, NH₂, oxo, S(O)₂CH₃, C₁₋₆ alkyl, C₁₋₆ haloalkyl, C₁₋₆ haloalkoxy and C₁₋₆ alkoxy.

According to an embodiment of the present disclosure, in the compound of formula (I), 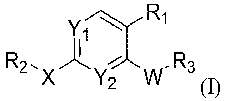 wherein,
Y₁ and Y₂ are both N, or Y₁ is N and Y₂ is CH;
X and W are both NH, or X is O and W is NH;
R₁ is selected from H, hydroxy, halogen, cyano, nitro, NH₂, C(O)NH₂, C(=S)NH₂, C(=NH)NH₂, C(=NOH)NH₂, C(=NCN)NH₂, C(=NCH₃)NH₂, S(O)₂NH₂, NHC(O)NH₂, NHC(O)CH₃, methyl, monofluoromethyl, difluoromethyl, trifluoromethyl, methoxy, hydroxypropyl, ethynyl, propynyl, vinyl, allyl, methylcarbonyl, cyclopropylcarbonyl, ethylcarbonyl, isopropylcarbonyl, methoxycarbonyl, monofluoromethylcarbonyl, difluoromethylcarbonyl, trifluoromethylcarbonyl, imidazolyl, pyrazolyl, triazolyl, oxadiazolyl, oxazolyl, isoxazolyl, 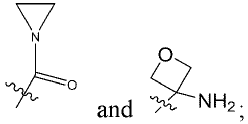
R₂ is selected from H, tert-butyl, (l-ethylaminoacyl)-piperidinyl, hydroxyethyl, isopropyl, fluoropropyl, C(CH₃)₂CF₃, cyclopropyl, hydroxycyclopropyl, cyclobutyl, hydroxycyclobutyl, cyclopentyl, hydroxycyclopentyl, cyclohexyl, hydroxycyclohexyl, methoxyphenyl, methoxybenzyl, methylsulfonylphenyl, (4-methylpiperazinyl)-phenyl, triazolyl, 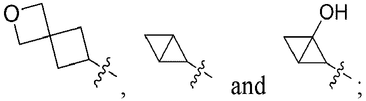
R₂ is preferably selected from methoxyphenyl, methoxybenzyl, methylsulfonylphenyl, (4-methylpiperazinyl)-phenyl, triazolyl, 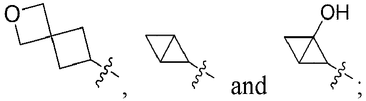 or
R₂, together with X, forms the following groups unsubstituted or substituted with one or two oxo, hydroxy, C₁₋₆ alkyl or C₁₋₆ alkoxy: ureido, pyrazolyl, pyrrolidinyl, imidazolidinyl, pyrrolinyl, piperidinyl, morpholinyl and piperazinyl; preferably, R₂, together with X, forms morpholinyl, *tert*-butylureido, pyrazolyl or (3-oxo)-piperazinyl;
R₃ is selected from H, (3-hydroxy-4-methyl)-cyclohexyl, formyl, dimethylcyclohexyl, vinylcarbonyl, (l-chloromethylcarbonyl)-piperidinyl, (1-carboxymethylcarbonyl)-piperidinyl, carboxyphenyl, (1-cyanomethylcarbonyl)-piperidinyl, (1-acylamino)-piperidinyl, piperidinonyl, hydroxyethylpiperidinonyl, piperidinyl, hydroxypiperidinyl, hydroxymethylphenyl, 1-(2-methylsulfonylethyl)-pyrazolyl and 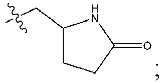 or
R₃, together with W, forms ureido, hydroxypiperidinyl, hydroxymethylpiperidinyl, hydroxymethylpyrrolidinyl, 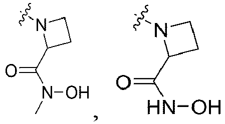 and (2-hydroxy-2-propyl)-azetidinyl.

According to an embodiment of the present disclosure, in the compound of formula (I), 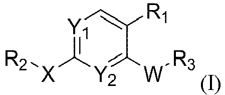 wherein,
Y₁ and Y₂ are both N, or Y₁ is N and Y₂ is CH;
X and W are both NH, or X is O and W is NH;
R₁ is selected from H, hydroxy, halogen, nitro, NH₂, C(=S)NH₂, C(=NH)NH₂, C(=NOH)NH₂, S(O)₂NH₂, C(=NCN)NH₂, C(=NCH₃)NH₂, NHC(O)NH₂, NHC(O)CH₃, methyl, monofluoromethyl, difluoromethyl, trifluoromethyl, methoxy, hydroxypropyl, propynyl, ethynyl, vinyl, allyl, methylcarbonyl, cyclopropylcarbonyl, ethylcarbonyl, isopropylcarbonyl, methoxycarbonyl, monofluoromethylcarbonyl, difluoromethylcarbonyl, trifluoromethylcarbonyl, imidazolyl, pyrazolyl, triazolyl, oxadiazolyl, oxazolyl, isoxazolyl, 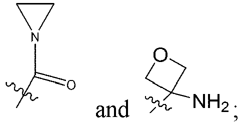
R₂ and R₃ are defined as in any of the embodiments above.

According to an embodiment of the present disclosure, in the compound of formula (I), wherein, 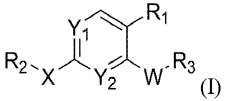
Y₁ and Y₂ are both N, or Y₁ is N and Y₂ is CH;
X and W are both NH, or X is O and W is NH;
R₁ is selected from H, hydroxy, halogen, nitro, NH₂, C(=S)NH₂, C(=NH)NH₂, C(=NOH)NH₂, S(O)₂NH₂, C(=NCN)NH₂, C(=NCH₃)NH₂, NHC(O)NH₂, NHC(O)CH₃, methyl, monofluoromethyl, difluoromethyl, trifluoromethyl, methoxy, hydroxypropyl, propynyl, ethynyl, vinyl, allyl, methylcarbonyl, cyclopropylcarbonyl, ethylcarbonyl, isopropylcarbonyl, methoxycarbonyl, monofluoromethylcarbonyl, difluoromethylcarbonyl, trifluoromethylcarbonyl, imidazolyl, pyrazolyl, triazolyl, oxadiazolyl, oxazolyl, isoxazolyl, 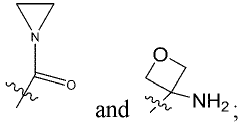
R₂ is selected from H, *tert-*butyl*,* (1-ethylaminoacyl)-piperidinyl, hydroxyethyl, isopropyl, fluoropropyl, C(CH₃)₂CF₃, cyclopropyl, hydroxycyclopropyl, cyclobutyl, hydroxycyclobutyl, cyclopentyl, hydroxycyclopentyl, cyclohexyl, hydroxycyclohexyl, methoxyphenyl, methoxybenzyl, methylsulfonylphenyl, (4-methylpiperazinyl)-phenyl, triazolyl, 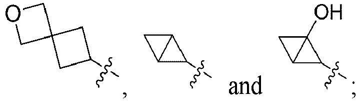 or
R₂, together with X, forms morpholinyl, *tert*-butylureido, pyrazolyl or (3-oxo)-piperazinyl;
R₃ is selected from H, (3-hydroxy-4-methyl)-cyclohexyl, formyl, dimethyl cyclohexyl, vinylcarbonyl, (l-chloromethylcarbonyl)-piperidinyl, (1-carboxymethylcarbonyl)-piperidinyl, carboxyphenyl, (l-cyanomethylcarbonyl)-piperidinyl, (1-acylamino)-piperidinyl, piperidinonyl, hydroxyethylpiperidinonyl, piperidinyl, hydroxypiperidinyl, hydroxymethylphenyl, 1-(2-methylsulfonylethyl)-pyrazolyl and 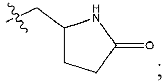 or
R₃, together with W, forms ureido, hydroxypiperidinyl, hydroxymethylpiperidinyl, hydroxymethylpyrrolidinyl, 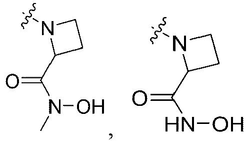 and (2-hydroxy-2-propyl)-azetidinyl.

According to an embodiment of the present disclosure, in the compound of formula (I), 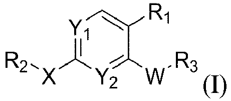 wherein,
Y₁ and Y₂ are both N, or Y₁ is N and Y₂ is CH;
X is O and W is NH;
R₁, R₂ and R₃ are defined as in any of the embodiments above.

According to an embodiment of the present disclosure, in the compound of formula (I), 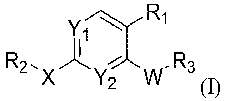 wherein,
Y₁ and Y₂ are both N, or Y₁ is N and Y₂ is CH;
X is O and W is NH;
R₁ is selected from H, hydroxy, halogen, cyano, nitro, NH₂, C(O)NH₂, C(=S)NH₂, C(=NH)NH₂, C(=NOH)NH₂, C(=NCN)NH₂, C(=NCH₃)NH₂, S(O)₂NH₂, NHC(O)NH₂, NHC(O)CH₃, methyl, monofluoromethyl, difluoromethyl, trifluoromethyl, methoxy, hydroxypropyl, ethynyl, propynyl, vinyl, allyl, methylcarbonyl, cyclopropylcarbonyl, ethylcarbonyl, isopropylcarbonyl, methoxycarbonyl, monofluoromethylcarbonyl, difluoromethylcarbonyl, trifluoromethylcarbonyl, imidazolyl, pyrazolyl, triazolyl, oxadiazolyl, oxazolyl, isoxazolyl, 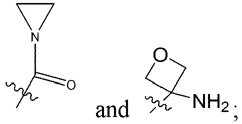
R₂ is selected from H, tert-butyl, (l-ethylaminoacyl)-piperidinyl, hydroxyethyl, isopropyl, fluoropropyl, C(CH₃)₂CF₃, cyclopropyl, hydroxycyclopropyl, cyclobutyl, hydroxycyclobutyl, cyclopentyl, hydroxycyclopentyl, cyclohexyl, hydroxycyclohexyl, methoxyphenyl, methoxybenzyl, methylsulfonylphenyl, (4-methylpiperazinyl)-phenyl, triazolyl, 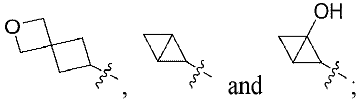 or
R₂, together with X, forms morpholinyl, *tert*-butylureido, pyrazolyl or (3-oxo)-piperazinyl;
R₃ is selected from H, (3-hydroxy-4-methyl)-cyclohexyl, formyl, dimethyl cyclohexyl, vinylcarbonyl, (l-chloromethylcarbonyl)-piperidinyl, (1-carboxymethylcarbonyl)-piperidinyl, carboxyphenyl, (l-cyanomethylcarbonyl)-piperidinyl, (1-acylamino)-piperidinyl, piperidinonyl, hydroxyethylpiperidinonyl, piperidinyl, hydroxypiperidinyl, hydroxymethylphenyl, 1-(2-methylsulfonylethyl)-pyrazolyl and 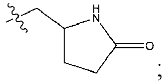 or
R₃, together with W, forms ureido, hydroxypiperidinyl, hydroxymethylpiperidinyl, hydroxymethylpyrrolidinyl, 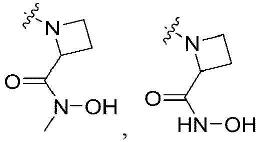 and (2-hydroxy-2-propyl)-azetidinyl.

According to an embodiment of the present disclosure, in the compound of formula (I), 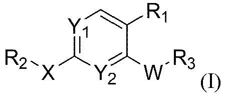 wherein,
Y₁ and Y₂ are both N, or Y₁ is N and Y₂ is CH;
X and W are both NH, or X is O and W is NH;
R₂, together with X, forms the following groups unsubstituted or substituted with one, two or more oxo, hydroxy, C₁₋₆ alkyl or C₁₋₆ alkoxy: ureido, 5-6 membered heterocyclyl and 5-6 membered heteroaryl;
Preferably, R₂, together with X, forms the following groups unsubstituted or optionally substituted with one, two or more R_{b}: NHC(O)NR₁₁R₁₂, azetidinyl, piperidinyl, piperazinyl, morpholinyl, pyrrolidinyl, imidazolidinyl, pyrrolinyl, pyranyl, imidazolyl, pyrazolyl, triazolyl, oxadiazolyl, isoxadiazolyl, thiadiazolyl, isothiadiazolyl, tetrazolyl, pyrrolyl, furanyl, thiazolyl, isothiazolyl, oxazolyl, isoxazolyl, pyridinyl, pyrimidinyl, pyridazinyl and pyrazinyl;
R₁, R₃, R₁₁, R₁₂ and R_{b} are defined as in any of the embodiments above.

According to an embodiment of the present disclosure, in the compound of formula (I), 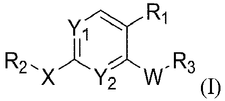 wherein,
Y₁ and Y₂ are both N, or Y₁ is N and Y₂ is CH;
X and W are both NH, or X is O and W is NH;
R₁ is selected from H, hydroxy, halogen, cyano, nitro, NH₂, C(O)NH₂, C(=S)NH₂, C(=NH)NH₂, C(=NOH)NH₂, C(=NCN)NH₂, C(=NCH₃)NH₂, S(O)₂NH₂, NHC(O)NH₂, NHC(O)CH₃, methyl, monofluoromethyl, difluoromethyl, trifluoromethyl, methoxy, hydroxypropyl, ethynyl, propynyl, vinyl, allyl, methylcarbonyl, cyclopropylcarbonyl, ethylcarbonyl, isopropylcarbonyl, methoxycarbonyl, monofluoromethylcarbonyl, difluoromethylcarbonyl, trifluoromethylcarbonyl, imidazolyl, pyrazolyl, triazolyl, oxadiazolyl, oxazolyl, isoxazolyl, 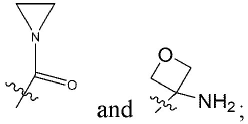
R₂, together with X, forms the following groups substituted with one or two oxo, hydroxy, C₁₋₆ alkyl or C₁₋₆ alkoxy: ureido, pyrrolidinyl, imidazolidinyl, pyrrolinyl, pyrazolyl, imidazolyl, piperidinyl, morpholinyl and piperazinyl;
R₃ is selected from H, (3-hydroxy-4-methyl)-cyclohexyl, formyl, dimethylcyclohexyl, vinylcarbonyl, (1-chloromethylcarbonyl)-piperidinyl, (1-carboxymethylcarbonyl)-piperidinyl, carboxyphenyl, (l-cyanomethylcarbonyl)-piperidinyl, (1-acylamino)-piperidinyl, piperidinonyl, hydroxyethylpiperidinonyl, piperidinyl, hydroxypiperidinyl, hydroxymethylphenyl, 1-(2-methylsulfonylethyl)-pyrazolyl and 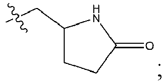 or
R₃, together with W, forms ureido, hydroxypiperidinyl, hydroxymethylpiperidinyl, hydroxymethylpyrrolidinyl, 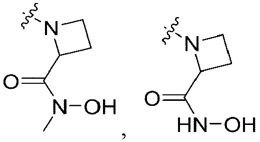 and (2-hydroxy-2-propyl)-azetidinyl.

According to an embodiment of the present disclosure, in the compound of formula (I), 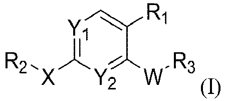 wherein,
Y₁ and Y₂ are both N, or Y₁ is N and Y₂ is CH;
X and W are both NH, or X is O and W is NH;
R₃, together with W, forms the following groups unsubstituted or substituted with one, two or more C(O)NHOH, C(O)N(OH)CH₃, oxo, hydroxy, C₁₋₆ alkyl, hydroxy-C₁₋₆ alkyl or C₁₋₆ alkoxy: ureido and 3-7 membered heterocyclyl;
R₁ and R₂ are defined as in any of the embodiments above.

According to an embodiment of the present disclosure, in the compound of formula (I), 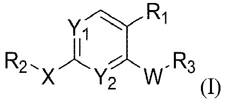 wherein,
Y₁ and Y₂ are both N, or Y₁ is N and Y₂ is CH;
X and W are both NH, or X is O and W is NH;
R₃, together with W, forms the following groups unsubstituted or substituted with one, two or more C(O)NHOH, C(O)N(OH)CH₃, oxo, hydroxy, C₁₋₆ alkyl, hydroxy-C₁₋₆ alkyl or C₁₋₆ alkoxy: ureido, pyrrolidinyl, imidazolidinyl, morpholinyl, pyrrolinyl, piperidinyl, pyrrolidinyl and azetidinyl;
R₁ and R₂ are defined as in any of the embodiments above.

According to an embodiment of the present disclosure, in the compound of formula (I), 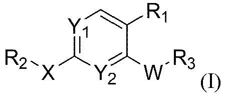 wherein,
Y₁ and Y₂ are both N, or Y₁ is N and Y₂ is CH;
X and W are both NH, or X is O and W is NH;
R₁ is selected from H, hydroxy, halogen, cyano, nitro, NH₂, C(O)NH₂, C(=S)NH₂, C(=NH)NH₂, C(=NOH)NH₂, C(=NCN)NH₂, C(=NCH₃)NH₂, S(O)₂NH₂, NHC(O)NH₂, NHC(O)CH₃, methyl, monofluoromethyl, difluoromethyl, trifluoromethyl, methoxy, hydroxypropyl, ethynyl, propynyl, vinyl, allyl, methylcarbonyl, cyclopropylcarbonyl, ethylcarbonyl, isopropylcarbonyl, methoxycarbonyl, monofluoromethylcarbonyl, difluoromethylcarbonyl, trifluoromethylcarbonyl, imidazolyl, pyrazolyl, triazolyl, oxadiazolyl, oxazolyl, isoxazolyl, 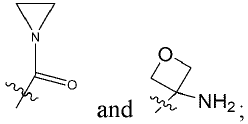
R₂ is selected from H, tert-butyl, (l-ethylaminoacyl)-piperidinyl, hydroxyethyl, isopropyl, fluoropropyl, C(CH₃)₂CF₃, cyclopropyl, hydroxycyclopropyl, cyclobutyl, hydroxycyclobutyl, cyclopentyl, hydroxycyclopentyl, cyclohexyl, hydroxycyclohexyl, methoxyphenyl, methoxybenzyl, methylsulfonylphenyl, (4-methylpiperazinyl)-phenyl, triazolyl, or 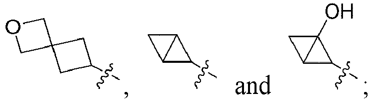
R₂, together with X, forms morpholinyl, *tert*-butylureido, pyrazolyl or (3-oxo)-piperazinyl;
R₃, together with W, forms hydroxypiperidinyl, hydroxymethylpiperidinyl, hydroxymethylpyrrolidinyl, 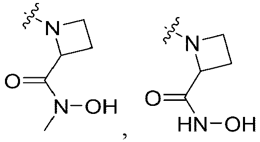 and (2-hydroxy-2-propyl)-azetidinyl.

According to an embodiment of the present disclosure, the compound of formula (I) is a compound of the following formula (II): 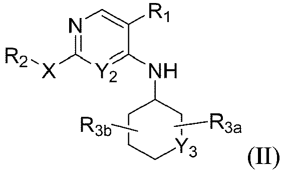 wherein,
Y₂, X, R₁ and R₂ are defined as in any of the embodiments of formula (I) above;
Y₃ is CR₈R₉ or NR₁₀;
R₈ and R₉ are the same or different, and are each independently selected from H, hydroxy, halogen, cyano, and the following groups unsubstituted or optionally substituted with one, two or more hydroxy or halogen: C₁₋₆ alkyl, C₁₋₆ alkoxy, C₁₋₆ alkenyl and C₁₋₆ alkynyl; or R₈ and R₉, together with the carbon atom attached thereto, form carbonyl, i.e., CR₈R₉ is C(=O);
R₁₀ is selected from H, C(O)NH₂, C(O)CH₂CN, C(O)CH₂COOH, and the following groups unsubstituted or optionally substituted with one, two or more hydroxy or halogen: C₁₋₆ alkyl, C₁₋₆ alkoxy, C₁₋₆ alkenyl and C₁₋₆ alkynyl;
R₃ₐ and R_{3b} are the same or different, and are each independently selected from H, hydroxy, halogen, cyano, oxo, and the following groups unsubstituted or optionally substituted with one, two or more hydroxy or halogen: C₁₋₆ alkyl, C₁₋₆ alkoxy, C₁₋₆ alkenyl and C₁₋₆ alkynyl.

According to an embodiment of the present disclosure, in the compound of formula (II), 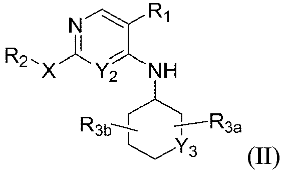 wherein,
Y₂, X, R₁ and R₂ are defined as in any of the embodiments of formula (I) above;
Y₃ is CR₈R₉ or NR₁₀;
R₈ is H, and R₉ is selected from H, hydroxy, halogen, cyano, and the following groups unsubstituted or optionally substituted with one, two or more hydroxy or halogen: C₁₋₆ alkyl, C₁₋₆ alkoxy, C₁₋₆ alkenyl and C₁₋₆ alkynyl; or R₈ and R₉, together with the carbon atom attached thereto, form carbonyl, i.e., CR₈R₉ is C(=O);
R₁₀ is selected from H, C(O)NH₂, C(O)CH₂CN, C(O)CH₂COOH, and C₁₋₆ alkyl unsubstituted or optionally substituted with one, two or more hydroxy or halogen;
R₃ₐ and R_{3b} are the same or different, and are each independently selected from H, hydroxy, halogen, cyano, oxo, and the following groups unsubstituted or optionally substituted with one, two or more hydroxy or halogen: C₁₋₆ alkyl, C₁₋₆ alkoxy, C₁₋₆ alkenyl and C₁₋₆ alkynyl.

According to an embodiment of the present disclosure, in the compound of formula (II), 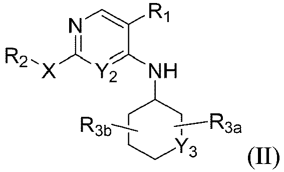 wherein,
Y₂, X, R₁ and R₂ are defined as in any of the embodiments of formula (I) above;
Y₃ is CR₈R₉;
R₈ is H, and R₉ is selected from hydroxy and C₁₋₆ alkoxy unsubstituted or optionally substituted with one, two or more hydroxy or halogen;
R₃ₐ is located at position 4 of a cyclohexyl ring and is selected from H, hydroxy, halogen, cyano, oxo, and the following groups unsubstituted or optionally substituted with one, two or more hydroxy or halogen: C₁₋₆ alkyl, C₁₋₆ alkoxy, C₁₋₆ alkenyl and C₁₋₆ alkynyl; and R_{3b} is selected from H, hydroxy, halogen, cyano, oxo, and the following groups unsubstituted or optionally substituted with one, two or more hydroxy or halogen: C₁₋₆ alkyl and C₁₋₆ alkoxy.

According to an embodiment of the present disclosure, the compound of formula (I) is a compound of the following formula (II-1): 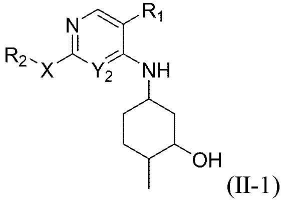 wherein,
the substituents Y₂, X, R₁ and R₂ are defined as above for formula (I).

According to an embodiment of the present disclosure, in the compound of formula (II-1),
Y₂is N or CH;
X is NH or O;
R₁ is selected from H, hydroxy, halogen, cyano, nitro, NR₁₁R₁₂, C(O)NR₁₁R₁₂, C(=S)NR₁₁R₁₂, S(O)₂NR₁₁R₁₂, C(=NR₁₃)NR₁₁R₁₂, NHC(O)NR₁₁R₁₂, P(O)₂NR₁₁R₁₂, P(O)R₁₃NR₁₁R₁₂, C(O)R₁₄, NHC(O)R₁₄, C(O)OR₁₅, and the following groups unsubstituted or optionally substituted with one, two or more Rₐ: C₁₋₆ alkyl, C₂₋₆ alkenyl, C₂₋₆ alkynyl, C₁₋₆ alkoxy, C₃₋₁₀ cycloalkyl, 3-10 membered heterocyclyl, C₆₋₁₄ aryl and 5-14 membered heteroaryl;
R₂ is selected from H and the following groups unsubstituted or optionally substituted with one, two or more R_{b}: C₁₋₆ alkyl, C₂₋₆ alkenyl, C₂₋₆ alkynyl, C₁₋₆ alkoxy, C₃₋₁₀ cycloalkyl, 3-10 membered heterocyclyl, C₆₋₁₄ aryl, C₆₋₁₄ aryl-C₁₋₁₀ alkyl, 5-14 membered heteroaryl and 5-14 membered heteroaryl-Ci-io alkyl; or
R₂, together with X, forms the following groups unsubstituted or optionally substituted with one, two or more R_{b}: NHC(O)NR₁₁R₁₂, 3-10 membered heterocyclyl, C₆₋₁₄ aryl and 5-14 membered heteroaryl;
R₁₁, R₁₂, R₁₃, R₁₄ and R₁₅ are defined as in any of the embodiments of formula (I) above;
each Rₐ is independently selected from CN, halogen, OH, NH₂, oxo, and the following groups unsubstituted or optionally substituted with one, two or more R_{f}: C₁₋₆ alkyl and C₁₋₆ alkoxy;
each R_{b} is independently selected from CN, halogen, OH, NH₂, COOH, NO₂, oxo, S(O)₂CH₃, C(O)NHCH₂CH₃, and the following groups unsubstituted or optionally substituted with one, two or more R_{f}: C₁₋₆ alkyl, C₁₋₆ alkoxy, C₃₋₁₀ cycloalkyl, 3-10 membered heterocyclyl, C₆₋₁₄ aryl and 5-14 membered heteroaryl;
R_{f} is defined as in any of the embodiments of formula (I) above.

According to an embodiment of the present disclosure, in the compound of formula (II-1),
Y₂ is N or CH;
X is NH or O;
R₁ is selected from H, hydroxy, halogen, cyano, nitro, NR₁₁R₁₂, C(O)NR₁₁R₁₂, C(=S)NR₁₁R₁₂, S(O)₂NR₁₁R₁₂, C(=NR₁₃)NR₁₁R₁₂, NHC(O)NR₁₁R₁₂, P(O)₂NR₁₁R₁₂, P(O)R₁₃NR₁₁R₁₂, C(O)R₁₄, NHC(O)R₁₄, C(O)OR₁₅, and the following groups unsubstituted or optionally substituted with one, two or more Rₐ: C₁₋₆ alkyl, C₂₋₆ alkenyl, C₂₋₆ alkynyl, C₁₋₆ alkoxy, C₃₋₆ cycloalkyl, 3-7 membered heterocyclyl and 5-6 membered heteroaryl;
R₂ is selected from H and the following groups unsubstituted or optionally substituted with one, two or more R_{b}: C₁₋₆ alkyl, C₂₋₆ alkenyl, C₂₋₆ alkynyl, C₁₋₆ alkoxy, C₃₋₆ cycloalkyl, 3-7 membered heterocyclyl, phenyl, benzyl, 5-6 membered heteroaryl and 5-6 membered heteroaryl-C₁₋₆ alkyl; or R₂, together with X, forms the following groups unsubstituted or optionally substituted with one, two or more R_{b}: NHC(O)NR₁₁R₁₂, 3-7 membered heterocyclyl and 5-6 membered heteroaryl;
R₁₁, R₁₂, R₁₃, R₁₄ and R₁₅ are defined as in any of the embodiments of formula (I) above;
each Ra is independently selected from CN, halogen, OH, NH₂, oxo, and the following groups unsubstituted or optionally substituted with one, two or more R_{f}: C₁₋₆ alkyl and C₁₋₆ alkoxy; and
each R_{b} is independently selected from CN, halogen, OH, NH₂, COOH, NO₂, oxo, S(O)₂CH₃, C(O)NHCH₂CH₃, and the following groups unsubstituted or optionally substituted with one, two or more R_{f}: C₁₋₆ alkyl, C₁₋₆ alkoxy, C₃₋₆ cycloalkyl, 3-7 membered heterocyclyl, phenyl and 5-6 membered heteroaryl;
each R_{c} is independently selected from CN, halogen, OH, NH₂, COOH, NO₂, C(O)NH₂, C(O)NHOH, C(O)N(OH)CH₃, oxo, C(O)CH₂COOH, C(O)CH₂CN, C(O)CH₂Cl, C(O)CH₂F, C(O)CH₂Br, and the following groups unsubstituted or optionally substituted with one, two or more R_{f}: C₁₋₆ alkyl, C₁₋₆ alkoxy, 3-7 membered heterocyclyl and 5-6 membered heteroaryl;
each R_{f} are the same or different, and are each independently selected from CN, halogen, OH, NH₂, oxo, S(O)₂CH₃,C₁₋₆ alkyl, C₁₋₆ haloalkyl, C₁₋₆ alkoxy and C₁₋₆ haloalkoxy.

According to an embodiment of the present disclosure, in the compound of formula (II-1), 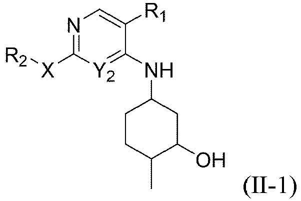 wherein,
Y₂ is N or CH;
X is NH or O;
R₁ is selected from H, hydroxy, halogen, cyano, nitro, NR₁₁R₁₂, C(O)NR₁₁R₁₂, C(=S)NR₁₁R₁₂, S(O)₂NR₁₁R₁₂, C(=NR₁₃)NR₁₁R₁₂, NHC(O)NR₁₁R₁₂, P(O)₂NR₁₁R₁₂, P(O)R₁₃NR₁₁R₁₂, C(O)R₁₄, NHC(O)R₁₄, C(O)OR₁₅, and the following groups unsubstituted or optionally substituted with one, two or more Rₐ: C₁₋₆ alkyl, C₂₋₆ alkenyl, C₂₋₆ alkynyl, C₁₋₆ alkoxy, imidazolyl, pyrazolyl, triazolyl, oxadiazolyl, oxetanyl, azetidinyl, isoxadiazolyl, thiadiazolyl, isothiadiazolyl, tetrazolyl, pyrrolyl, furanyl, thiazolyl, isothiazolyl, oxazolyl, isoxazolyl, pyridinyl, pyrimidinyl, pyridazinyl, pyrazinyl, pyranyl and thienyl;
R₂ is selected from H and the following groups unsubstituted or optionally substituted with one, two or more R_{b}: C₁₋₆ alkyl, C₂₋₆ alkenyl, C₂₋₆ alkynyl, C₁₋₆ alkoxy, C₃₋₆ cycloalkyl, phenyl, benzyl, piperidinyl, piperazinyl, morpholinyl, imidazolyl, pyrazolyl, triazolyl, oxadiazolyl, isoxadiazolyl, thiadiazolyl, isothiadiazolyl, tetrazolyl, pyrrolidinyl, oxetanyl, pyrrolyl, furanyl, thiazolyl, isothiazolyl, oxazolyl, isoxazolyl, pyridinyl, pyrimidinyl, pyridazinyl, pyrazinyl, pyranyl, thienyl and 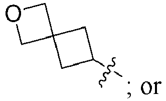
R₂, together with X, forms the following groups unsubstituted or optionally substituted with one, two or more R_{b}: NHC(O)NR₁₁R₁₂, azetidinyl, piperidinyl, piperazinyl, morpholinyl, pyrrolidinyl, imidazolidinyl, pyrrolinyl, pyranyl, imidazolyl, pyrazolyl, triazolyl, oxadiazolyl, isoxadiazolyl, thiadiazolyl, isothiadiazolyl, tetrazolyl, pyrrolyl, furanyl, thiazolyl, isothiazolyl, oxazolyl, isoxazolyl, pyridinyl, pyrimidinyl, pyridazinyl and pyrazinyl;
R₁₁ and R₁₂ are independently selected from H and C₁₋₆ alkyl unsubstituted or optionally substituted with one, two or more Rₑ; or
R₁₁ and R₁₂, together with the nitrogen atom attached thereto, form 3-7 membered azacycloalkyl unsubstituted or optionally substituted with one, two or more Rₑ;
R₁₃ is selected from H, cyano, methyl and hydroxy;
R₁₄ is selected from H, hydroxy, halogen, and the following groups unsubstituted or optionally substituted with one, two or more R_{f}: C₁₋₆ alkyl, C₂₋₆ alkenyl, C₂₋₆ alkynyl, C₁₋₆ alkoxy and C₃₋₆ cycloalkyl;
R₁₅ is selected from H and C₁₋₆ alkyl unsubstituted or optionally substituted with one, two or more R_{f};
each Rₐ is independently selected from CN, halogen, OH, NH₂, oxo, C₁₋₆ alkyl, C₁₋₆ haloalkyl, C₁₋₆ alkoxy, C₁₋₆ haloalkoxy and CH₂CH₂S(O)₂CH₃; and
each R_{b} is independently selected from CN, halogen, OH, NH₂, COOH, NO₂, oxo, S(O)₂CH₃, C(O)NHCH₂CH₃, and the following groups unsubstituted or optionally substituted with one, two or more R_{f}: C₁₋₆ alkyl, C₁₋₆ alkoxy, phenyl and piperidinyl;
each Rₑ and each R_{f} are independently selected from CN, halogen, OH, NH₂, oxo, S(O)₂CH₃,C₁₋₆ alkyl, C₁₋₆ haloalkyl, C₁₋₆ alkoxy and C₁₋₆ haloalkoxy.

According to an embodiment of the present disclosure, in the compound of formula (II-1), 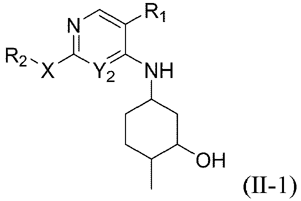 wherein,
Y₂ is N or CH;
X is NH or O;
R₁ is selected from H, hydroxy, halogen, cyano, nitro, NH₂, C(O)NH₂, C(=S)NH₂, C(=NH)NH₂, C(=NOH)NH₂, C(=NCN)NH₂, C(=NCH₃)NH₂, NHC(O)NH₂, S(O)₂NH₂, NHC(O)CH₃, methyl, monofluoromethyl, difluoromethyl, trifluoromethyl, methoxy, hydroxypropyl, ethynyl, vinyl, propynyl, allyl, methylcarbonyl, cyclopropylcarbonyl, ethylcarbonyl, isopropylcarbonyl, methoxycarbonyl, monofluoromethylcarbonyl, difluoromethylcarbonyl, trifluoromethylcarbonyl, imidazolyl, pyrazolyl, triazolyl, oxadiazolyl, oxazolyl, isoxazolyl, 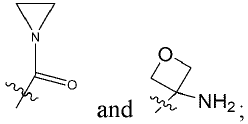
R₂ is selected from H, tert-butyl, (1-ethylaminoacyl)-piperidinyl, hydroxyethyl, isopropyl, fluoropropyl, C(CH₃)₂CF₃, cyclopropyl, hydroxycyclopropyl, cyclobutyl, hydroxycyclobutyl, hydroxycyclopentyl, cyclopentyl, hydroxycyclohexyl, cyclohexyl, methoxyphenyl, methoxybenzyl, methylsulfonylphenyl, (4-methylpiperazinyl)-phenyl, triazolyl, 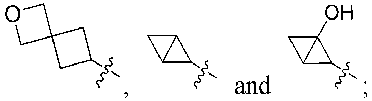 or
R₂, together with X, forms the following groups unsubstituted or substituted with one or two oxo, hydroxy, C₁₋₆ alkyl or C₁₋₆ alkoxy: ureido, pyrazolyl, pyrrolidinyl, imidazolidinyl, pyrrolinyl, piperidinyl, morpholinyl and piperazinyl.

According to an embodiment of the present disclosure, in the compound of formula (II-1), 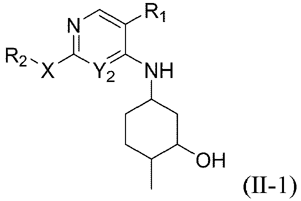 wherein,
Y₂ is N or CH;
X is NH or O;
R₁ is selected from H, hydroxy, halogen, cyano, nitro, NH₂, C(O)NH₂, C(=S)NH₂, C(=NH)NH₂, C(=NOH)NH₂, C(=NCN)NH₂, C(=NCH₃)NH₂, S(O)₂NH₂, NHC(O)NH₂, NHC(O)CH₃, methyl, monofluoromethyl, difluoromethyl, trifluoromethyl, methoxy, hydroxypropyl, ethynyl, propynyl, vinyl, allyl, methylcarbonyl, cyclopropylcarbonyl, ethylcarbonyl, isopropylcarbonyl, methoxycarbonyl, monofluoromethylcarbonyl, difluoromethylcarbonyl, trifluoromethylcarbonyl, imidazolyl, pyrazolyl, triazolyl, oxadiazolyl, oxazolyl, isoxazolyl, 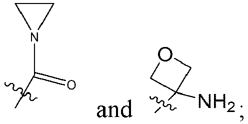
R₂ is selected from H, methoxyphenyl, methoxybenzyl, methylsulfonylphenyl, (4-methylpiperazinyl)-phenyl, triazolyl, 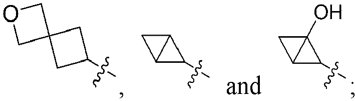 or
R₂, together with X, forms morpholinyl, *tert*-butylureido, pyrazolyl or (3-oxo)-piperazinyl.

According to an embodiment of the present disclosure, the compound of formula (II-1) has a steric structure of the following formula (II-1a): 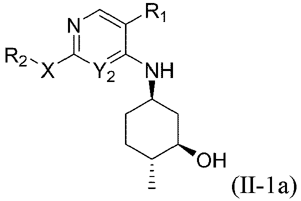 wherein all the groups are defined as in any of the embodiments of the compound of formula (I) or formula (II-1) above.

As an example, the compound of formula (I) can be selected from the compounds exemplified in Table 1 below and pharmaceutically acceptable salts thereof:

**Table 1**

| | | | |
|---|---|---|---|
| Example | Compound No. | Compound structure | IUPAC name |
| 1 | T001 | 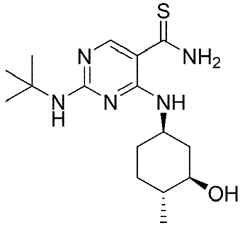 | 2-(*tert*-butylamino)-4-(((1*R*,3*R*,4*R*)-3-hydr oxy-4-methylcyclohexyl)amino)pyrimidine -5-carbothioamide |
| 1 | T001A | 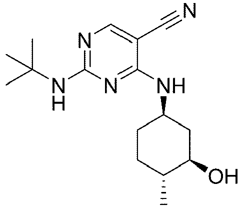 | 2-(*tert*-butylamino)-4-(((1*R*,3*R*,4*R*)-3-hydr oxy-4-methylcyclohexyl)amino)pyrimidine -5-carbonitrile |
| 2 | T002 | 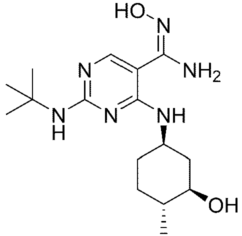 | (*E*)-2-(*tert*-butylamino)-*N'*-hydroxy-4-(((1 *R*,3*R*,4*R*)-3-hydroxy-4-methylcyclohexyl)a mino)pyrimidine-5-carboximidamide |
| 3 | T003 | 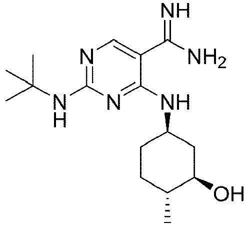 | 2-(*tert*-butylamino)-4-(((1*R*,3*R*,4*R*)-3-hydr oxy-4-methylcyclohexyl)amino)pyrimidine -5-carboximidamide |
| 4 | T004 | 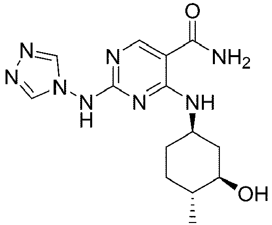 | 2-((4*H*-1,2,4-triazol-4-yl)amino)-4-(((1*R*,3 *R*,4*R*)-3-hydroxy-4-methylcyclohexyl)ami no)pyrimidine-5-carboxamide |
| 5 | T005A | 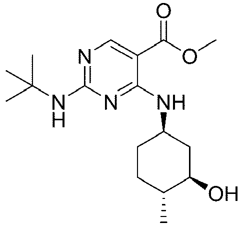 | Methyl 2-(*tert*-butylamino)-4-(((1*R*,3*R*,4*R*)-3-hydr oxy-4-methylcyclohexyl)amino)pyrimidine -5-carboxylate |
| 5 | T005 | 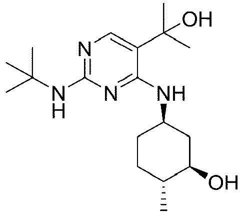 | (1*R,*2*R,*5*R*)-5-((2-(*tert*-butylamino)-5-(2-h ydroxypropan-2-yl) pyrimidin-4-yl)amino)-2-methylcyclohexa n-1-ol |
| 6 | T006 | 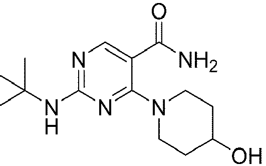 | 2-(*tert*-butylamino)-4-(4-hydroxypiperidin-1-yl)pyrimidine-5-carboxamide |
| 7 | T007 | 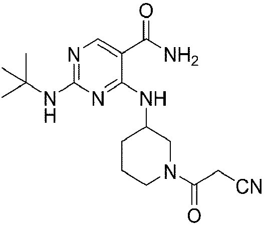 | 2-(*tert*-butylamino)-4-((1-(2-cyanoacetyl)p iperidin-3-yl)amino)pyrimidine-5-carboxa mide |
| 8 | T008 | 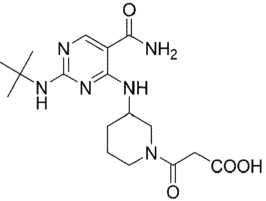 | 3-(3-((2-(*tert*-butylamino)-5-carbamoylpyr imidin-4-yl)amino)piperidin-1-yl)-3-oxopr opanoic acid |
| 9 | T009 | 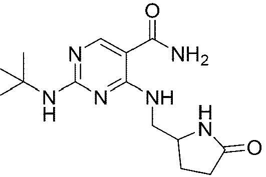 | 2-(*tert*-butylamino)-4-(((5-oxopyrrolidin-2 -yl)methyl)amino)pyrimidine-5-carboxami de |
| 10 | T010 | 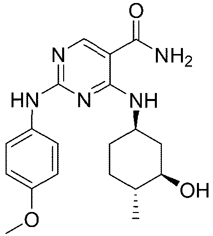 | 4-(((1*R*,3*R*,4*R*)-3-hydroxy-4-methylcycloh exyl)amino)-2-((4-methoxyphenyl)amino) pyrimidine-5-carboxamide |
| 11 | T011 | 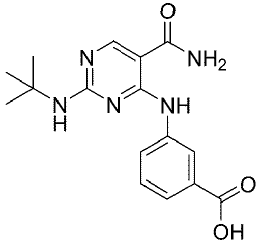 | 3-((2-(*tert*-butylamino)-5-carbamoylpyrimi din-4-yl)amino)benzoic acid |
| 12 | T012 | 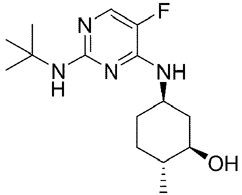 | (1*R*,2*R,*5*R*)-5-((2-(*tert*-butylamino)-5-fluor opyrimidin-4-yl )amino)-2-methylcyclohex an-1-ol |
| 13 | T013 | 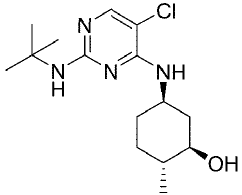 | (1*R*,2*R*,5*R*)-5-((2-(tert-butylamino)-5-chlor opyrimidin-4-yl)amino)-2-methylcyclohex an-1-ol |
| 14 | T014 | 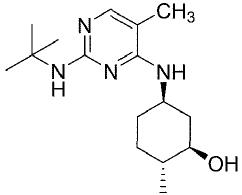 | (1*R*,2*R*,5*R*)-5-((2-(*tert*-butylamino )-5-meth ylpyrimidin-4-yl)amino)-2-methylcyclohex -1-ol |
| 15 | T015 | 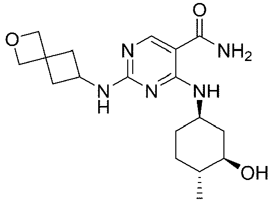 | 2-((2-oxaspiro[3.3]heptan-6-yl)amino)-4-(( (1*R*,3*R*,4*R*)-3-hydroxy-4-methylcyclohexyl )amino)pyrimidine-5-carboxamide |
| 16 | T016 | 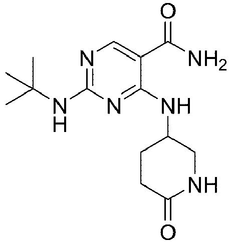 | 2-(*tert*-butylamino)-4-((6-oxopiperidin-3-y l)amino)pyrimidine-5-carboxamide |
| 17 | T017 | 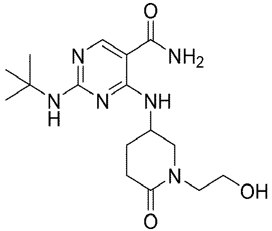 | 2-(*tert*-butylamino)-4-((1-(2-hydroxyethyl) -6-oxopiperidin-3-yl)amino)pyrimidine-5-c arb oxamide |
| 18 | T018 | 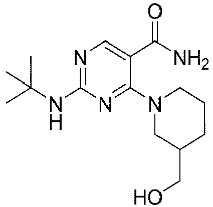 | 2-(*tert*-butylamino)-4-(3 -(hydroxymethyl)p iperidin-1-yl)pyrimidine-5-carboxamide |
| 19 | T019 | 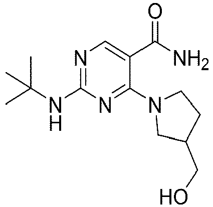 | 2-(*tert*-butylamino)-4-(3 -(hydroxymethyl)p yrrolidin-1-yl)pyrimidine-5-carboxamide |
| 20 | T020 | 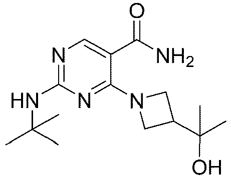 | 2-(*tert*-butylamino)-4-(3-(2-hydroxypropan -2-yl)azetidin-1-yl)pyrimidine-5-carboxam ide |
| 21 | T021 | 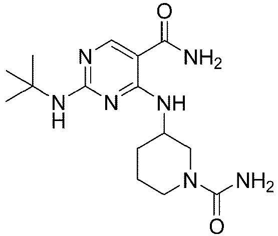 | 2-(*tert*-butylamino)-4-((1-carbamoylpiperi din-3-yl)amino)pyrimidine-5-carboxamide |
| 22 | T022 | 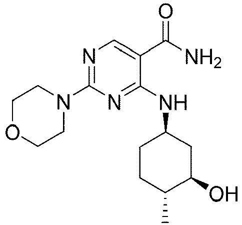 | 4-(((1*R*,3*R*,4*R*)-3-hydroxy-4-methylcycloh exyl)amino)-2-morpholinopyrimidine-5-ca rboxamide |
| 23 | T023 | 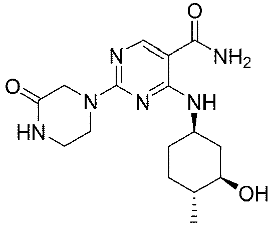 | 4-(((1*R*,3*R*,4*R*)-3-hydroxy-4-methylcycloh exyl)amino)-2-(3-oxopiperazin-1-yl)pyrimi dine-5-carboxamide |
| 24 | T024 | 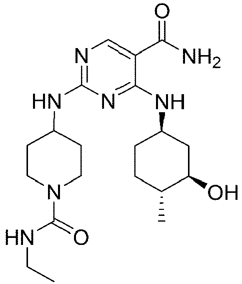 | 2-((1-(ethylcarbamoyl)piperidin-4-yl)amin o)-4-(((1*R*,3*R*,4*R*)-3-hydroxy-4-methylcycl ohexyl)amino)pyrimidine-5-carboxamide |
| 25 | T025 | 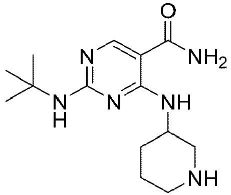 | 2-(*tert*-butylamino)-4-(piperidin-3-ylamino )pyrimidine-5-carboxamide |
| 26 | T026 | 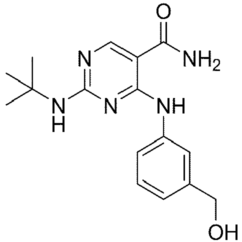 | 2-(*tert*-butylamino)-4-((3-(hydroxymethyl) phenyl)amino)pyrimidine-5-carboxamide |
| 27 | T027 | 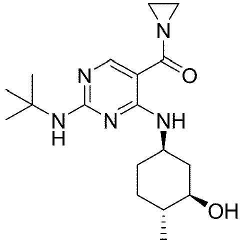 | Aziridin-1-yl(2-(*tert*-butylamino)-4-(((1*R*, 3*R*,4*R*)-3-hydroxy-4-methylcyclohexyl)am ino)pyrimidin-5-yl)methanone |
| 28 | T028 | 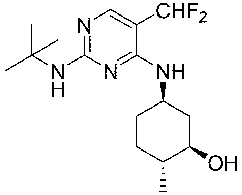 | (1*R*,2*R*,5*R*)-5-((2-(*tert*-butylamino)-5-(difl uoromethyl)pyrimidin-4-yl)amino)-2-meth ylcyclohex-1-ol |
| 29 | T029 | 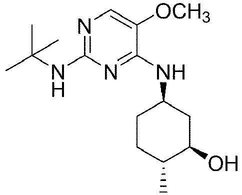 | (1R,2R, 5*R*)-5-((2-(*tert*-butylamino)-5-meth oxypyrimidin-4-yl)amino)-2-methylcycloh ex-1-ol |
| 30 | T030 | 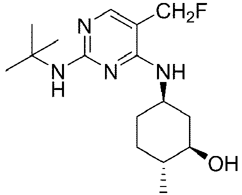 | (1*R,*2*R,*5*R*)-5-((2-(*tert*-butylamino)-5-(fluo romethyl)pyrimidin-4-yl)amino)-2-methylc yclohex-1-ol |
| 31 | T031 | 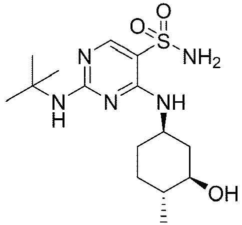 | 2-(*tert*-butylamino)-4-(((1*R*,3*R*,4*R*)-3-hydr oxy-4-methylcyclohexyl)amino)pyrimidine -5-sulfamide |
| 32 | T032 | 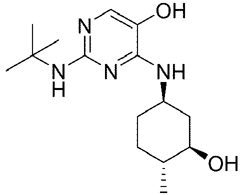 | 2-(*tert*-butylamino)-4-(((1*R*,3*R*,4*R*)-3-hydr oxy-4-methylcyclohexyl)amino)pyrimidine -5-ol |
| 33 | T033 | 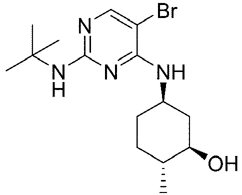 | (1R,2R, 5*R*)-5-((5-bromo-2-(*tert*-butylamin o)pyrimidin-4-yl)amino)-2-methylcyclohe xan-1-ol |
| 34 | T034 | 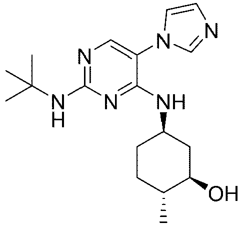 | (1*R*,2*R*,5*R*)-5-((2-(*tert*-butylamino)-5-(1*H-*imidazol-1-yl)pyrimidin-4-yl)amino)-2-me thylcyclohex-1-ol |
| 35 | T035 | 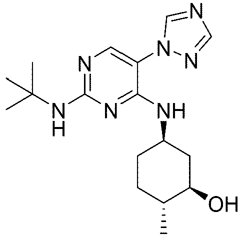 | (1*R*,2*R*,5*R*)-5-((2-(*tert*-butylamino)-5-(1*H-*1,2,4-triazol-1-yl)pyrimidin-4-yl)amino)-2-methylcyclohexan-1-ol |
| 36 | T036 | 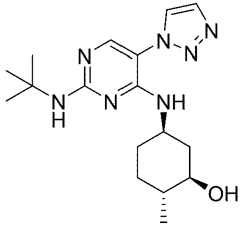 | (1*R*,2*R*,5*R*)-5-((2-(*tert*-butylamino)-5-(1*H-*1,2,3-triazol-1-yl)pyrimidin-4-yl)amino)-2-methylcyclohexan-1-ol |
| 37 | T037 | 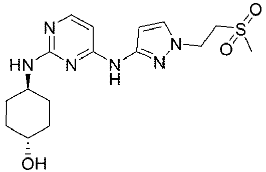 | (1*R*,4*R*)-4-((4-((1-(2-(methylsulfonyl)ethyl )-1*H*-pyrazol-3-yl)amino)pyrimidin-2-yl)a mino)cyclohexan-1-ol |
| 38 | T038 | 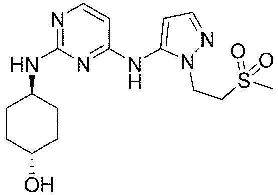 | (1*R*,4*R*)-4-((4-((1-(2-(methylsulfonyl)ethyl )-1*H*-pyrazol-5-yl)amino)pyrimidin-2-yl)a mino)cyclohexan-1-ol |
| 39 | T039 | 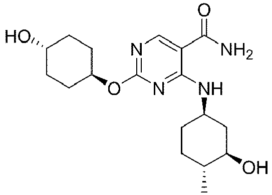 | 4-(((1*R*,3*R*,4*R*)-3-hydroxy-4-methylcycloh exyl)amino)-2-(((1*R*,4*R*)-4-hydroxycycloh exyl)oxy)pyrimidine-5-carboxamide |
| 40 | T040 | 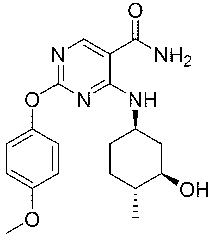 | 4-(((1*R*,3*R*,4*R*)-3-hydroxy-4-methylcycloh exyl)amino)-2-(4-methoxyphenoxy)pyrimi dine-5-carboxamide |
| 41 | T041 | 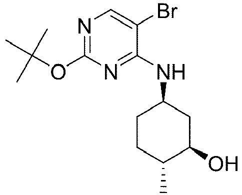 | (1R,2R, 5*R*)-5-((5-bromo-2-(*tert*-butoxy)py rimidin-4-yl)amino)-2-methylcyclohexan-1 -ol |
| 42 | T042 | 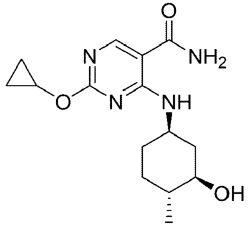 | 2-cyclopropoxy-4-(((1*R*,3*R*,4*R*)-3-hydroxy -4-methylcyclohexyl)amino)pyrimidine-5-carboxamide |
| 43 | T043 | 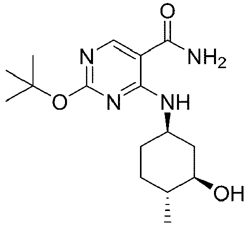 | 2-(*tert*-butoxy)-4-(((1*R*,3*R*,4*R*)-3-hydroxy-4-methylcyclohexyl)amino)pyrimidine-5-c arb oxamide |
| 44 | T044 | 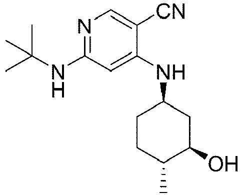 | 6-(*tert*-butylamino)-4-(((1*R*,3*R*,4*R*)-3-hydr oxy-4-methylcyclohexyl)amino)nicotinonit rile |
| 45 | T045 | 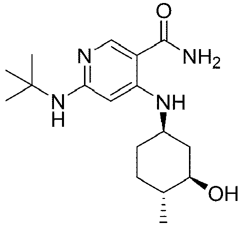 | 6-(*tert*-butylamino)-4-(((1*R*,3*R*,4*R*)-3-hydr oxy-4-methylcyclohexyl)amino)nicotinami de |
| 46 | T046 | 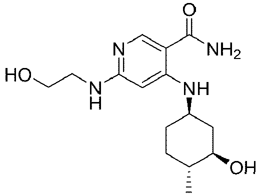 | 4-(((1*R*,3*R*,4*R*)-3-hydroxy-4-methylcycloh exyl)amino)-6-((2-hydroxyethyl)amino)nic otinamide |
| 47 | T047 | 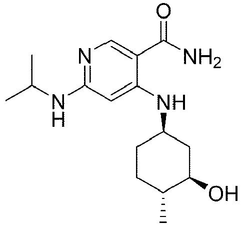 | 4-(((1*R*,3*R*,4*R*)-3-hydroxy-4-methylcycloh exyl)amino)-6-(isopropylamino)nicotinami de |
| 48 | T048 | 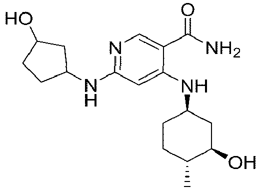 | 4-(((1*R*,3*R*,4*R*)-3-hydroxy-4-methylcycloh exyl)amino)-6-((3-hydroxycyclopentyl)ami no)nicotinamide |
| 49 | T049 | 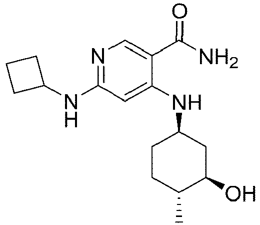 | 6-(cyclobutylamino)-4-(((1*R*,3*R*,4*R*)-3-hyd roxy-4-methylcyclohexyl)amino)nicotinam ide |
| 50 | T050 | 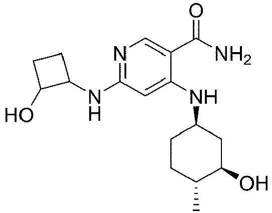 | 4-(((1*R*,3*R*,4*R*)-3-hydroxy-4-methylcycloh exyl)amino)-6-((2-hydroxycyclobutyl)ami no)nicotinamide |
| 51 | T051 | 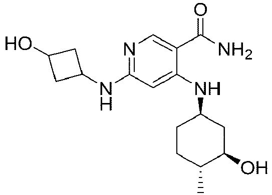 | 4-(((1*R*,3*R*,4*R*)-3-hydroxy-4-methylcycloh exyl)amino)-6-((3-hydroxycyclobutyl)ami no)nicotinamide |
| 52 | T052 | 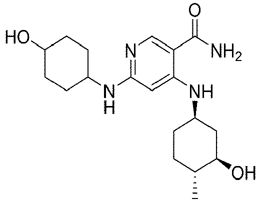 | 4-(((1*R*,3*R*,4*R*)-3-hydroxy-4-methylcycloh exyl)amino)-6-((4-hydroxycyclohexyl)ami no)nicotinamide |
| 53 | T053 | 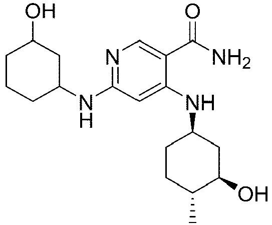 | 4-(((1*R*,3*R*,4*R*)-3-hydroxy-4-methylcycloh exyl)amino)-6-((3-hydroxycyclohexyl)ami no)nicotinamide |
| 54 | T054 | 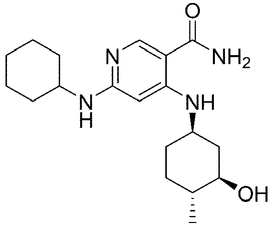 | 6-(cyclohexylamino)-4-(((1*R*,3*R*,4*R*)-3-hyd roxy-4-methylcyclohexyl)amino)nicotinam ide |
| 55 | T055 | 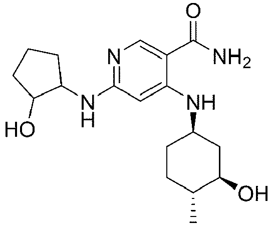 | 4-(((1*R*,3*R*,4*R*)-3-hydroxy-4-methylcycloh exyl)amino)-6-((2-hydroxycyclopentyl)ami no)nicotinamide |
| 56 | T056 | 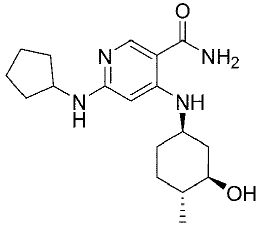 | 6-(cyclopentylamino)-4-(((1*R*,3*R*,4*R*)-3-hy droxy-4-methylcyclohexyl)amino)nicotina mide |
| 57 | T057 | 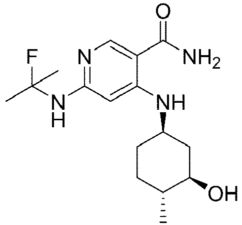 | 6-((2-fluoropropan-2-yl)amino)-4-(((1*R*,3*R* ,4*R*)-3-hydroxy-4-methylcyclohexyl)amino )nicotinamide |
| 58 | T058 | 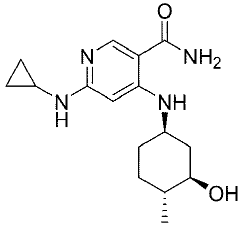 | 6-(cyclopropylamino)-4-(((1*R*,3*R*,4*R*)-3-hy droxy-4-methylcyclohexyl)amino)nicotina mide |
| 59 | T059 | 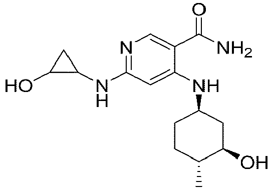 | 4-(((1*R*,3*R*,4*R*)-3-hydroxy-4-methylcycloh exyl)amino)-6-((2-hydroxycyclopropyl)am ino)nicotinamide |
| 60 | T060 | 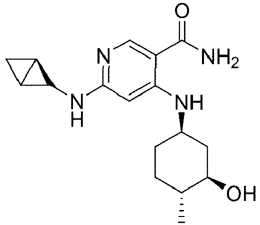 | 6-(((1*R*,2*R*)-bicyclo[1.1.0]butan-2-yl)amin o)-4-(((1*R*,3*R*,4*R*)-3-hydroxy-4-methylcycl ohexyl)amino)nicotinamide |
| 61 | T062 | 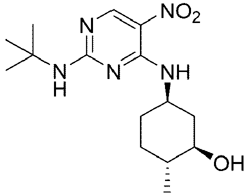 | (1*R*,2*R*,5*R*)-5-((2-(*tert*-butylamino)-5-nitro pyrimidin-4-yl)amino)-2-methylcyclohex-1-ol |
| 62 | T063 | 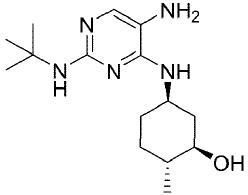 | (1*R*,2*R*,5*R*)-5-((5-amino-2-(*tert*-butylamin o)pyrimidin-4-yl)amino)-2-methylcyclohe xan-1-ol |
| 63 | T064 | 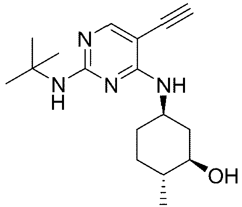 | (1R,2R, 5*R*)-5-((2-(*tert-*butylamino)-5 -ethy nylpyrimidin-4-yl)amino)-2-methylcyclohe x-1-ol |
| 64 | T065 | 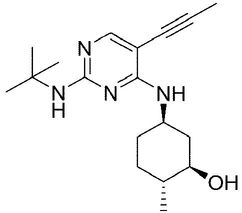 | (1*R,*2*R,*5*R*)*-*5*-*((2-(*tert*-butylamino)-5-(pro p-1-yn-1-yl)pyrimidin-4-yl)amino)-2-meth ylcyclohexan-1-ol |
| 65 | T066 | 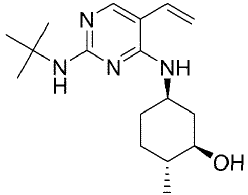 | (1*R*,2*R*,5*R*)-5-((2-(*tert*-butylamino)-5-vinyl pyrimidin-4-yl)amino)-2-methylcyclohex-1-ol |
| 66 | T067 | 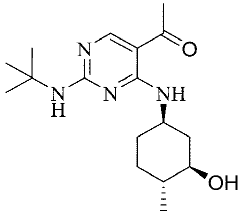 | 1-(2-(*tert*-butylamino)-4-(((1*R*,3*R*,4*R*)-3-h ydroxy-4-methylcyclohexyl)amino)pyrimi din-5-yl)ethan-1-one |
| 67 | T068 | 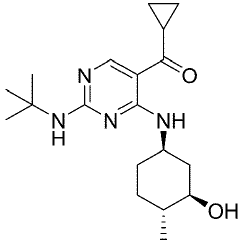 | (2-(*tert*-butylamino )-4-(((1*R*,3*R*,4*R*)-3-hyd roxy-4-methylcyclohexyl)amino)pyrimidin -5-yl)(cyclopropyl)methanone |
| 68 | T069 | 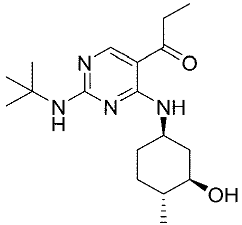 | 1-(2-(*tert*-butylamino)-4-(((1*R*,3*R*,4*R*)-3-h ydroxy-4-methylcyclohexyl)amino)pyrimi din-5-yl)propan-1-one |
| 69 | T070 | 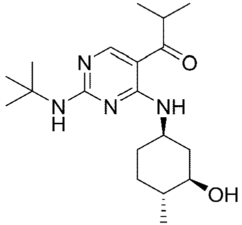 | 1-(2-(*tert*-butylamino)-4-(((1*R*,3*R*,4*R*)-3-h ydroxy-4-methylcyclohexyl)amino)pyrimi din-5-yl)-2-methylpropann-1-one |
| 70 | T071 | 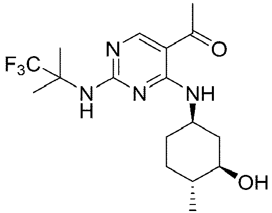 | 1-(4-(((1*R*,3*R*,4*R*)-3-hydroxy-4-methylcycl ohexyl)amino)-2-((1,1,1-trifluoro-2-methyl propan-2-yl)amino)pyrimidin-5-yl)ethan-1 -one |
| 71 | T072 | 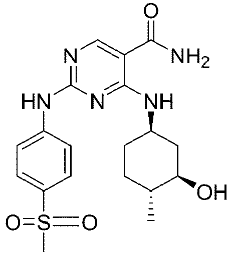 | 4-(((1*R*,3*R*,4*R*)-3-hydroxy-4-methylcycloh exyl)amino)-2-((4-(methylsulfonyl)phenyl) amino)pyrimidine-5-carboxamide |
| 72 | T073 | 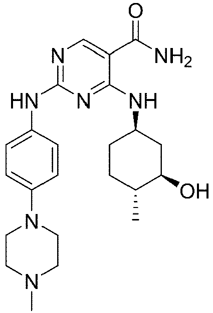 | 4-(((1*R*,3*R*,4*R*)-3-hydroxy-4-methylcycloh exyl)amino)-2-((4-(4-methylpiperazin-1-yl )phenyl)amino)pyrimidine-5-carboxamide |
| 73 | T074 | 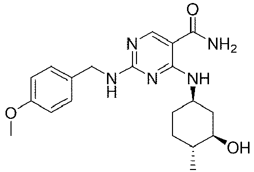 | 4-(((1*R*,3*R*,4*R*)-3-hydroxy-4-methylcycloh exyl)amino)-2-((4-methoxybenzyl)amino)p yrimidine-5-carboxamide |

The pharmaceutically acceptable salts of the compounds disclosed herein may be acid addition salts or base addition salts, and may be, for example, acid addition salts formed with inorganic or organic acids as follows: hydrochloric acid, sulfuric acid, nitric acid, phosphoric acid, formic acid, acetic acid, tartaric acid, fumaric acid, citric acid, malic acid, oxalic acid, ascorbic acid, succinic acid, methanesulfonic acid, ethanesulfonic acid, benzenesulfonic acid, etc., or may be salts formed with sodium ion, potassium ion, calcium ion, ammonium ion, etc. The compounds can be specified as follows.

The present disclosure also provides a method for preparing the compound of formula (I), which may be one of the following several schemes:

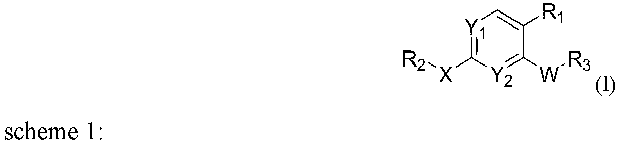

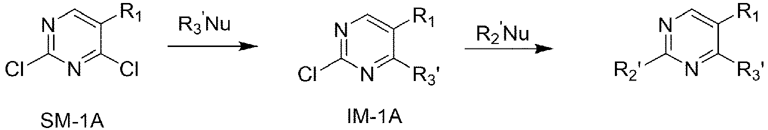

In this scheme, R2' is R₂-X, R₃' is R₃-W, Nu is a nucleophilic group, and R₁, R₂, R₃, X, and W are defined as in any of the embodiments above.

Scheme 1 comprises the following steps 1a and 1b:
(1a) subjecting SM-1A to a reaction with a nucleophilic reagent containing R₃' in the presence of a Lewis base to give IM-1A;
the above reaction conditions can be conventionally selected ones; for example, the Lewis base may be selected from any one or two or more of triethylamine, diisopropyl ethyl amine, pyridine, potassium carbonate, sodium carbonate, potassium *tert*-butoxide, sodium *tert*-butoxide, potassium hydroxide and sodium hydroxide, or an excess of the nucleophilic reagent containing R₃' is used as the Lewis base;
the reaction may be carried out in the presence or absence of a solvent; when a solvent is present, the solvent may be an organic solvent, and the organic solvent is preferably selected from one, two or more of methanol, ethanol, isopropanol, *tert*-butanol, acetonitrile, tetrahydrofuran, dioxane, dimethylformamide, dimethyl sulfoxide, acetone and dichloromethane;
the molar ratio of the compound SM-1A to the nucleophilic reagent containing R₃' can be 1:(0.8-10); the molar ratio of the compound SM-1A to the Lewis base can be 1:(0.8-5); the weight-volume ratio of the compound SM-1A to the organic solvent can be 1:(0-100) g/mL, and is preferably 1:(3-100) g/mL; the reaction can be carried out at -10 °C to 40 °C, preferably at -10 °C to 40 °C; the reaction time can be 1-24h.
(1b) subjecting IM-1A to a reaction with a nucleophilic reagent containing R₂ in the presence of a Lewis base to give a corresponding product or intermediate;
the above reaction conditions can be conventionally selected ones; for example, the Lewis base may be selected from any one or two or more of triethylamine, diisopropyl ethyl amine, pyridine, potassium carbonate, sodium carbonate, potassium *tert*-butoxide, sodium *tert*-butoxide, potassium hydroxide and sodium hydroxide, or an excess of the nucleophilic reagent containing R₂' is used as the Lewis base;
the reaction may be carried out in the presence or absence of a solvent; when a solvent is present, the solvent may be an organic solvent, and the organic solvent is preferably selected from one, two or more of methanol, ethanol, isopropanol, *tert*-butanol, acetonitrile, tetrahydrofuran, dioxane, dimethylformamide, dimethyl sulfoxide, acetone and dichloromethane;
the molar ratio of the compound SM-1A to the nucleophilic reagent containing R₂' can be 1:(0.8-10); the molar ratio of the compound SM-1A to the Lewis base can be 1:(0.8-5); the weight-volume ratio of the compound SM-1A to the organic solvent can be 1:(0-100) g/mL, and is preferably 1:(3-100) g/mL; the reaction can be carried out at 20 °C to 150 °C, preferably at 50 °C to 150 °C; the reaction time can be 1-24h.

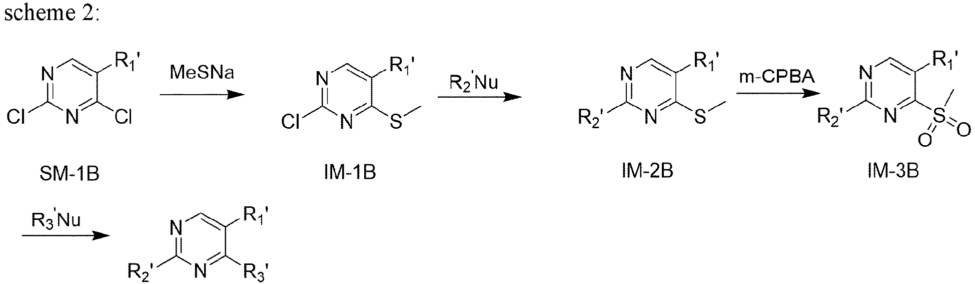

In this scheme, R₁' is R₁ or a group that can derive into R₁, R₂' is R₂-X, R₃' is R₃-W, Nu is a nucleophilic group, and R₁, R₂, R₃, X and W are defined as in any of the embodiments above.

Scheme 2 comprises the following steps 2a-2d:
(2a) subjecting SM-1B to a reaction with sodium thiomethoxide to give IM-1B, and optionally, carrying out derivatization reaction on R₁' in the IM-1B before proceeding to subsequent steps;
the above reaction conditions can be conventionally selected ones; for example, the molar ratio of the compound SM-1B to the sodium thiomethoxide can be 1:(0.8-5);
the reaction may be carried out in the presence or absence of a solvent; when a solvent is present, the solvent may be an organic solvent, and the organic solvent is preferably selected from one, two or more of methanol, ethanol, isopropanol, tert-butanol, acetonitrile, tetrahydrofuran, dioxane, dimethylformamide, dimethyl sulfoxide, acetone and dichloromethane;
the weight-volume ratio of the compound SM-1B to the organic solvent can be 1:(0-100) g/mL, and is preferably 1:(3-100) g/mL; the reaction can be carried out at -10 °C to 100 °C, preferably at -10 °C to 40 °C; the reaction time can be 1-24h.
(2b) subjecting IM-1B to a reaction with a nucleophilic reagent containing R₂ in the presence of a Lewis base to give IM-2B;
the above reaction conditions can be conventionally selected ones; for example, the Lewis base may be selected from any one or two or more of triethylamine, diisopropyl ethyl amine, pyridine, potassium carbonate, sodium carbonate, potassium *tert*-butoxide, sodium *tert*-butoxide, potassium hydroxide and sodium hydroxide, or an excess of the nucleophilic reagent containing R₂' is used as the Lewis base;
the reaction may be carried out in the presence or absence of a solvent; when a solvent is present, the solvent may be an organic solvent, and the organic solvent is preferably selected from one, two or more of methanol, ethanol, isopropanol, tert-butanol, acetonitrile, tetrahydrofuran, dioxane, dimethylformamide, dimethyl sulfoxide, acetone and dichloromethane;
the molar ratio of the compound SM-1B to the nucleophilic reagent containing R₂' can be 1:(0.8-10); the molar ratio of the compound SM-1B to the Lewis base can be 1:(0.8-5); the weight-volume ratio of the compound SM-1B to the organic solvent can be 1:(0-100) g/mL, and is preferably 1:(3-100) g/mL; the reaction can be carried out at 10 °C to 150 °C, preferably at 50 °C to 150 °C; the reaction time can be 1-24h.
(2c) subjecting IM-2B to a reaction with *m*-chloroperoxybenzoic acid to give IM-3B;
the above reaction conditions can be conventionally selected ones; for example, the molar ratio of the compound IM-2B to *m*-chloroperoxybenzoic acid can be 1:(0.8-10);
the reaction may be carried out in the presence or absence of a solvent; when a solvent is present, the solvent may be an organic solvent, and the organic solvent is preferably selected from one, two or more of methanol, ethanol, isopropanol, tert-butanol, acetonitrile, tetrahydrofuran, dioxane, dimethylformamide, dimethyl sulfoxide, acetone and dichloromethane;
the weight-volume ratio of the compound IM-2B to the organic solvent can be 1:(0-100) g/mL, and is preferably 1:(3-100) g/mL; the reaction can be carried out at -20 °C to 150 °C, preferably at -20 °C to 40 °C; the reaction time can be 1-24h.
(2d) subjecting IM-3B to a reaction with a nucleophilic reagent containing R₃' in the presence of a Lewis base to give a corresponding product or intermediate, and optionally, further carrying out a derivatization reaction;
the above reaction conditions can be conventionally selected ones; for example, the Lewis base may be selected from any one or two or more of triethylamine, diisopropyl ethyl amine, pyridine, potassium carbonate, sodium carbonate, potassium *tert*-butoxide, sodium *tert*-butoxide, potassium hydroxide and sodium hydroxide, or an excess of the nucleophilic reagent containing R₃' is used as the Lewis base;
the reaction may be carried out in the presence or absence of a solvent; when a solvent is present, the solvent may be an organic solvent, and the organic solvent is preferably selected from one, two or more of methanol, ethanol, isopropanol, tert-butanol, acetonitrile, tetrahydrofuran, dioxane, dimethylformamide, dimethyl sulfoxide, acetone and dichloromethane;
the molar ratio of the compound IM-3B to the nucleophilic reagent containing R₃' can be 1:(0.8-10); the molar ratio of the compound IM-3B to the Lewis base can be 1:(0.8-5); the weight-volume ratio of the compound IM-3B to the organic solvent can be 1:(0-100) g/mL, and is preferably 1:(3-100) g/mL; the reaction can be carried out at 20 °C to 150 °C, preferably at 50 °C to 150 °C; the reaction time can be 1-24h.

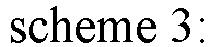

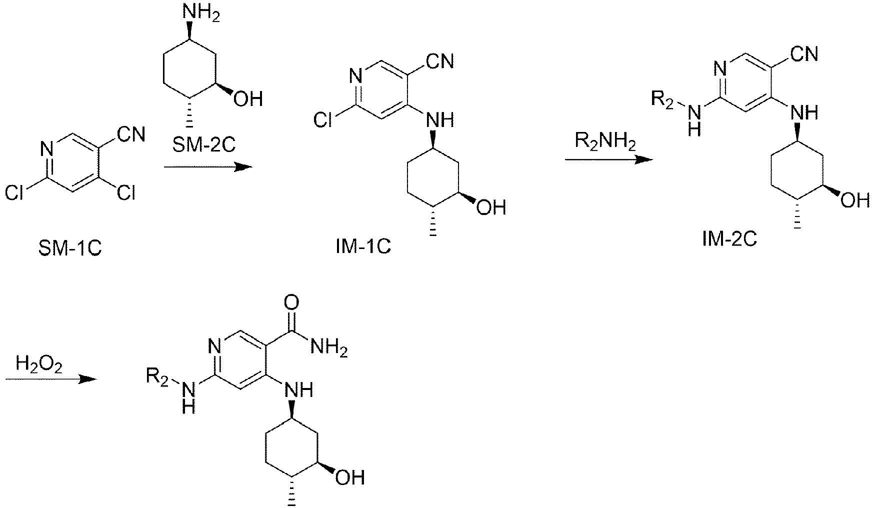

In this scheme, R₂ is defined as in any of the embodiments above.

Scheme 3 comprises the following steps 3a-3c:
(3a) subjecting SM-1C to a reaction with SM-2C in the presence of a Lewis base to give IM-1C;
the above reaction conditions can be conventionally selected ones; for example, the Lewis base may be selected from any one or two or more of triethylamine, diisopropyl ethyl amine, pyridine, potassium carbonate, sodium carbonate, potassium *tert*-butoxide, sodium *tert*-butoxide, potassium hydroxide and sodium hydroxide, or an excess of the SM-2C is used as the Lewis base;
the reaction may be carried out in the presence or absence of a solvent; when a solvent is present, the solvent may be an organic solvent, and the organic solvent is preferably selected from one, two or more of methanol, ethanol, isopropanol, tert-butanol, acetonitrile, tetrahydrofuran, dioxane, dimethylformamide, dimethyl sulfoxide, acetone and dichloromethane;
the molar ratio of the compound SM-1C to the SM-2C can be 1:(0.8-10); the molar ratio of the compound SM-1C to the Lewis base can be 1:(0.8-5); the weight-volume ratio of the compound SM-1C to the organic solvent can be 1:(0-100) g/mL, and is preferably 1:(3-100) g/mL; the reaction can be carried out at 10 °C to 150 °C, preferably at 25 °C to 120 °C; the reaction time can be 1-24h.
(3b) subjecting IM-1C to a reaction with R₂NH₂ in the presence of a Lewis base to give a corresponding product or intermediate;
the above reaction conditions can be conventionally selected ones; for example, the Lewis base may be selected from any one or two or more of triethylamine, diisopropylethylamine, pyridine, potassium carbonate, sodium carbonate, potassium *tert*-butoxide, sodium *tert*-butoxide, potassium hydroxide and sodium hydroxide, or an excess of the R₂NH₂ is used as the Lewis base;
the reaction may be carried out in the presence or absence of a solvent; when a solvent is present, the solvent may be preferably an organic solvent, and the organic solvent is preferably selected from one, two or more of methanol, ethanol, isopropanol, *tert*-butanol, acetonitrile, tetrahydrofuran, dioxane, dimethylformamide, dimethyl sulfoxide, acetone and dichloromethane;
the molar ratio of the compound IM-1C to the R₂NH₂ can be 1:(0.8-10); the molar ratio of the compound IM-1C to the Lewis base can be 1:(0.8-5); the weight-volume ratio of the compound IM-1C to the organic solvent can be 1:(0-100) g/mL, and is preferably 1:(3-100) g/mL; the reaction can be carried out at 20 °C to 150 °C, preferably at 50 °C to 150 °C; the reaction time can be 1-24h.
(3c) subjecting IM-2C to a reaction with H₂O₂ in the presence of a Lewis base to give a corresponding product;
the above reaction conditions can be conventionally selected ones; for example, the Lewis base may be selected from any one or two or more of triethylamine, diisopropylethylamine, pyridine, potassium carbonate, sodium carbonate, potassium *tert*-butoxide, sodium *tert*-butoxide, potassium hydroxide and sodium hydroxide;
the reaction may be carried out in the presence or absence of a solvent; when a solvent is present, the solvent may be an organic solvent, and the organic solvent is preferably selected from one, two or more of water, methanol, ethanol, isopropanol, tert-butanol, acetonitrile, tetrahydrofuran, dioxane, dimethylformamide, dimethyl sulfoxide, acetone and dichloromethane;
the molar ratio of the compound IM-2C to the H₂O₂ can be 1:(0.8-20); the molar ratio of the compound IM-2C to the Lewis base can be 1:(0.8-5); the weight-volume ratio of the compound IM-2C to the solvent can be 1:(0-100) g/mL, and is preferably 1:(3-100) g/mL; the reaction can be carried out at -78 °C to 100 °C, preferably at -78 °C to 20 °C; the reaction time can be 1-24h.

It will be understood by those skilled in the art that the compound of formula (I) and the racemate, the stereoisomer, the tautomer and the nitrogen oxide thereof can be used as a starting material or an intermediate to prepare pharmaceutically acceptable salts of the compound of formula (I) and the racemate, the stereoisomer, the tautomer and the nitrogen oxide thereof. Therefore, the present disclosure also provides use of the compound of formula (I) and the racemate, the stereoisomer, the tautomer and the nitrogen oxide thereof in preparing pharmaceutically acceptable salts of the compound of formula (I) and the racemate, the stereoisomer, the tautomer and the nitrogen oxide thereof.

The present disclosure also provides use of at least one of the compound of formula I and the racemate, the stereoisomer, the tautomer, the nitrogen oxide, the isotopically labeled compound, the solvate, the polymorph, the metabolite, the ester, the pharmaceutically acceptable salt or the prodrug thereof in preparing a medicament, wherein the medicament is an inhibitor of protein kinase. The medicament is used to treat, prevent or ameliorate disorders in an animal or human, including pulmonary fibrosis, hepatic fibrosis or the like. Furthermore, the compounds are active against protein kinases, in particular JNK1 and/or JNK2. The method provided herein comprises administering to a subject in need thereof an effective amount of the compound disclosed herein (including the compound of formula (I) and the racemate, the stereoisomer, the tautomer and the nitrogen oxide thereof, or the pharmaceutically acceptable salts thereof; the same applies hereinafter).

The present disclosure also provides a pharmaceutical composition comprising a therapeutically effective amount of the compound of formula (I) and the racemate, the stereoisomer, the tautomer, the nitrogen oxide, the isotopically labeled compound, the solvate, the polymorph, the metabolite, the ester, the pharmaceutically acceptable salt or the prodrug thereof. The pharmaceutical composition may optionally further comprise a pharmaceutically acceptable auxiliary material, such as a carrier or an excipient. As an example, the auxiliary material may be one or more selected from: a disintegrant, a glidant, a lubricant, a diluent, a filler, an adhesive and a colorant.

The present disclosure also provides a method for modulating JNK kinase function, which comprises administering to an individual in need thereof an effective amount of one or more compounds disclosed herein or a pharmaceutical composition comprising the compounds.

In yet another aspect, the present disclosure provides a method for inhibiting a kinase in a cell expressing said kinase, which comprises contacting said cell with an effective amount of the compound disclosed herein. In one embodiment, the kinase is JNK1, JNK2, or a mutant or an isoform thereof, or a combination thereof, and the compound is one or more compounds of formula (I), e.g., a compound in Table 1.

In another aspect, provided herein is a method for treating or preventing a hepatic fibrosis disease, such as non-alcoholic steatohepatitis, steatosis (i.e., fatty liver), cirrhosis, primary sclerosing cholangitis, primary biliary cirrhosis, hepatitis, hepatocellular carcinoma, and hepatic fibrosis accompanied by long-term or repeated alcohol intake (alcoholic hepatitis), accompanied by an infection (e.g., viral infection, such as HCV), accompanied by liver transplantation or accompanied by drug-induced liver injury (e.g., acetaminophen toxicity), and the method comprises administering to a subject in need thereof an effective amount of the compound disclosed herein. In some aspects, provided herein is a method for treating or preventing diabetes or metabolic syndrome resulting in hepatic or pulmonary fibrosis diseases, such as non-alcoholic steatohepatitis, steatosis (i.e., fatty liver), cirrhosis, primary sclerosing cholangitis, primary biliary cirrhosis and hepatitis, and the method comprises administering to a subject in need thereof an effective amount of the compound disclosed herein.

In another aspect, provided herein is a method for treating or preventing one or more disorders selected from idiopathic pulmonary fibrosis (IPF), systemic sclerosis, scleroderma, chronic allograft nephropathy, antibody-mediated rejection and lupus, and the method comprises administering to a subject in need thereof an effective amount of the compound disclosed herein. In some such embodiments, the lupus is lupus erythematosus (e.g., discoid lupus erythematosus or cutaneous lupus erythematosus) or systemic lupus.

In another aspect, provided herein is a method for treating or preventing a disorder treatable or preventable by inhibiting JNK1 and/or JNK2, and the method comprises administering to a subject in need thereof an effective amount of the compound disclosed herein. Examples of such a disorder include rheumatoid arthritis; rheumatoid spondylitis; osteoarthritis; asthma; bronchitis; allergic rhinitis; chronic obstructive pulmonary disease; cystic fibrosis; inflammatory bowel disease; irritable bowel syndrome; mucous colitis; ulcerative colitis; Crohn's disease; Huntington's disease; hepatitis; pancreatitis; nephritis; multiple sclerosis; lupus erythematosus; type II diabetes; obesity; atherosclerosis; post-angioplasty restenosis; left ventricular hypertrophy; myocardial infarction; stroke; ischemic injury of the heart, lung, intestine, kidney, liver, pancreas, spleen and brain; acute or chronic organ transplant rejection; preservation of organs for transplantation; organ failure or loss of limb (e.g., including but not limited to those resulting from ischemia-reperfusion injury, trauma, gross bodily injury, car accident, crush injury or transplantation failure); graft versus host disease; endotoxic shock; multiple organ failure; psoriasis; burns caused by exposure to fire, chemicals or radiation; eczema; dermatitis; skin grafting; ischemia; ischemic disorders associated with surgery or traumatic injury (e.g., vehicular accident, gunshot wound or limb crush); epilepsy; Alzheimer's disease; Parkinson's disease; immune response to bacterial or viral infection; cachexia; angiogenic and proliferative diseases; solid tumor; and cancers of various tissues, such as colon, rectum, prostate, liver, lung, bronchus, pancreas, brain, head, neck, stomach, skin, kidney, cervix, blood, larynx, esophagus, mouth, pharynx, bladder, ovary or uterus.

In another aspect, provided herein is a compound of formula (I) for use in modulating JNK kinase function, and the modulating comprises administering to an individual in need thereof an effective amount of one or more compounds disclosed herein or a pharmaceutical composition comprising the compounds.

In yet another aspect, the present disclosure provides a compound of formula (I) for use in a method for inhibiting a kinase in a cell expressing said kinase, and the method comprises contacting said cell with an effective amount of the compound disclosed herein. In one embodiment, the kinase is JNK1, JNK2, or a mutant or an isoform thereof, or a combination thereof, and the compound is one or more compounds of formula (I), e.g., a compound in Table 1.

In yet another aspect, provided herein is a compound of formula (I) for use in a method for treating or preventing a hepatic fibrosis disease, such as non-alcoholic steatohepatitis, steatosis (i.e., fatty liver), cirrhosis, primary sclerosing cholangitis, primary biliary cirrhosis, hepatitis, hepatocellular carcinoma, and hepatic fibrosis accompanied by long-term or repeated alcohol intake (alcoholic hepatitis), accompanied by an infection (e.g., viral infection, such as HCV), accompanied by liver transplantation or accompanied by drug-induced liver injury (e.g., acetaminophen toxicity), and the method comprises administering to a subject in need thereof an effective amount of the compound disclosed herein. In some aspects, provided herein is a method for treating or preventing diabetes or metabolic syndrome resulting in hepatic or pulmonary fibrosis diseases, such as non-alcoholic steatohepatitis, steatosis (i.e., fatty liver), cirrhosis, primary sclerosing cholangitis, primary biliary cirrhosis and hepatitis, and the method comprises administering to a subject in need thereof an effective amount of the compound disclosed herein.

In yet another aspect, provided herein is a compound of formula (I) for use in a method for treating or preventing one or more disorders selected from idiopathic pulmonary fibrosis (IPF), systemic sclerosis, scleroderma, chronic allograft nephropathy, antibody-mediated rejection and lupus, and the method comprises administering to a subject in need thereof an effective amount of the compound disclosed herein. In some such embodiments, the lupus is lupus erythematosus (e.g., discoid lupus erythematosus or cutaneous lupus erythematosus) or systemic lupus.

In yet another aspect, provided herein is a compound of formula (I) for use in a method for treating or preventing a disorder treatable or preventable by inhibiting JNK1 and/or JNK2, and the method comprises administering to a subject in need thereof an effective amount of the compound disclosed herein. Examples of such a disorder include rheumatoid arthritis; rheumatoid spondylitis; osteoarthritis; asthma; bronchitis; allergic rhinitis; chronic obstructive pulmonary disease; cystic fibrosis; inflammatory bowel disease; irritable bowel syndrome; mucous colitis; ulcerative colitis; Crohn's disease; Huntington's disease; hepatitis; pancreatitis; nephritis; multiple sclerosis; lupus erythematosus; type II diabetes; obesity; atherosclerosis; post-angioplasty restenosis; left ventricular hypertrophy; myocardial infarction; stroke; ischemic injury of the heart, lung, intestine, kidney, liver, pancreas, spleen and brain; acute or chronic organ transplant rejection; preservation of organs for transplantation; organ failure or loss of limb (e.g., including but not limited to those resulting from ischemia-reperfusion injury, trauma, gross bodily injury, car accident, crush injury or transplantation failure); graft versus host disease; endotoxic shock; multiple organ failure; psoriasis; burns caused by exposure to fire, chemicals or radiation; eczema; dermatitis; skin grafting; ischemia; ischemic disorders associated with surgery or traumatic injury (e.g., vehicular accident, gunshot wound or limb crush); epilepsy; Alzheimer's disease; Parkinson's disease; immune response to bacterial or viral infection; cachexia; angiogenic and proliferative diseases; solid tumor; and cancers of various tissues, such as colon, rectum, prostate, liver, lung, bronchus, pancreas, brain, head, neck, stomach, skin, kidney, cervix, blood, larynx, esophagus, mouth, pharynx, bladder, ovary or uterus.

### Definitions and Description

Unless otherwise stated, the definitions of groups and terms described in the specification and claims of the present application, including definitions thereof as examples, exemplary definitions, preferred definitions, definitions documented in tables, definitions of specific compounds in the examples, and the like, may be arbitrarily combined and incorporated with each other. The definitions of groups and the structures of the compounds in such combinations and incorporations should fall within the scope of the present specification.

When a numerical range defined by "integer" is recited in the specification and claims of this application, it shall be construed as reciting both endpoints of the range and every integer within the range. For example, "an integer of 0-10" shall be construed to include every integer of 0, 1, 2, 3, 4, 5, 6, 7, 8, 9 and 10. When the numerical range is defined as "numbers", it shall be construed as including both endpoints of the range, each integer within the range, and each decimal within the range. For example, "numbers of 0-10" shall be construed as including not only each of integers 0, 1, 2, 3, 4, 5, 6, 7, 8, 9 and 10, but also at least the sums of each integer and 0.1, 0.2, 0.3, 0.4, 0.5, 0.6, 0.7, 0.8 or 0.9.

Unless otherwise stated, "the compound of the present disclosure" or "the compound disclosed herein", as used herein, is intended to encompass a triazole derivative of formula (I) and a racemate, a stereoisomer, a tautomer or a nitrogen oxide thereof, or pharmaceutically acceptable salts thereof.

The term "halogen" refers to F, Cl, Br and I. In other words, F, Cl, Br and I may be described as "halogen" in the specification.

The term "C₁₋₄₀ alkyl" preferably refers to a linear or branched saturated monovalent hydrocarbyl having 1-40 carbon atoms, and is preferably C₁₋₁₀ alkyl and C₁₋₆ alkyl. "C₁₋₁₀ alkyl" preferably refers to a linear or branched saturated monovalent hydrocarbyl having 1, 2, 3, 4, 5, 6, 7, 8, 9 or 10 carbon atoms. "C₁₋₆ alkyl" preferably refers to a linear or branched saturated monovalent hydrocarbyl having 1, 2, 3, 4, 5 or 6 carbon atoms. The alkyl is, for example, methyl, ethyl, propyl, butyl, pentyl, hexyl, isopropyl, isobutyl, *sec*-butyl, tert-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl or 1,2-dimethylbutyl, or isomers thereof. In particular, the group has 1, 2, 3, 4, 5 or 6 carbon atoms (i.e., "C₁₋₆ alkyl"), such as methyl, ethyl, propyl, butyl, isopropyl, isobutyl, sec-butyl or tert-butyl; more particularly, the group has 1, 2 or 3 carbon atoms (i.e., "C₁₋₃ alkyl"), such as methyl, ethyl, *n*-propyl or isopropyl.

The term "C₁₋₄₀ alkoxy" refers to the group -OR, wherein R is a substituted or unsubstituted C₁₋₄₀ alkyl, wherein "C₁₋₄₀ alkyl" is defined as above. Similarly, the term "C₁₋₁₀ alkoxy" refers to the group -OC₁₋₁₀ alkyl, "C₁₋₆ alkoxy" refers to the group -OC₁₋₆ alkyl, and "C₁₋₃ alkoxy" refers to the group -OC₁₋₃ alkyl, wherein "C₁₋₁₀ alkyl", "C₁₋₆ alkyl" and "C₁₋₃ alkyl" are defined as above. Specifically, the alkoxy includes, but is not limited to, methoxy, ethoxy, *n*-propoxy, isopropoxy, *n*-butoxy, *tert*-butoxy, *sec*-butoxy, *n*-pentoxy, *n*-hexyloxy and 1,2-dimethylbutoxy.

The term "C₂₋₄₀ alkenyl" preferably refers to a linear or branched monovalent hydrocarbyl comprising one or more double bonds and having 2-40 carbon atoms, and is preferably "C₂₋₁₀ alkenyl". "C₂₋₁₀ alkenyl" preferably refers to a linear or branched monovalent hydrocarbyl comprising one or more double bonds and having 2, 3, 4, 5, 6, 7, 8, 9 or 10 carbon atoms, for example, having 2, 3, 4, 5 or 6 carbon atoms (i.e., C₂₋₆ alkenyl) or having 2 or 3 carbon atoms (i.e., C₂₋₃ alkenyl). It should be understood that in the case that the alkenyl comprises more than one double bond, the double bonds can be separated from one another or conjugated. The alkenyl is, for example, vinyl, allyl, (E)-2-methylvinyl, (Z)-2-methylvinyl, (E)-but-2-enyl, (Z)-but-2-enyl, (*E*)-but-1-enyl, (*Z*)-but-1-enyl, pent-4-enyl, (E)-pent-3-enyl, (Z)-pent-3-enyl, (E)-pent-2-enyl, (Z)-pent-2-enyl, (*E*)-pent-1-enyl, (*Z*)-pent-1-enyl, hex-5-enyl, (E)-hex-4-enyl, (Z)-hex-4-enyl, (E)-hex-3-enyl, (Z)-hex-3-enyl, (E)-hex-2-enyl, (Z)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (E)-2-methylbut-2-enyl, (Z)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, *(*E)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethylprop-1-enyl, 1-propylvinyl or 1-isopropylvinyl.

The term "C₂₋₄₀ alkynyl" refers to a linear or branched monovalent hydrocarbyl comprising one or more triple bonds and having 2-40 carbon atoms, and is preferably "C₂-C₁₀ alkynyl". The term "C₂-₁₀ alkynyl" preferably refers to a linear or branched monovalent hydrocarbyl comprising one or more triple bonds and having 2, 3, 4, 5, 6, 7, 8, 9 or 10 carbon atoms, for example having 2, 3, 4, 5 or 6 carbon atoms (i.e., "C₂-₆ alkynyl") or having 2 or 3 carbon atoms ("C₂-₃ alkynyl"). The alkynyl is, for example, ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl or 3,3-dimethylbut-1-ynyl. In particular, the alkynyl is ethynyl, prop-1-ynyl or prop-2-ynyl.

The term "C₃₋₂₀ cycloalkyl" refers to a saturated monovalent monocyclic or bicyclic hydrocarbon ring which may be a spiro or bridged ring, and it has 3-20 carbon atoms and is preferably "C₃₋₁₀ cycloalkyl". The term "C₃₋₁₀ cycloalkyl" refers to a saturated monovalent monocyclic or bicyclic hydrocarbon ring which may be a spiro or bridged ring, and it has 3, 4, 5, 6, 7, 8, 9 or 10 carbon atoms. The C₃₋₁₀ cycloalkyl may be a monocyclic hydrocarbyl such as cyclopropyl, cyclobutyl, cyclopentyl, cyclohexyl, cycloheptyl, cyclooctyl, cyclononyl or cyclodecyl, or may be a bicyclic hydrocarbyl such as a decahydronaphthalene ring. The term "C₃₋₆ cycloalkyl" refers to a saturated monovalent monocyclic or bicyclic hydrocarbon ring which may be a spiro or bridged ring, and it has 3, 4, 5 or 6 carbon atoms. The C₃₋₆ cycloalkyl group may be, for example, cyclopropyl, cyclobutyl, cyclopentyl, cyclohexyl, bicyclo[1.1.0]butyl, spiropentyl, spiro[2.3]hexyl, bicyclo[1.1.1]pentyl, bicyclo[2.1.0]pentyl, bicyclo[2.1.1]hexyl or bicyclo[3.1.0]hexyl.

The term "3-20 membered heterocyclyl" refers to a monovalent 3-20 membered non-aromatic ring having ring carbon atoms and 1-5 ring heteroatoms, wherein the non-aromatic ring may be saturated or contain one or more double bonds, and it may be monocyclic, bicyclic, spiro or bridged ring, wherein each ring heteroatom is independently selected from N, O, S, B, P and Si. The "3-20-membered heterocyclyl" may be, for example, "3-10-membered heterocyclyl", "3-7-membered heterocyclyl" or "5-6-membered heterocyclyl". The term "3-10 membered heterocyclyl" refers to a monovalent 3-10 membered non-aromatic ring having ring carbon atoms and 1-5 ring heteroatoms, wherein the non-aromatic ring may be monocyclic, bicyclic, spiro or bridged ring, and each ring heteroatom is independently selected from N, O, S, B, P and Si; preferably, the 3-10 membered heterocyclyl comprises 1-3 heteroatoms selected from N, O and S. The term "3-7 membered heterocyclyl" refers to a monovalent 3-7 membered non-aromatic ring having ring carbon atoms and 1-3 ring heteroatoms, wherein the non-aromatic ring may be monocyclic, bicyclic, spiro or bridged ring, and each ring heteroatom is independently selected from N, O, S, B, P and Si; preferably, the 3-7 membered heterocyclyl comprises 1-3 heteroatoms selected from N, O and S. The term "5-6 membered heterocyclyl" refers to a monovalent 5-6 membered non-aromatic ring having ring carbon atoms and 1-3 ring heteroatoms, wherein the non-aromatic ring is usually monocyclic, and each ring heteroatom is independently selected from N, O, S, B, P and Si; preferably, the 5-6 membered heterocyclyl comprises 1-3 heteroatoms selected from N, O and S. The heterocyclyl may be connected to the rest of the molecule through any one of the carbon atoms or the nitrogen atom (if present). Each atom of the heterocyclyl is independently optionally substituted, e.g., with 1-5 substituents, 1-3 substituents or 1 substituent, regardless of whether the heterocyclyl is modified by "substituted" or not, and suitable substituents include, but are not limited to, hydroxy, amino, oxo, halogen, cyano, nitro, C₁₋₄₀ alkyl, C₂₋₄₀ alkenyl, C₂₋₄₀ alkynyl, and the like. In particular, the heterocyclyl may include, but is not limited to: 3 membered rings, such as azirdinyl, oxiranyl and thiorenyl; 4 membered rings, such as azetidinyl, oxetanyl and thietanyl; 5 membered rings, such as dihydrofuranyl, tetrahydrofuranyl, dihydrothienyl, tetrahydrothienyl, dioxolyl, pyrrolidinyl, dihydropyrrolyl, imidazolidinyl, pyrazolidinyl, pyrrolinyl, dioxolanyl, oxasulfuranyl, disulfuranyl, oxazolidin-2-one, triazolinyl, oxadiazolinyl, and thiadiazolinyl; or 6 membered rings, such as dihydropyranyl, tetrahydropyranyl, piperidinyl, morpholinyl, dihydropyridinyl, thiacyclohexyl, dithianyl, thiomorpholinyl, piperazinyl, dithiacyclohexyl, dioxanyl and trithianyl; or 7 membered rings, such as diazepanyl, azepanyl, oxepanyl and thiepanyl. The heterocyclyl is bicyclic, such as but not limited to a 5,5 membered ring, e.g., a hexahydrocyclopenta[c]pyrrol-2(1*H*)-yl ring, or a 5,6 membered bicyclic ring, e.g., a hexahydropyrrolo[1,2-*a*]pyrazin-2(1*H*)-yl ring. The ring containing nitrogen atoms may be partially unsaturated, i.e., it may comprise one or more double bonds, such as but not limited to 2,5-dihydro-1*H*-pyrrolyl, 4*H*-[1,3,4]thiadiazinyl, 4,5-dihydrooxazolyl or 4*H*-[1,4]thiazinyl, or it may be benzo-fused, such as but not limited to dihydroisoquinolyl.

The term "C₆₋₂₀ aryl" preferably refers to an aromatic or partially aromatic monovalent monocyclic, bicyclic or tricyclic hydrocarbon ring having 6-20 carbon atoms, and is preferably "C₆₋₁₄ aryl". The term "C₆₋₁₄ aryl" preferably refers to an aromatic or partially aromatic monovalent monocyclic, bicyclic or tricyclic hydrocarbon ring having 6, 7, 8, 9, 10, 11, 12, 13 or 14 carbon atoms ("C₆₋₁₄ aryl"), in particular a ring having 6 carbon atoms ("C₆ aryl"), such as phenyl or biphenyl, a ring having 9 carbon atoms ("C₉ aryl"), such as indanyl or indenyl, a ring having 10 carbon atoms ("C₁₀ aryl"), such as tetrahydronaphthyl, dihydronaphthyl or naphthyl, a ring having 13 carbon atoms ("C₁₃ aryl"), such as fluorenyl, or a ring having 14 carbon atoms ("C₁₄ aryl"), such as anthryl.

The term "5-20 membered heteroaryl" refers to a monovalent aromatic monocyclic, bicyclic or tricyclic ring having 5-20 ring atoms, and comprising 1-5 heteroatoms independently selected from N, O and S, and is, for example, "5-14 membered heteroaryl". The term "5-14 membered heteroaryl" refers to a monovalent aromatic monocyclic, bicyclic or tricyclic ring having 5, 6, 7, 8, 9, 10, 11, 12, 13 or 14 ring atoms, in particular 5, 6, 9 or 10 carbon atoms, and comprising 1-5, preferably 1-3 heteroatoms independently selected from N, O and S, and it may be benzo-fused in each case. The term "5-6 membered heteroaryl" refers to a monovalent monocyclic aromatic ring having 5 or 6 ring atoms, and comprising 1-3 heteroatoms each independently selected from N, O and S, and it may be benzo-fused in each case. In particular, the heteroaryl is selected from thienyl, furyl, pyrrolyl, oxazolyl, thiazolyl, imidazolyl, pyrazolyl, isoxazolyl, isothiazolyl, oxadiazolyl, triazolyl, thiadiazolyl, thia-4*H*-pyrazolyl and the like and benzo derivatives thereof, such as benzofuranyl, benzothienyl, benzoxazolyl, benzoisoxazolyl, benzimidazolyl, benzotriazolyl, indazolyl, indolyl, , isoindolyl, and the like; or pyridinyl, pyridazinyl, pyrimidinyl, pyrazinyl, triazinyl and the like and benzo derivatives thereof, such as quinolyl, quinazolinyl, isoquinolyl, and the like; or azocinyl, indolizinyl, purinyl and the like and benzo derivatives thereof; or cinnolinyl, phthalazinyl, quinazolinyl, quinoxalinyl, naphthyridinyl, pteridinyl, carbazolyl, acridinyl, phenazinyl, phenothiazinyl, phenoxazinyl, and the like.

Unless otherwise stated, the heterocyclyl, heteroaryl or heteroarylene includes all possible isomeric forms thereof, e.g., positional isomers thereof. Accordingly, for some illustrative non-limiting examples, pyridinyl or pyridinylene includes pyridin-2-yl, pyridinylene-2-yl, pyridin-3-yl, pyridinylene-3-yl, pyridin-4-yl, and pyridinylene-4-yl; thienyl or thienylene includes thien-2-yl, thien-2-ylene, thien-3-yl, and thien-3-ylene.

The above definition for the term "alkyl", such as "C₁₋₄₀ alkyl", is also applicable to other terms containing "C₁₋₄₀ alkyl", such as the terms "C₁₋₄₀ alkoxy", "C₁₋₄₀ alkylsilyl" and "C₁₋₄₀ alkylsilyloxy". Likewise, the above definitions of the terms "C₂₋₄₀ alkenyl", "C₂₋₄₀ alkynyl", "C₃₋₂₀ cycloalkyl", "C₅₋₂₀ cycloalkenyl", "3-20 membered heterocyclyl", "C₆₋₂₀ aryl" and "5-20 membered heteroaryl" are also applicable to other terms containing the same, such as the terms "C₂₋₄₀ alkenyloxy", "C₂₋₄₀ alkynyloxy", "C₃₋₂₀ cycloalkyloxy", "3-20 membered heterocyclyl", "3-20 membered heterocyclyloxy", "C₆₋₂₀ aryloxy", "C₆₋₂₀ arylalkyl" and "5-20 membered heteroarylalkyl".

Unless otherwise indicated, the term "leaving group", as used herein, shall refer to a charged or uncharged atom or group that is liberated during a substitution or replacement reaction. Suitable examples include, but are not limited to, H, F, Br, Cl, I, mesylate group, tosylate group, and the like.

In any method for preparing the compound disclosed herein, it may be necessary and/or desirable to protect sensitive or reactive groups on any molecule concerned. This can be achieved by conventional protective groups, as described in textbooks or in reference books in the art. The protective group may be removed at a convenient subsequent stage using methods known in the art. Those skilled in the art will recognize that, other reagents, including but not limited to Pd/C, Pd(OH)₂, PdCl₂, Pd(OAc)₂/Et₃SiH, Raney nickel, an appropriately selected acid, an appropriately selected base, fluoride, and the like, may be used in this deprotection step depending on the particular protective group.

According to the preparation method of the present disclosure, "optionally further" means that the subsequent step or operation, as required, may or may not be carried out. For example, "optionally further salifying" means that the salifying step may or may not be carried out. Those skilled in the art will be able to determine whether to perform the step or operation as required for the target compound.

The target compound may be isolated according to known methods, for example by extraction, filtration or column chromatography.

According to the structure, the compounds disclosed herein may be chiral and may therefore exist in various enantiomeric forms. These compounds may therefore exist in racemic or optically active form. The compounds disclosed herein or intermediates thereof may be separated into enantiomers by chemical or physical methods well known to those skilled in the art, or used in this form for synthesis. In the case of racemic amines, diastereoisomers are prepared from mixtures by reaction with optically active resolving agents. Examples of suitable resolving agents are optically active acids such as R- or *S*-tartaric acid, diacetyltartaric acid, dibenzoyltartaric acid, mandelic acid, malic acid, lactic acid, suitable *N*-protected amino acids (e.g., *N*-benzoylproline or *N*-benzenesulfonylproline) or various optically active camphorsulfonic acids. Enantiomeric resolution by chromatography can be advantageously performed with the aid of optically active resolving agents, such as dinitrobenzoylphenylglycine, cellulose triacetate or other carbohydrate derivatives or chirally derivatized methacrylate polymers immobilized on silica gel. Suitable eluents for this purpose are mixtures of solvent containing water or alcohol, for example, hexane/isopropanol/acetonitrile.

Those skilled in the art will appreciate that not all nitrogen-containing heterocycles can form N-oxides, as nitrogen needs to have available lone pairs of electrons used for oxidation to oxides; those skilled in the art will identify nitrogen-containing heterocycles capable of forming N-oxides. Those skilled in the art will also recognize that tertiary amines are capable of forming N-oxides. Synthetic methods for preparing N-oxides of heterocycles and tertiary amines are well known to those skilled in the art and include oxidation by peroxy acids such as peroxyacetic acid and *m*-chloroperoxybenzoic acid (MCPBA), hydrogen peroxide, alkyl hydroperoxides such as tert-butyl hydroperoxide, sodium perborate, and dioxiranes such as dimethyldioxirane. These methods for preparing N-oxides have been widely described and reviewed in the literature.

A pharmaceutically acceptable salt may be, for example, acid addition salts of the compounds disclosed herein having a nitrogen atom in the chain or ring with sufficient basicity, for example, acid addition salts formed with the following inorganic acids: hydrochloric acid, hydrofluoric acid, hydrobromic acid, hydroiodic acid, sulfuric acid, pyrosulfuric acid, phosphoric acid or nitric acid; hydrosulfates; or acid addition salts with the following organic acids: formic acid, acetic acid, acetoacetic acid, pyruvic acid, trifluoroacetic acid, propionic acid, butyric acid, hexanoic acid, heptanoic acid, undecanoic acid, lauric acid, benzoic acid, salicylic acid, 2-(4-hydroxybenzoyl)benzoic acid, camphoric acid, cinnamic acid, cyclopentanepropionic acid, digluconic acid, 3-hydroxy-2-naphthoic acid, nicotinic acid, pamoic acid, pectinic acid, peroxosulfuric acid, 3-phenylpropionic acid, picric acid, pivalic acid, 2-hydroxyethanesulfonic acid, itaconic acid, sulfamic acid, trifluoromethanesulfonic acid, dodecylsulfuric acid, ethanesulfonic acid, benzenesulfonic acid, *p*-toluenesulfonic acid, methanesulfonic acid, 2-naphthalenesulfonic acid, naphthalenedisulfonic acid, camphorsulfonic acid, citric acid, tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid, glucoheptoic acid, glycerophosphoric acid, aspartic acid, sulfosalicylic acid, hemisulfuric acid, or thiocyanic acid.

In addition, another suitable pharmaceutically acceptable salt of the compounds disclosed herein having sufficient acidity is an alkali metal salt (e.g., sodium salt or potassium salt), an alkaline earth metal salt (e.g., calcium salt or magnesium salt), an ammonium salt, or a salt formed with an organic base which provides a physiologically acceptable cation, for example a salt formed with: a sodium ion, a potassium ion, *N*-methylglucamine, dimethylglucamine, ethylglucamine, lysine, dicyclohexylamine, 1,6-hexanediamine, ethanolamine, glucosamine, meglumine, sarcosine, serinol, trihydroxymethylaminomethane, aminopropanediol, or 1-amino-2,3,4-butanetriol. As an example, the pharmaceutically acceptable salts include salts formed by the group -COOH with the following: a sodium ion, a potassium ion, a calcium ion, a magnesium ion, *N*-methylglucamine, dimethylglucamine, ethylglucamine, lysine, dicyclohexylamine, 1,6-hexanediamine, ethanolamine, glucosamine, meglumine, sarcosine, serinol, trihydroxymethylaminomethane, aminopropanediol and 1-amino-2,3,4-butanetriol; when 1, 2 or 3 of M₁, M₂ and M₃ of the present disclosure is H, pharmaceutically acceptable salts of the present disclosure include, for example, salts formed by -OP(O)(OM₁)(OM₂), -P(O)(OM₁)(OM₂), -OS(O)₂OM₃ and -S(O)₂OM₃ with the following: a sodium ion, a potassium ion, a calcium ion, a magnesium ion, *N*-methylglucamine, dimethylglucamine, ethylglucamine, lysine, dicyclohexylamine, 1,6-hexanediamine, ethanolamine, glucosamine, meglumine, sarcosine, serinol, trihydroxymethylaminomethane, aminopropanediol and 1-amino-2,3,4-butanetriol.

In addition, the basic nitrogen-containing groups may be quaternized with the following agents: lower alkyl halides such as methyl, ethyl, propyl and butyl chlorides, bromides and iodides; dialkyl sulfates such as dimethyl sulfate, diethyl sulfate, dibutyl sulfate, and dipentyl sulfate; long chain halides such as decyl, lauryl, myristyl and stearyl chlorides, bromides and iodides; and aralkyl halides such as benzyl and phenethyl bromides. As an example, pharmaceutically acceptable salts include hydrochloride, sulfate, nitrate, bisulfate, hydrobromide, acetate, oxalate, citrate, mesylate, formate, meglumine, and the like.

Since the compounds disclosed herein may have a plurality of salt-forming sites, the "pharmaceutically acceptable salt" includes not only a salt formed at 1 salt-forming site of the compounds disclosed herein but also salts formed at 2, 3 or all of the salt-forming sites thereof. For this purpose, the molar ratio of the compound of formula (I) to a radical ion (anion) of an acid or a cation of a base required for salt formation may vary within a wide range, and may be, for example, 4:1 to 1:4, such as 3:1, 2:1, 1:1, 1:2 or 1:3.

According to the present disclosure, the pharmaceutically acceptable anions include anions selected from those generated by the ionization of inorganic or organic acids. The "inorganic acid" includes, but is not limited to, hydrochloric acid, hydrofluoric acid, hydrobromic acid, hydroiodic acid, sulfuric acid, pyrosulfuric acid, phosphoric acid, or nitric acid. The "organic acid" includes, but is not limited to, formic acid, acetic acid, acetoacetic acid, pyruvic acid, trifluoroacetic acid, propionic acid, butyric acid, hexanoic acid, heptanoic acid, undecanoic acid, lauric acid, benzoic acid, salicylic acid, 2-(4-hydroxybenzoyl)benzoic acid, camphoric acid, cinnamic acid, cyclopentanepropionic acid, digluconic acid, 3-hydroxy-2-naphthoic acid, nicotinic acid, pamoic acid, pectinic acid, peroxosulfuric acid, 3-phenylpropionic acid, picric acid, pivalic acid, 2-hydroxyethanesulfonic acid, itaconic acid, sulfamic acid, trifluoromethanesulfonic acid, dodecylsulfuric acid, ethanesulfonic acid, benzenesulfonic acid, *p*-toluenesulfonic acid, methanesulfonic acid, 2-naphthalenesulfonic acid, naphthalenedisulfonic acid, camphorsulfonic acid, citric acid, tartaric acid, stearic acid, lactic acid, oxalic acid, malonic acid, succinic acid, malic acid, adipic acid, alginic acid, maleic acid, fumaric acid, D-gluconic acid, mandelic acid, ascorbic acid, glucoheptoic acid, glycerophosphoric acid, aspartic acid, sulfosalicylic acid, hemisulfuric acid, or thiocyanic acid.

The term "anion generated by ionization" includes all anionic forms of the inorganic and organic acids that may be generated by ionization. For example, different anions may be generated by primary, secondary or tertiary ionization. As an example, phosphoric acid can be subjected to primary ionization to generate dihydrogen phosphate, secondary ionization to generate hydrogen phosphate, and tertiary ionization to generate phosphate; sulfuric acid can be subjected to primary ionization to generate hydrogen sulfate and secondary ionization to generate sulfate. For the compound of formula (I) disclosed herein, a multivalent anion generated by multi-stage ionization can be shared by multiple molecules. All such possible anions are included within the scope of anions of the present disclosure.

The term "tautomer" refers to functional isomers resulting from the rapid movement of an atom in a molecule between two positions. The compounds disclosed herein may exhibit the tautomerism. Tautomeric compounds may exist in two or more interconvertible forms. Prototropic tautomers result from the migration of a covalently bonded hydrogen atom between two atoms. Tautomers generally exist in an equilibrium form. Trying to separate a single tautomer usually lead to a mixture, the physicochemical properties of which are consistent with the mixture of the compound. The position of the equilibrium depends on the chemical properties of the molecule. For example, in many aliphatic aldehydes and ketones such as acetaldehyde, the keto form predominates; whereas in phenol, the enol form predominates. The present disclosure comprises all tautomeric forms of the compound.

The term "effective amount" or "therapeutically effective amount" refers to an amount of the compounds disclosed herein sufficient to effect the intended use, including but not limited to the treatment of a disease as defined below. The therapeutically effective amount may vary depending on the following factors: the intended use (*in vitro* or *in vivo*)*,* or the subject and diseases or disorders being treated, such as weight and age of the subject, severity of the diseases or disorders, and mode of administration, and it can be readily determined by one of ordinary skill in the art. The specific dosage will vary depending on the following factors: the selected particular compound, the dosage regimen to be followed, whether to administer in combination with other compounds, the schedule of administration, the tissue to be administered and the physical delivery system carried.

The term "auxiliary material" refers to a pharmaceutically acceptable inert ingredient. Examples of types of excipients include, without limitation, adhesives, disintegrants, lubricants, glidants, stabilizers, fillers, diluents, and the like. Excipients are capable of enhancing the handling characteristics of the pharmaceutical formulation, i.e., making the formulation more amenable to direct compression by increasing flowability and/or adhesiveness. Examples of typical pharmaceutically acceptable carriers suitable for use in the above formulations include: saccharides, such as lactose, sucrose, mannitol, and sorbitol; starches, such as corn starch, tapioca starch and potato starch; cellulose and its derivatives, such as sodium carboxymethylcellulose, ethyl cellulose and methyl cellulose; calcium phosphates, such as dicalcium phosphate and tricalcium phosphate; sodium sulfate; calcium sulfate; polyvinylpyrrolidone; polyvinyl alcohol; stearic acid; alkaline earth metal stearate, such as magnesium stearate and calcium stearate; vegetable oils such as peanut oil, cottonseed oil, sesame oil, olive oil and corn oil; nonionic, cationic and anionic surfactants; a glycol polymer; fatty alcohols; and grain hydrolysis solids and other nontoxic compatible auxiliary materials commonly available in pharmaceutical formulations, such as fillers, adhesives, disintegrants, buffers, preservatives, antioxidants, lubricants, and colorants.

### Beneficial effects of the present disclosure:

The compound provided herein has excellent JNK inhibitory activity. In addition, the compound disclosed herein has better safety and metabolic stability. The compound disclosed herein is simple in preparation and easy to be purified, thereby having good application prospect.

### DETAILED DESCRIPTION

The compounds of the general formulas disclosed herein and the preparation method and use thereof will be described in detail with reference to the following examples. The following examples are merely exemplary illustration and explanation of the present disclosure, and should not be construed as limiting the protection scope of the present disclosure. All techniques implemented based on the aforementioned content of the present disclosure are encompassed within the protection scope of the present disclosure.

Unless otherwise stated, the starting materials and reagents used in the following examples are all commercially available products or can be prepared by known methods.

### <Preparation Examples>

### Example 1: Preparation of Compounds (T001A) and (T001)

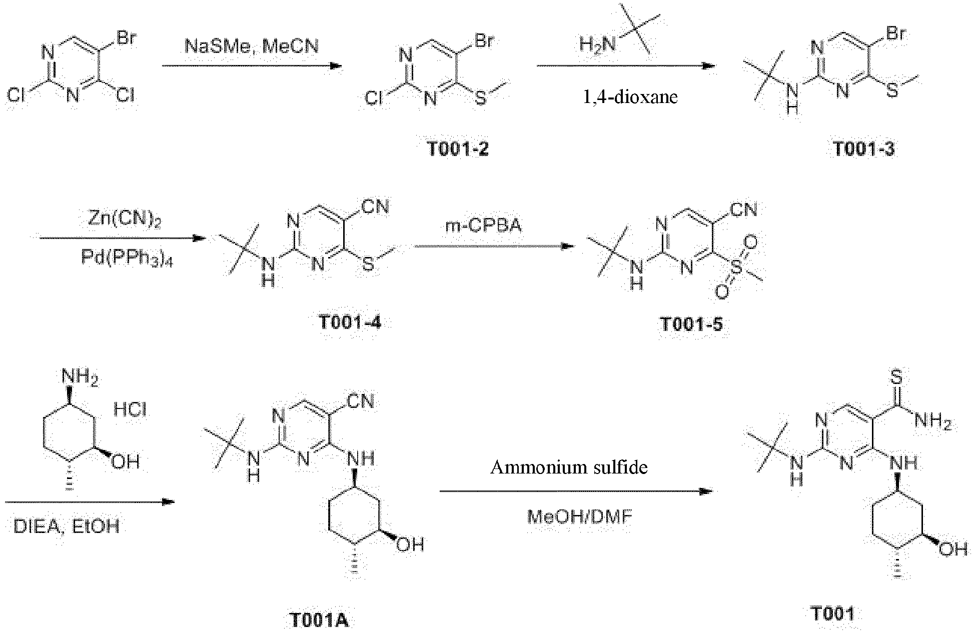

### 1.1 Preparation of compound (T001-2)

Sodium thiomethoxide (2.4 g, 34.8 mmol) was added to a solution of 5-bromo-2,4-dichloropyrimidine (4 g, 17.4 mmol) in acetonitrile (40 mL) at normal temperature. After the addition was completed, the reaction mixture was stirred at room temperature overnight. Water was added, and the reaction mixture was extracted with ethyl acetate. The organic phase was collected, dried, concentrated under reduced pressure and purified by silica gel column chromatography (EA/PE = 1/50) to give 3.48 g of a white solid with a yield of 82.8%.

¹H NMR (400 MHz, CDCl₃): δ = 8.31 (s, 1H), 2.59 (s, 3H).

### 1.2 Preparation of compound (T001-3)

Compound T001-2 (3.48 g, 14.54 mmol), dioxane (10 mL) and *tert*-butylamine (6.38 g, 87.2 mmol) were added to a sealed tube. After the addition was completed, the tube was sealed and the reaction mixture was reacted at 100 °C for 72 h. After the reaction was completed, as detected by TLC plate (PE/EA = 10/1, R_{f}= 0.6), the reaction mixture was cooled to room temperature, concentrated under reduced pressure and purified by silica gel column chromatography (EA/PE = 1/20) to give 2.35 g of a white solid with a yield of 59%.

¹H NMR (400 MHz, DMSO-*d₆*): δ = 8.08 (s, 1H), 7.04 (s, 1H), 1.44 (s, 3H), 1.38 (s, 9H).

### 1.3 Preparation of compound (T001-4)

Compound T001-3 (2 g, 7.2 mmol), DMF (5 mL), zinc cyanide (1.7 g, 14.5 mmol) and tetrakis(triphenylphosphine)palladium(0) (0.84 g, 0.72 mmol) were added to a microwave tube at normal temperature. After the addition was completed, nitrogen was introduced, the tube was sealed, and the reaction mixture was reacted at 120 °C for 1.5 h under microwave in a temperature mode. The reaction mixture was then cooled to room temperature, concentrated under reduced pressure and purified by silica gel column chromatography (EA/PE = 1/20) to give 1.48 g of a white solid with a yield of 91%.

### 1.4 Preparation of compound (T001-5)

*m*-chloroperoxybenzoic acid (4 g, 19.9 mmol) was added in portions to a solution of compound T001-4 (1.48 g, 6.63 mmol) in dichloromethane (30 mL) under an ice bath. After the addition was completed, the reaction mixture was stirred at room temperature for 4 h and the reaction was completed as detected. Excessive *m*-chloroperoxybenzoic acid was washed away with saturated sodium bicarbonate solution, and the organic phase was washed with water, dried, concentrated under reduced pressure and purified by silica gel column chromatography (EA/PE = 3/20) to give 0.44 g of a white solid with a yield of 26.2%.

### 1.5 Preparation of compound (T001A)

*N*,*N-*diisopropylethylamine (i.e., DIEA, 0.15 g, 1.18 mmol) and (1*R*,2*R*,5*R*)-5-amino-2-methylcyclohexanol hydrochloride (CAS: 1403864-98-1, 0.13 g, 0.787mmol) were added to a solution of compound T001-5 (0.2 g, 0.787mmol) in ethanol (3 mL) at normal temperature. After the addition was completed, the reaction mixture was heated at 90 °C for 0.5 h, and the reaction was completed as detected by liquid chromatography. The reaction mixture was concentrated under reduced pressure and purified by silica gel column chromatography (EA/PE = 3/10) to give 80 mg of a white solid with a yield of 33.4%.

¹H NMR (400 MHz, CD₃OD): δ = 8.04 (s, 1H), 4.14-4.07 (m, 1H), 3.16-3.10 (m, 1H), 2.22-2.19 (m, 1H), 1.93-1.89 (m, 1H), 1.81-1.77 (m, 1H), 1.44 (s, 9H), 1.39-1.29 (m, 3H), 1.12-1.05 (m, 1H), 1.02 (d, J = 6.8 Hz, 3H). Rt = 3.629 min, [M+H]⁺ = 304.2.

### 1.6 Preparation of compound (T001)

Compound T001A (80 mg, 0.264 mmol), DMF (1 mL), methanol (1 mL) and aqueous ammonium sulfide solution (20%, 0.8 mL, 0.246 mmol) were added to a microwave tube at normal temperature. After the addition was completed, nitrogen was introduced, the tube was sealed, and the reaction mixture was reacted at 100 °C for 1 h under microwave in a temperature mode. The reaction mixture was cooled to room temperature, concentrated under reduced pressure, purified by preparative high performance liquid chromatography (ammonium bicarbonate method) and lyophilized to give 19 mg of a white solid with a yield of 21.3%.

¹H NMR (400 MHz, CD₃OD): δ = 8.15 (s, 1H), 4.08-4.02 (m, 1H), 3.16-3.09 (m, 1H), 2.36-2.33 (m, 1H), 2.07-2.04 (m, 1H), 1.81-1.77 (m, 1H), 1.46 (s, 9H), 1.35-1.24 (m, 3H), 1.13-1.06 (m, 1H), 1.03 (d, J = 6 Hz, 3H); Rt = 3.569 min, [M+H]⁺ = 338.1.

### Example 2: Preparation of Compound (T002)

Hydroxylamine hydrochloride (114 mg, 1.65 mmol) and triethylamine (166 mg, 1.65 mmol) were added to a solution of compound T001A (100 mg, 0.33 mmol) in ethanol (2 mL) at normal temperature. The reaction mixture was warmed to 80 °C and heated for 2 h, and then cooled to room temperature, concentrated under reduced pressure, purified by preparative high performance liquid chromatography (ammonium bicarbonate method) and lyophilized to give 100 mg of a white solid with a yield of 90%.

¹H NMR (400 MHz, DMSO-*d₆*): δ = 9.48(s, 1H), 8.43-8.34(m, 1H), 8.09 (s, 1H), 6.17 (s, 1H), 5.72 (s, 1H), 4.57-4.56 (m, 1H), 3.90-3.87 (m, 1H), 2.98-2.96 (m, 1H), 2.17-2.14 (m, 1H), 1.95-1.92 (m, 1H), 1.69-1.65 (m, 1H), 1.36 (s, 9H), 1.23-0.98 (m, 4H), 0.94 (d, J = 6 Hz, 3H); Rt = 3.214 min, [M+H]⁺ = 337.2.

### Example 3: Preparation of Compound (T003)

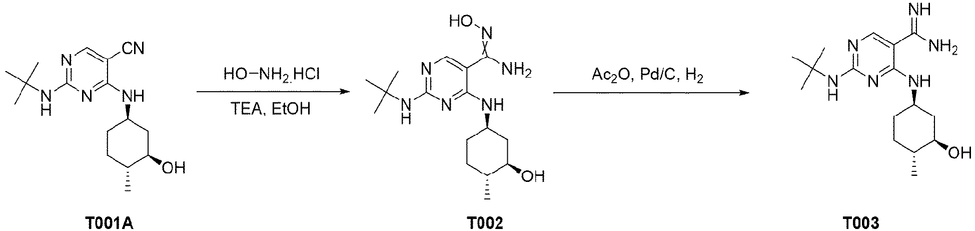

Two drops of acetic acid, 2 drops of acetic anhydride and wet palladium on carbon (about 10 mg) were added to a solution of compound T002 (50 mg, 0.148 mmol) in methanol (3 mL) at normal temperature. After the addition was completed, purge with nitrogen and purge with hydrogen were carried out successively, and the reaction mixture was stirred at room temperature overnight. The reaction mixture was concentrated under reduced pressure, purified by preparative high performance liquid chromatography (ammonium bicarbonate method) and lyophilized to give 17.1 mg of a yellow solid with a yield of 35.8%.

¹H NMR (400 MHz, DMSO-*d₆*): δ =10.62-10.60 (br, 1H), 6.17(s, 1H), 6.57-6.28 (brs, 1H), 6.24-6.07 (brs, 1H), 6.04-5.96 (brs, 2H), 4.55-4.54 (m, 1H), 3.87-3.84 (m, 1H), 2.98-2.93 (m, 1H), 2.15-2.12 (m, 1H), 1.92-1.90 (m, 1H), 1.67-1.63 (m, 1H), 1.36 (s, 9H), 1.20-0.97 (m, 4H), 0.93 (d, J = 6.4 Hz, 3H); Rt = 2.846 min, [M+H]⁺ = 321.2.

### Example 4: Preparation of Compound (T004)

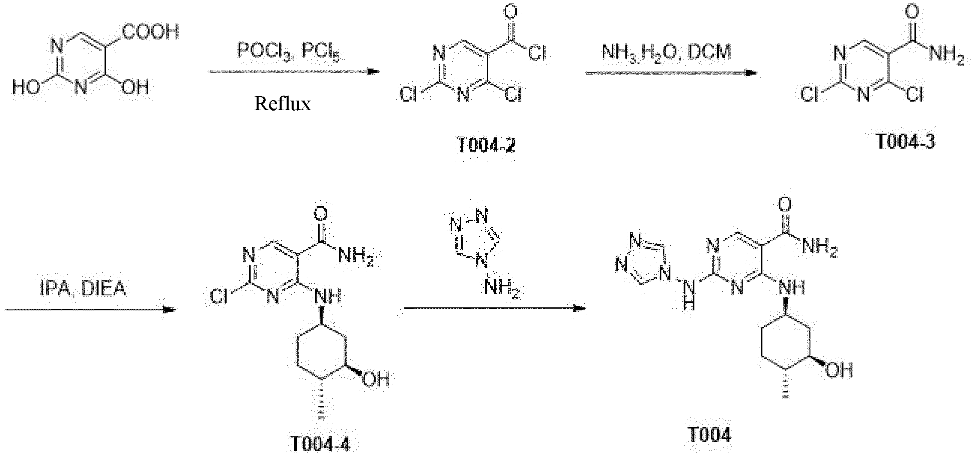

### 4.1 Preparation of compound (T004-2)

Phosphorus pentachloride (29.6 g, 142 mmol) was slowly added in portions to a solution of 2,4-dihydroxypyrimidine-5-carboxylic acid (CAS: 23945-44-0, 6.2 g, 40 mmol) in phosphorus oxychloride (30 mL) with stirring under an ice-water bath, and the reaction mixture was stirred for 30 min under an ice bath. The reaction mixture was then gradiently warmed to reflux (oil bath temperature: 120 °C). The reaction mixture was was refluxed for 16 h, and then cooled to room temperature. The reaction mixture was carefully concentrated under reduced pressure to give phosphorus oxychloride as distillate, and the reaction was quenched with warm water. The residue was dissolved in dichloromethane and filtered. The mother solution was concentrated to give 6.7 g of oil (crude product) with a yield of 78.8%, which was directly used in the next step.

### 4.2 Preparation of compound (T004-3)

Aqueous ammonia (14 mL) was added dropwise to a solution of compound T004-2 (8.8 g, 41.5 mmol) in dichloromethane (30 mL) with stirring at -20 °C, and a white solids appeared. The reaction mixture was stirred at -20 °C for 30 min, filtered, washed with water and dried to give 2.8 g of a white solid with a yield of 35%.

### 4.3 Preparation of compound (T004-4)

Diisopropylethylamine (516 mg, 4.0 mmol) was added dropwise to a solution of T004-3 (384 mg, 2.0 mmol) and (1*R*,2*R*,5*R*)-5-amino-2-methylcyclohexanol hydrochloride (397 mg, 2.4 mmol) in isopropanol (10 mL) with stirring under an ice bath. After stirring overnight at room temperature, the reaction was completed. The reaction mixture was concentrated under reduced pressure, added with water, and extracted with ethyl acetate, and the organic phase was washed with saturated brine and dried over anhydrous sodium sulfate. The organic phase was then concentrated under reduced pressure, slurried with dichloromethane and filtered to give 500 mg of a white solid with a yield of 88%.

### 4.4 Preparation of compound (T004)

Compound T004-4 (50 mg, 0.175 mmol), 4-amino-1,2,4-triazole (29.4 mg, 0.35 mmol), Pd₂(dba)₃ (10 mg, 0.075 mmol), Xantphos (13 mg, 0.026 mmol) and potassium *tert*-butoxide (39.2 mg, 0.35 mmol) were dispersed in anhydrous toluene (5 mL). After purged with nitrogen, the reaction mixture was heated to 110 °C and refluxed for 12 h. The reaction mixture was cooled to room temperature, concentrated under reduced pressure, purified by preparative high performance liquid chromatography (ammonium bicarbonate method) and lyophilized to give 17 mg of a white solid with a yield of 29%.

¹HNMR (400MHz, DMSO-*d₆*) δ 10.57 (s, 1H), 9.03 (d,J=7.6 Hz, 1H),8.64 (s, 1H), 8.44 (s,1H),7.81 (brs,1H),7.23 (brs,1H), 4.54 (d,J=5.6 Hz, 1H),3.57 (brs, 1H), 2.89-2.85 (m,1H), 2.02-1.97 (m, 1H), 1.78-1.75 (m, 1H), 1.60-1.56 (m, 1H), 1.19-0.99 (m, 3H), 0.88-0.79 (m, 4H). LCMS: Rt = 2.207 min, [M+H]⁺ = 333.1.

### Example 5: Preparation of Compounds (T005) and (T005A)

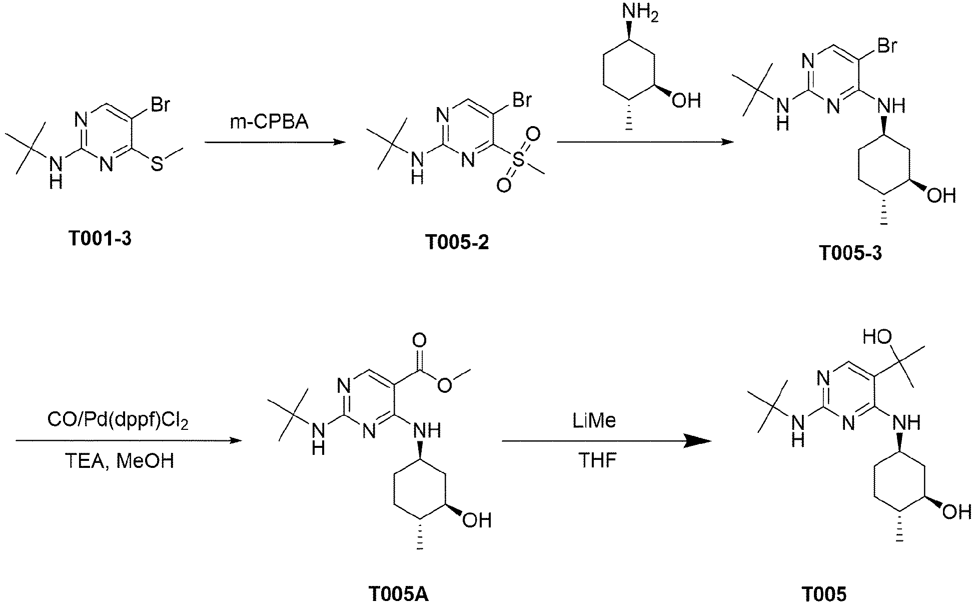

### 5.1 Preparation of compound (T005-3)

*m*-chloroperoxybenzoic acid (7.5 g, 43.6 mmol) was added in portions to a solution of compound T001-3 (4 g, 14.5 mmol) in dichloromethane (80 mL) under an ice bath. After the addition was completed, the reaction mixture was stirred at normal temperature for 3 h. After the reaction was completed as detected by liquid chromatography, the reaction mixture was washed with aqueous sodium bicarbonate solution and extracted with dichloromethane. The organic phase was collected, dried and concentrated under reduced pressure to give compound T005-2 (crude product).

DIEA (3.75 g, 29.0 mmol) and (1*R*,2*R*,5*R*)-5-amino-2-methylcyclohexanol hydrochloride (2.4 g, 14.5 mmol) were added to a solution of compound T005-2 (crude product) in DMF (40 mL) at room temperature. After the addition was completed, the reaction mixture was stirred at 120 °C overnight. After the reaction was completed as detected by liquid chromatography, the reaction mixture was concentrated to dryness under reduced pressure and purified by normal phase column chromatography (EA/PE = 1/5) to give 1.637 g of a yellow oil with a two-step yield of 31.5%.

### 5.2 Preparation of compound (T005A)

Compound T005-3 (300 mg, 0.84 mmol), triethylamine (254 mg, 2.52 mmol), bis(triphenylphosphine)palladium(II) dichloride (61 mg, 0.084 mmol) and methanol (20 mL) were added to an autoclave at normal temperature. After the addition was completed, the reaction mixture was purged with carbon monoxide, and then pressurized to 1.5 MPa and stirred overnight at 100 °C. After the reaction was completed as detected by liquid chromatography, the reaction mixture was concentrated to dryness under reduced pressure and purified by normal phase column chromatography (EA/PE = 1/5) to obtain 267 mg of yellow oil with a yield of 94.6%.

¹H NMR (400 MHz, CD₃OD): δ = 8.40 (s, 1H), 4.08-4.06 (m, 1H), 3.79 (s, 3H), 3.17-3.11 (m, 1H), 2.33-2.30 (m, 1H), 2.05-2.00 (m, 1H), 1.82-1.77 (m, 1H), 1.46 (s, 9H), 1.35-1.25 (m, 3H), 1.11-1.07 (m, 1H), 1.03 (d, J = 6.4 Hz, 3H). Rt = 3.861 min, [M+H]⁺ = 338.2.

### 5.3 Preparation of compound (T005)

A solution of methyllithium in ether (1.6M) (1.8 mL, 2.97 mmol) was slowly added dropwise to a solution of compound T005A (100 mg, 0.297 mmol) in tetrahydrofuran (3 mL) under an ice bath. After the addition was completed, the reaction mixture was warmed to room temperature and stirred overnight. After the reaction was completed as detected by liquid chromatography, the reaction was quenched with aqueous ammonium chloride solution, and the reaction mixture was extracted with ethyl acetate. The organic phase was collected, dried, concentrated under reduced pressure, purified by preparative high performance liquid chromatography (ammonium bicarbonate method) and lyophilized to give 50 mg of a white solid with a yield of 33.3%.

¹H NMR (400 MHz, DMSO-*d₆*): δ = 7.54 (s, 1H), 6.92 (d, J = 7.6 Hz, 1H), 5.73 (s, 1H), 5.34 (s, 1H), 4.56 (d, J = 5.6 Hz, 1H), 3.88-3.83 (m, 1H), 2.99-2.93 (m, 1H), 2.17-2.11 (m, 1H), 1.94-1.91 (m, 1H), 1.68-1.64 (m, 1H), 1.40 (s, 6H), 1.34 (s, 9H), 1.24-0.98 (m, 4H), 0.94(d, J = 6.8 Hz, 3H). Rt = 3.005 min, [M+H]⁺ = 337.2.

### Example 6: Preparation of Compound (T006)

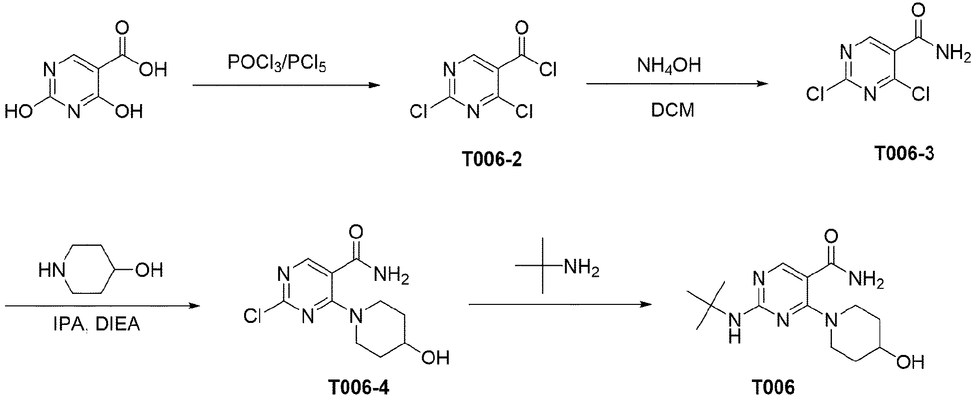

### 6.1 Preparation of compound (T006-2)

Phosphorus pentachloride (29.6 g, 142 mmol) was slowly added in portions to a solution of 2,4-dihydroxypyrimidine-5-carboxylic acid (CAS: 38324-83-3, 6.2 g, 40 mmol) in phosphorus oxychloride (30 mL) with stirring under an ice-water bath, and the reaction mixture was stirred for 30 min in an ice bath. The reaction mixture was then gradiently warmed to reflux (oil bath temperature: 120 °C). The reaction mixture was refluxed for 16 h and then cooled to room temperature. The reaction mixture was carefully concentrated under reduced pressure to give phosphorus oxychloride as distillate, and the reaction was quenched with warm water. The residue was dissolved in dichloromethane and filtered. The mother solution was concentrated to give 6.7 g of oil (crude product) with a yield of 78.8%, which was directly used in the next step.

### 6.2 Preparation of compound (T006-3)

Aqueous ammonia (14 mL) was added dropwise to a solution of compound T006-2 (8.8 g, 41.5 mmol) in dichloromethane (30 mL) with stirring at -20 °C and a white solid appeared. The reaction mixture was stirred at -20 °C for 30 min, filtered, washed with water and dried to give 2.8 g of a white solid with a yield of 35%.

### 6.3 Preparation of compound (T006-4)

Diisopropylethylamine (193 mg, 1.5 mmol) was added dropwise to a solution of compound T006-3 (192 mg, 1.0 mmol) and 4-hydroxypiperidine (CAS: 5382-16-1, 111 mg, 1.1 mmol) in isopropanol (5 mL) with stirring under an ice bath. After stirring for 2 h, the reaction was completed. The reaction mixture was concentrated under reduced pressure, added with water and extracted with ethyl acetate, and the organic phase was washed with saturated brine, dried over anhydrous sodium sulfate, concentrated under reduced pressure, slurried with dichloromethane and filtered to give 54 mg of a white solid with a yield of 21%.

### 6.4 Preparation of compound (T006)

Compound T006-4 (54 mg, 0.21 mmol), *tert*-butylamine (438 mg) and acetonitrile (5 mL) were added to a sealed glass tube. The tube was sealed, and the reaction mixture was heated to 90 °C. After 12 h of reaction, the reaction mixture was cooled to room temperature, concentrated, purified by preparative high performance liquid chromatography (ammonium bicarbonate method) and lyophilized to give 27 mg of a white solid with a yield of 44%.

¹HNMR (400 MHz, CD₃OD) δ 8.09 (s, 1H), 3.87-3.93 (m, 3H), 3.19-3.25 (m, 2H), 1.91-1.96 (m, 2H),1.55-1.59(m, 2H), 1.46 (s, 9H). LCMS: Rt = 2.22 min, [M+H]⁺ = 294.1.

### Example 7: Preparation of Compound (T007)

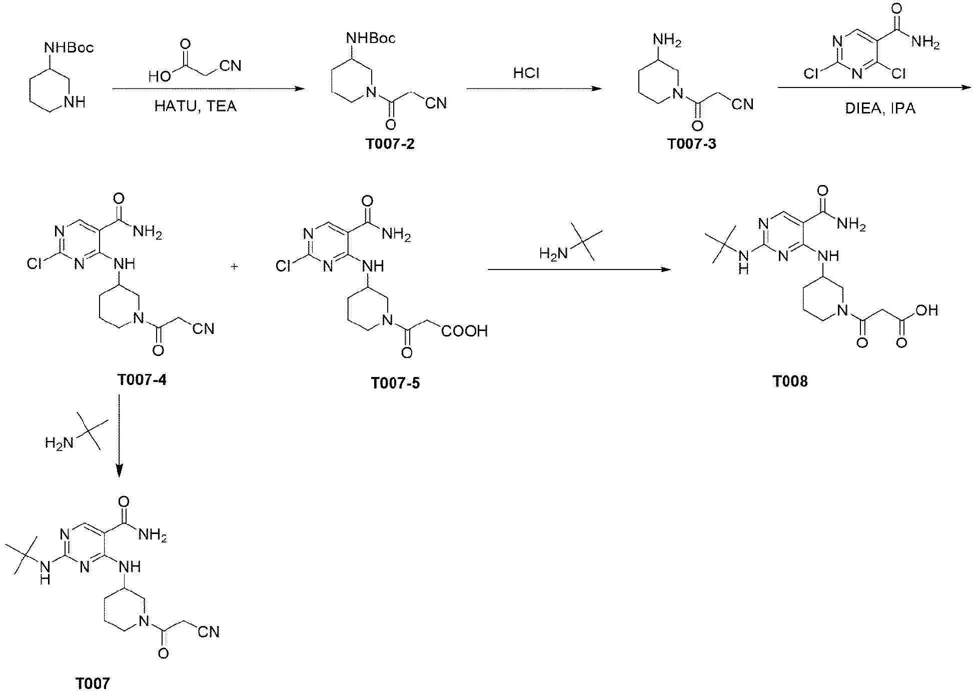

### 7.1 Preparation of compound (T007-2)

Compound 3-*tert*-butoxycarbonylaminopiperidine (CAS: 172603-05-3, 1 g, 4.98 mmol), cyanoacetic acid (636 mg, 7.4 mmol) and triethylamine (1.0 g, 9.96 mmol) were dissolved in dichloromethane (20 mL) at room temperature, and 2-(7-azabenzotriazol-1-yl)-*N,N,N',N'*-tetramethyluronium hexafluorophosphate (2.26 g, 5.96 mmol) was added, and the reaction mixture was stirred at room temperature overnight. The reaction mixture was added with water and extracted with dichloromethane. The organic phase was concentrated under reduced pressure, and the crude product was purified by normal phase column chromatography to give 898 mg of a yellow solid with a yield of 69%.

### 7.2 Preparation of compound (T007-3)

Compound T007-2 (848 mg, 3.17 mmol) was dissolved in a solution of hydrochloric acid in ethyl acetate (2 mL) at room temperature, and the reaction mixture was stirred for 4 h and concentrated under reduced pressure to give 380 mg of a white solid with a yield of 71%.

### 7.3 Preparation of compound (T007-4)

Compound T007-3 (350 mg, 2.09 mmol) and compound T006-3 (482 mg, 2.5 mmol) were dissolved in isopropanol (10 mL), and DIEA (808 mg, 6.27 mmol) was added dropwise with stirring under an ice bath, and the reaction mixture was stirred at room temperature overnight. The reaction mixture was concentrated under reduced pressure, added with water and extracted with ethyl acetate, and the organic phase was washed with saturated brine and dried over anhydrous sodium sulfate. The residue obtained after concentration was slurried with dichloromethane and filtered to give 300 mg of T007-4 (a white solid) with a yield of 44.6%. The filtrate was concentrated and subjected to high performance liquid chromatography (ammonium bicarbonate method) to give 70 mg of cyano hydrolyzed by-product T007-5.

### 7.4 Preparation of compound (T007)

Compound T007-4 (200 mg, 0.62 mmol), acetonitrile (2 mL) and *tert*-butylamine (1 mL) were added to a sealed tube. The tube was sealed, and the reaction mixture was heated to 90 °C and reacted overnight. The reaction mixture was then cooled to room temperature, concentrated, purified by preparative high performance liquid chromatography (ammonium bicarbonate method) and lyophilized to give 15 mg of a white solid with a yield of 6.7%.

¹H NMR (400 MHz, DMSO-*d₆*):δ 8.27 (d, J = 7.6 Hz, 1H), 8.02 (s, 1H),7.49 (s, 1H), 7.13 s, 1H), 6.61 (br, 1H), 3.76-3.74 (m, 2H), 3.63 (s, 2H), 3.60-3.56 (m, 1H), 3.04-2.94 (m, 2H), 1.83-1.81 (m, 1H), 1.68-1.69 (m, 1H), 1.58-1.36 (m, 2H), 1.36 (s, 9H). LCMS: Rt = 3.992 min, [M+H]⁺= 360.2.

### Example 8: Preparation of Compound (T008)

Compound T007-5 (70 mg, 0.185 mmol) was dissolved in acetonitrile (1 mL), and *tert*-butylamine (1 mL) was added. The reaction mixture was heated to 90 °C in a sealed tube and reacted overnight. The reaction mixture was then cooled to room temperature, concentrated, purified by preparative high performance liquid chromatography (ammonium bicarbonate method) and lyophilized to give 8.7 mg of a white solid with a yield of 11.5%.

¹H NMR (400 MHz, DMSO-*d₆*): δ 8.28-8.26 (m, 1H), 4.25-4.20 (m, 1H), 3.90-3.85 (m, 1H), 3.70-3.30 (m, 4H), 2.05-1.95 (m, 1H), 1.8-1.60 (m, 4H), 1.36 (s, 9H). LCMS: Rt = 2.688 min, [M+H]⁺ = 378.2.

### Example 9: Preparation of Compound (T009)

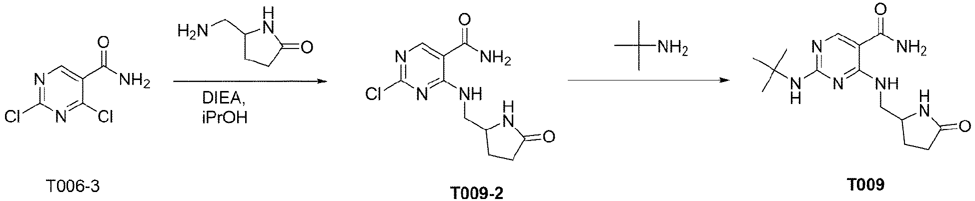

### 9.1 Preparation of compound (T009-2)

DIEA (516 mg, 4.0 mmol) was added dropwise to a solution of compound T006-3 (384 mg, 2.0 mmol) and 5-aminomethyl-2-pyrrolidone (272 mg, 2.4 mmol) in isopropanol (10 mL) with stirring under an ice bath. After the addition was completed, the reaction mixture was stirred at room temperature for 4 h, and the reaction was completed. A white solid was precipitated out. The reaction mixture was filtered and washed with water to give 400 mg of a white product with a yield of 74%.

### 9.2 Preparation of compound (T009)

Compound T009-2 (210 mg, 0.73 mmol), *tert*-butylamine (730 mg, 7.4 mmol) and *N,N*-dimethylformamide (5 mL) were added to a sealed tube. After the tube was sealed, the reaction mixture was heated to 90 °C and reacted for 12 h. The reaction mixture was then cooled to room temperature, concentrated under reduced pressure, purified by preparative high performance liquid chromatography (ammonium bicarbonate method) and lyophilized to give 67 mg of a white solid with a yield of 30%.

¹HNMR (400MHz, CD₃OD) δ 8.30 (s, 1H), 4.01-3.95 (m, 1H), 3.74-3.71 (m, 1H), 3.70-3.56 (m, 1H), 2.44-2.23 (m, 3H), 1.94-1.85 (m, 1H), 1.48 (s, 9H). LCMS: Rt = 2.673 min, [M+H]⁺ = 307.1.

### Example 10: Preparation of Compound (T010)

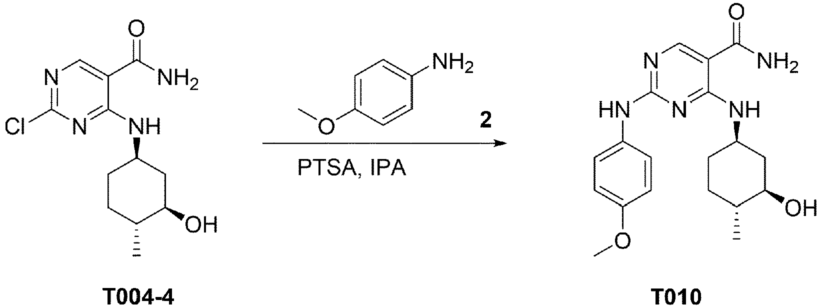

*p*-toluenesulfonic acid monohydrate (134 mg, 0.705 mmol) was added to a mixed solution of compound T004-4 (80 mg, 0.282 mmol) and *p*-methoxyaniline (42 mg, 0.338 mmol) in isopropanol (10 mL) at room temperature. After purged with nitrogen, the reaction mixture was heated to reflux for 16 h. After the reaction mixture was cooled to room temperature, a white solid was precipitated out, and the reaction mixture was filtered, washed with isopropanol and dried under vacuum to give 65 mg of pure product with a yield of 62%.

¹H NMR (400 MHz, CD₃OD): δ 8.27 (s, 1H), 7.38-7.36 (m, 2H), 7.02-7.00 (m, 2H), 4.02-3.98 (m, 1H), 3.83 (s, 3H), 3.13-3.07 (m, 1H), 2.30-2.27 (m, 1H), 2.00-1.97 (m, 1H), 1.81-1.77 (m, 1H), 1.38-1.29 (m, 4H), 1.02 (d, J = 6.4 Hz, 3H). Rt = 3.012 min, [M+H]⁺= 372.1.

### Example 11: Preparation of Compound (T011)

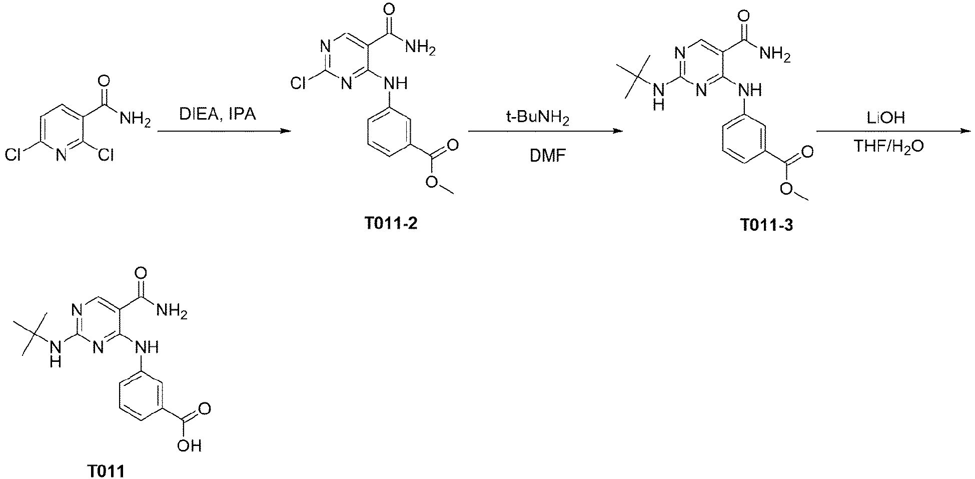

### 11.1 Preparation of compound (T011-2)

Methyl 3-amino-benzoate (474 mg, 3.1 mmol, CAS: 4518-10-9) and DIEA (503 mg, 3.9 mmol) were added to a solution of 2,6-dichloro-nicotinamide (500 mg, 2.6 mmol, CAS: 62068-78-4) in isopropanol (5 mL), and the reaction mixture was heated to reflux for 16 h. After the reaction was completed as detected by liquid chromatography, the reaction mixture was filtered and concentrated under reduced pressure to give compound T011-2 (600 mg, crude product), which was used directly in the next step. LCMS: Rt = 1.450 min, [M+H]⁺ = 306.8.

### 11.2 Preparation of compound (T011-3)

*Tert*-butylamine (0.5 mL) was added to a solution of compound T011-2 (150 mg, 0.49 mmol) in DMF (0.5 mL), and the reaction mixture was reacted at 100 °C for 18 h in a sealed tube. After the reaction was completed as detected by chromatography, the reaction mixture was concentrated under reduced pressure to give 200 mg of compound T011-3 (crude product), which was used directly in the next step. LCMS: Rt = 1.572 min, [M+H]⁺ = 343.9.

### 11.3 Preparation of compound (T011)

Water (0.3 mL) and lithium hydroxide (100 mg, 2.5 mmol) were added to a solution of compound T011-3 (crude product, 0.49 mmol) in THF (1.5 mL), and the reaction mixture was stirred at room temperature for 12 h. After the reaction was completed as detected by liquid chromatography, the reaction mixture was adjusted to pH = 4.0 with 1 mol/L hydrochloric acid, concentrated by rotary evaporation, dissolved in DMF (2 mL), filtered to remove insoluble matter, purified by preparative high performance liquid chromatography (ammonium bicarbonate method) and lyophilized to give 24 mg of a white solid with a three-step yield of 15%.

¹H NMR (400 MHz, DMSO-*d₆*): δ = 12.89 (br, 1H), 11.72 (brs, 1H), 8.58 (s, 1H), 8.27 (s, 1H), 7.88-7.72 (m, 2H), 7.61 (d, J =7.8 Hz, 1H), 7.43 (t, J = 8 Hz, 1H), 7.27 (br, 1H), 7.05 (brs, 1H), 1.38 (s, 9H). LCMS: Rt = 2.623 min, [M+H]⁺ = 330.1.

### Example 12: Preparation of Compound (T012)

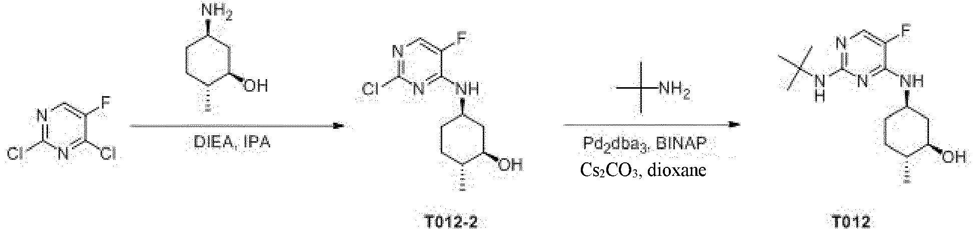

### 12.1 Preparation of compound (T012-2)

DIEA (997 mg, 7.7 mmol) and (1*R*,2*R*,5*R*)-5-amino-2-methylcyclohexanol hydrochloride (467 mg, 2.8 mmol) were added to a solution of 2,4-dichloro-5-fluoropyrimidine (500 mg, 2.5 mmol, CAS: 2927-71-1) in isopropanol (10 mL) at normal temperature. The reaction mixture was reacted for 3 h at room temperature, and the reaction was completed as detected by liquid chromatography. The reaction mixture was concentrated under reduced pressure and purified by silica gel column chromatography (EA/PE = 1/3) to give 510 mg of a yellow solid with a yield of 69.1%.

### 12.2 Preparation of compound (T012)

Compound T012-2 (510 mg, 1.78 mmol), dioxane (5 mL) and *tert*-butylamine (2 mL) were added to a sealed tube at normal temperature. After the addition was completed, the tube was sealed and the reaction mixture was heated to 90 °C and reacted for 3 h. The reaction mixture was cooled to room temperature, concentrated under reduced pressure and extracted with water and ethyl acetate, and the organic phase was dried and concentrated to give 420 mg of crude product as a yellow solid with a yield of 72.7%.

¹H NMR (400 MHz, DMSO-*d₆*): δ = 8.82 (s, 1H), 8.52 (d, J = 7.6 Hz, 1H), 7.96 (s, 1H), 8.52 (d, J = 5.2 Hz, 1H), 4.10-4.06 (m, 1H), 3.06-3.01 (m, 1H), 2.13-2.10 (m, 1H), 1.88-1.85 (m, 1H), 1.74-1.70 (m, 1H), 1.45-1.36 (m, 10H), 1.29-1.23 (m, 2H), 1.03-0.93 (m, 4H). Rt = 4.064 min, [M+H]⁺ = 324.1.

### Example 13: Preparation of Compound (T013)

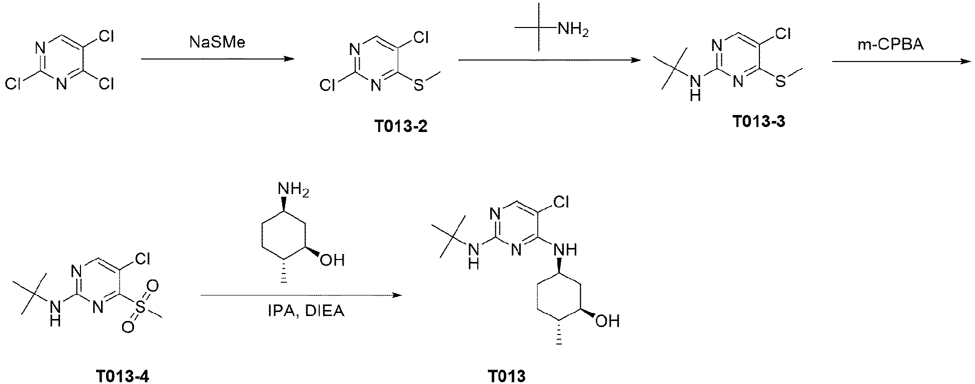

### 13.1 Preparation of compound (T013-2)

Compound 2,4,5-trichloropyrimidine (CAS: 5750-76-5, 366 mg, 2 mmol) and sodium thiomethoxide (154 mg, 2.2 mmol) were dissolved in tetrahydrofuran (50 mL) at room temperature, and the reaction mixture was stirred overnight at room temperature. After the reaction was completed as detected by LC-MS, the reaction mixture was purified by column chromatography to give 335 mg of a white solid with a yield of 86.3%.

### 13.2 Preparation of compound (T013-3)

Compound T013-2 (340 mg, 1.75 mmol) was dissolved in dioxane (3 mL), and *tert*-butylamine (1.5 mL) was added. The reaction mixture was reacted at 100 °C overnight in a sealed tube. The reaction mixture was cooled, concentrated and purified by column chromatography to give 110 mg of a white solid with a yield of 27%.

### 13.3 Preparation of compound (T013-4)

Compound T013-3 (110 mg, 0.47 mmol) was dissolved in dichloromethane (20 mL), and *m*-CPBA (246 mg, 1.42mmol) was added in portions under an ice bath. After the addition was completed, the reaction was warmed to room temperature and stirred overnight. The reaction mixture was diluted with dichloromethane (30 mL) and washed with saturated sodium bicarbonate solution, and the organic phase was dried over anhydrous sodium sulfate and subjected to column chromatography to give 60 mg of a white solid with a yield of 48.8%.

### 13.4 Preparation of compound (T013)

Compound T013-4 (60 mg, 0.228 mmol) was dissolved in dioxane (10 mL), and (1*R*,2*R*,5*R*)-5-amino-2-methylcyclohexanol hydrochloride (56.5 mg, 0.342 mmol) and diisopropylethylamine (88.3 mg, 0.684 mmol) were added. The reaction mixture was stirred at 100 °C for 20 h. The reaction mixture was concentrated under reduced pressure, purified by preparative high performance liquid chromatography (ammonium bicarbonate method) and lyophilized to give 20 mg of a white solid with a yield of 28%.

¹H NMR (400MHz, CDCl₃) δ 7.73 (s, 1H), 5.07 (br, 1H), 4.91 (s, 1H), 3.99-3.95 (m, 1H), 3.32-3.26 (m, 1H), 2.39-2.35 (m, 1H), 2.06-2.02 (m, 1H), 1.83-1.77 (m, 1H), 1.38(s, 9H),1.38-1.25 (m, 3H), 1.15-1.05 (m, 1H), 1.03 (d, J = 6.6 Hz, 3H). LCMS: Rt = 4.524 min, [M+H]⁺ = 313.1.

### Example 14: Preparation of Compound (T014)

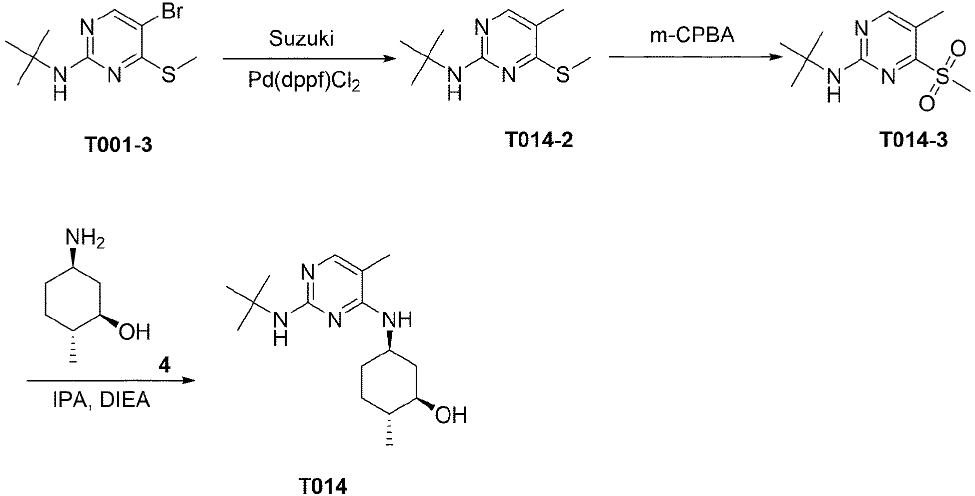

### 14.1 Preparation of compound (T014-2)

Compound T001-3 (230 mg, 0.836 mmol), methylboronic acid (251 mg, 4.18 mmol), potassium carbonate (346 mg, 0.250 mmol) and Pd(dppf)Cl₂ (50 mg, 0.068 mmol) were dispersed in a mixed solution of dioxane (5 mL) and water (2 mL). After purged with nitrogen, the reaction mixture was heated to reflux for 4 h, and the reaction was completed as detected by liquid chromatography. The reaction mixture was concentrated under reduced pressure and purified by column chromatography to give 130 mg of an oil in with a yield of 74%.

### 14.2 Preparation of compound (T014-3)

3-chlorobenzoperoxoic acid (296 mg, 1.70 mmol) was added to a solution of compound T014-2 (180 mg, 0.853 mmol) in dichloromethane (2 mL) with stirring under an ice bath. After stirring at room temperature for 4 h, the reaction was completed. The reaction mixture was diluted with dichloromethane and filtered, and the organic phase was washed with sodium bicarbonate solution, concentrated under reduced pressure at low temperature and purified by column chromatography to give 150 mg of a white solid with a yield of 72%.

### 14.3 Preparation of compound (T014)

Compound T014-3 (50 mg, 0.205 mmol), isopropanol (2 mL), (1*R*,3*R*,4*R*)-5-amino-2-methylcyclohexanol hydrochloride (34 mg, 0.205 mmol) and DIEA (53 mg, 0.41 mmol) were added to an 8 mL microwave tube. The microwave tube was sealed, the reaction mixture was reacted at 140 °C for 2 h in a microwave reactor. The reaction mixture was then cooled to room temperature, concentrated under reduced pressure, purified by preparative high performance liquid chromatography (ammonium bicarbonate method) and lyophilized to give 8.1 mg of a white solid with a yield of 13.5%.

¹H NMR (400MHz, CD₃OD) δ 7.43 (s, 1H), 4.23-4.17 (m, 1H), 3.20-3.14 (m, 1H), 2.24-2.21 (m, 1H), 2.20 (s, 3H), 1.96-1.92 (m, 1H), 1.86-1.82 (m, 1H), 1.55-1.52 (m, 11H), 1.48-1.36 (m, 1H), 1.09-1.07 (m, 1H), 1.04 (d, J = 6.4 Hz, 3H). LCMS: Rt = 3.168 min, [M+H]⁺ = 293.2.

### Preparation of other compounds of the same type

Reference was made to the above process routes and procedures of Examples 1-14 to prepare the compounds in Table 2 below:

**Table 2**

| Example | Compound No. | Ms+1 |
|---|---|---|
| 15 | T015 | 362.1 |
| 16 | T016 | 307.1 |
| 17 | T017 | 351.1 |
| 18 | T018 | 308.1 |
| 19 | T019 | 294.2 |
| 20 | T020 | 308.2 |
| 21 | T021 | 336.3 |
| 22 | T022 | 336.1 |
| 23 | T023 | 349.1 |
| 24 | T024 | 420.1 |
| 25 | T025 | 293.3 |
| 26 | T026 | 316.1 |
| 27 | T027 | 348.3 |
| 28 | T028 | 329.1 |
| 29 | T029 | 309.1 |
| 30 | T030 | 311.1 |

### Example 31: Preparation of Compound (T031)

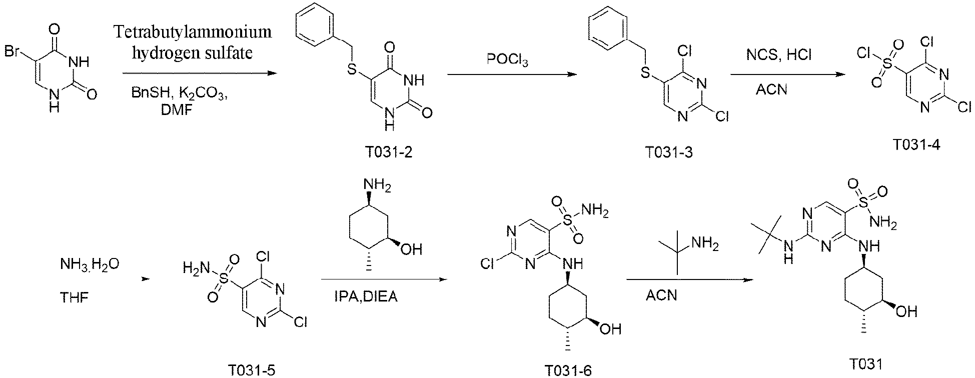

### 31.1 Preparation of compound (T031-2)

5-bromouracil (CAS: 51-20-7, 3 g, 0.015 mol), benzylmercaptan (2.1 g, 0.017 mol), tetrabutylammonium hydrogen sulfate (CAS: 32503-27-8, 1.3 g, 0.25 mol) and potassium carbonate (4.5 g, 0.033 mol) were dissolved in DMF (20 mL), and the reaction mixture was reacted at 60 °C overnight (about 16 h). After the reaction was completed as detected by liquid chromatography, the reaction mixture was filtered, and the filtrate was concentrated under reduced pressure and slurried with petroleum ether to give 2.1 g of a white solid with a yield of 57.2%.

LCMS: Rt = 0.964 min, [M+H]⁺ = 235.1.

### 31.2 Preparation of compound (T031-3)

Compound T031-2 was dissolved in phosphorus oxychloride, and the reaction mixture was reacted at 110 °C overnight. After the reaction was completed as detected by liquid chromatography, the reaction mixture was concentrated under reduced pressure, and aqueous sodium bicarbonate solution and ethyl acetate were added to the concentrated solution for liquid separation. The organic phase was concentrated and purified by silica gel column chromatography (PE) to give 420 mg of a yellow oil with a yield of 24.7%.

¹H NMR (400 MHz, CD₃OD): δ 8.74 (s, 1H), 7.43 (d, J = 8.4 Hz, 2H), 7.35 (t, J = 7.2 Hz, 2H), 7.31-7.26 (m, 1H), 4.46 (s, 2H).

### 31.3 Preparation of compound (T031-4)

Hydrochloric acid solution (2 M, 0.3 mL) was dissolved in acetonitrile (10 mL), and *N*-chlorosuccinimide (NCS, CAS: 128-09-6; 635 mg, 4.74 mmol) was added under an ice-bath. The reaction mixture was stirred for 5 min, and then a solution of compound T031-3 (320 mg, 1.18 mmol) in acetonitrile was added dropwise to the reaction mixture. The resulting reaction mixture was reacted for 30 min under an ice bath, and the reaction was completed as detected by liquid chromatography. The reaction mixture was concentrated under reduced pressure, and water and ethyl acetate were added to the concentrated solution for liquid separation, and the organic phase was concentrated to give 300 mg of crude product, which was used directly in the next step.

### 31.4 Preparation of compound (T031-5)

Compound T031-4 (crude product) was dissolved in tetrahydrofuran solution, and aqueous ammonia (0.3 mL) was added dropwise under an ice bath. The reaction mixture was reacted at 0 °C for 5 min. After the reaction was completed as detected by liquid chromatography, the reaction mixture was concentrated under reduced pressure, and water and ethyl acetate were added to the concentrated solution for liquid separation. The organic phase was concentrated and purified by silica gel column chromatography (EA/PE = 1/3) to give 190 mg of a yellow oil with a two-step yield of 71.1%.

LCMS: Rt = 1.390 min, [M-H]⁻ = 225.9.

### 31.5 Preparation of compound (T031-6)

DIEA (325 mg, 2.52 mmol) and compound (1*R*,2*R*,5*R*)-5-amino-2-methylcyclohexanol hydrochloride (166 mg, 100 mmol) were added to a solution of compound T031-5 (190 mg, 0.84 mmol) in isopropanol (4 mL) at normal temperature, and the reaction mixture was reacted at room temperature for 3 h. After the reaction was completed as detected by liquid chromatography, the reaction mixture was concentrated under reduced pressure and purified by silica gel column chromatography (EA/PE = 1/1) to give 110 mg of a yellow oil with a yield of 40.8%.

### LCMS: Rt = 1.036 min, [M+H]⁺ = 320.8.

### 31.6 Preparation of compound (T031)

Compound T031-6 (90 mg, 0.28 mmol), acetonitrile (2 mL) and *tert*-butylamine (0.5 mL) were added to a sealed tube at normal temperature. After the addition was completed, the tube was sealed and the reaction mixture was heated to 90 °C and reacted for 6 h. The reaction mixture was then cooled to room temperature, concentrated under reduced pressure, purified by preparative high performance liquid chromatography (aqueous ammonia method) and lyophilized to give 2.2 mg of a white solid with a yield of 2.2%.

¹H NMR (400 MHz, CD₃OD):δ 8.75 (s, 1H), 4.13-4.07 (m, 1H), 3.10-3.03 (m, 1H), 2.23-2.20 (m, 1H), 1.94-1.91 (m, 1H), 1.75-1.70 (m, 1H), 1.37 (s, 9H),1.32-1.18 (m, 3H), 1.05-0.97 (m, 1H), 0.95 (d, J = 6.4 Hz, 3H). LCMS: Rt = 3.060 min, [M+H]⁺ = 358.1.

### Example 32: Preparation of Compound (T032)

Reference was made to the process route and procedures of Example 31, benzylthiol was replaced by benzyl alcohol, and substitution reaction was performed before debenzylation reaction, thus obtaining compound T032; [M + H]⁺ = 295.3.

### Example 33: Preparation of Compound (T033)

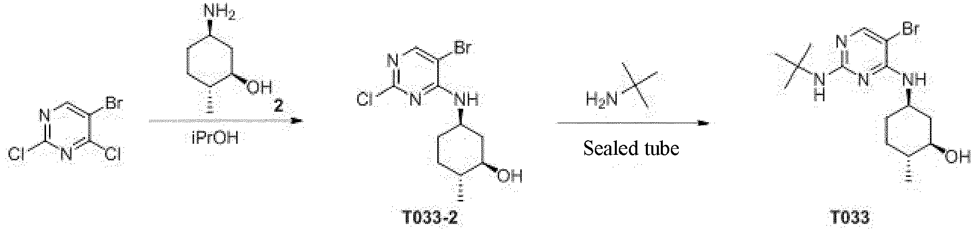

### 33.1 Preparation of compound (T033-2)

(1*R*,*2R*,5*R*)-5-amino-2-methylcyclohexanol hydrochloride (119.8 mg, 0.724 mmol) and DIEA (169.8 mg, 1.316 mmol) were added to a solution of compound 5-bromo-2,4-dichloropyrimidine (150 mg, 0.658 mmol) in isopropanol (2 mL) at normal temperature. After the addition was completed, the reaction mixture was heated to 70 °C and reacted for 1 h. The reaction mixture was then cooled to room temperature, concentrated under reduced pressure and purified by normal phase column chromatography (EA/PE = 3/10) to give 377 mg of a colorless oil with a yield of 89.5%.

### 33.2 Preparation of compound (T033)

Compound T033-2 (377 mg, 1.178 mmol), dioxane (1.2 mL) and *tert*-butylamine (517 mg, 7.068 mmol) were added to a sealed tube at normal temperature. After the addition was completed, the tube was sealed, and the reaction mixture was heated to 100 °C and reacted for 72 h. The reaction mixture was then cooled to room temperature, concentrated under reduced pressure, purified by preparative high performance liquid chromatography (ammonium bicarbonate method) and lyophilized to give 61 mg of a white solid with a yield of 14.5%.

¹H NMR (400 MHz, DMSO-*d₆*): δ = 7.80 (s, 1H), 6.28-6.26 (m, 1H), 4.64-4.63 (m, 1H), 3.95-3.93 (m, 1H), 2.98-2.95 (m, 1H), 2.01-1.98 (m, 1H), 1.76-1.65 (m, 2H), 1.43-1.18 (m, 13H), 0.98-0.90 (m, 3H). Rt = 2.918 min, [M+H]⁺ = 359.1.

### Example 34: Preparation of Compound (T034)

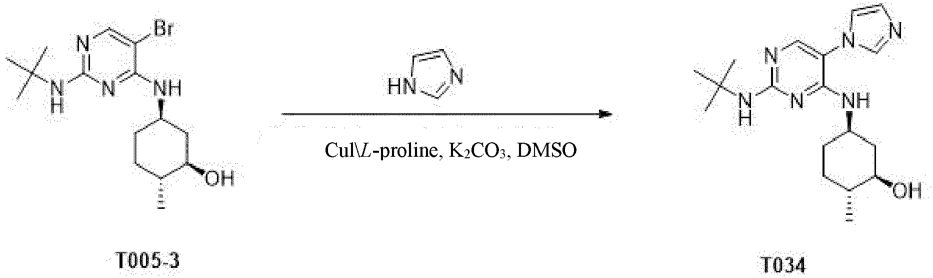

Compound T005-3 (210 mg, 0.58 mmol), imidazole (47.6 mg, 0.7 mmol), potassium carbonate (296 mg, 2.32 mmol), *L*-proline (66.7 mg, 0.58 mmol) and copper(I) iodide (55.1 mg, 0.29 mmol) were dissolved in dimethyl sulfoxide (3 mL). The reaction mixture was reacted overnight at 120 °C under nitrogen atmosphere, and the reaction was completed as detected by liquid chromatography. The reaction mixture was filtered, added with water and extracted with ethyl acetate, and the organic phase was dried over anhydrous sodium sulfate, concentrated under reduced pressure, purified by preparative high performance liquid chromatography (ammonium bicarbonate method) and lyophilized to give 8.8 mg of a white solid with a yield of 4%.

¹H NMR (400 MHz, CD₃OD): δ 7.67 (s, 1H), 7.62 (s, 1H), 7.15-713 (m, 2H), 4.11-4.04 (m, 1H), 3.15-3.09 (m, 1H), 2.19-2.14 (m, 1H), 1.90-1.86 (m, 1H), 1.77- 1.73 (m, 1H), 1.46 (s, 9H), 1.33-1.25 (m, 3H), 1.07-1.00 (d, J = 6.4 Hz, 3H). LCMS: Rt = 3.244 min, [M+H]⁺ = 345.2.

### Example 35: Preparation of Compound (T035)

Reference was made to the process route and procedures of Example 34 above, and imidazole was replaced by 1,2,4-triazole, thus obtaining compound T035; [M + H]⁺ = 346.3.

### Example 36: Preparation of Compound (T036)

Reference was made to the process route and procedures of Example 34 above, and imidazole was replaced by 1,2,3-triazole, thus obtaining compound T036; [M + H]⁺ = 346.3.

### Example 37: Preparation of Compound (T037)

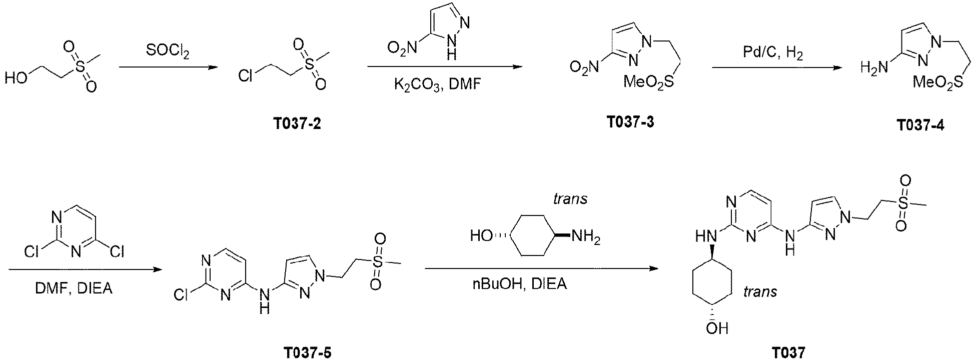

### 37.1 Preparation of compound (T037-2)

Thionyl chloride (5.76 g, 48.3 mmol) was slowly added dropwise to a solution of 2-hydroxyethyl methyl sulfone (CAS: 15205-66-0, 2 g, 16.1 mmol) and pyridine (1.27 g, 16.1 mmol) in toluene (20 mL) at room temperature. After the addition was completed, the reaction mixture was heated to 80 °C and reacted for 10 h. The reaction mixture was then cooled to room temperature, and the reaction was quenched with saturated aqueous sodium bicarbonate solution (50 mL). Liquid separation was performed to give a toluene layer, and the aqueous layer was extracted with ethyl acetate. The organic phases were combined, dried and concentrated to give 1.5 g of 1-chloro-2-methanesulfonyl ethane (crude product, brownish yellow oil) with a yield of 66%.

¹H NMR (400 MHz, CDCl₃): δ 3.93 (t, J = 6.4 Hz, 2H), 3.45 (t, J = 6.4 Hz, 2H), 3.04 (s, 3H).

### 37.2 Preparation of compound (T037-3)

T037-2 (377 mg, 2.65 mmol), 3-nitropyrazole (250 mg, 2.21 mmol), potassium carbonate (460 mg, 3.31 mmol), potassium iodide (about 5 mg, catalytic amount) and DMF (3 mL) were added to a Biotage microwave tube. Nitrogen was introduced, the tube was sealed, and the reaction mixture was reacted at 150 °C for 3 h under microwave in a temperature mode. The reaction mixture was then cooled to room temperature, diluted with water, extracted with ethyl acetate and concentrated. The residue was purified by silica gel column chromatography (PE/EA = 25% to 40%) to give 160 mg of a yellow solid with a yield of 33%.

¹H NMR (400 MHz, CDCl₃): δ 7.63 (d, J = 2.4 Hz, 1H), 6.92 (d, J = 2.4 Hz, 1H), 4.72 (t, J = 6.4 Hz, 2H), 3.69 (t, J = 6.4 Hz, 2H), 2.78 (s, 3H).

### 37.3 Preparation of compound (T037-4)

Compound T037-3 (160 mg, 0.727 mmol) and Pd/C (aqueous, about 30 mg) were dispersed in ethyl acetate (40 mL) and methanol (10 mL), and hydrogenation at normal pressure was carried out using a balloon. The reaction mixture was reacted at room temperature for 16 h. The reaction mixture was then filtered, dried and concentrated to give 160 mg of a crude product in the form of brown oil.

### 37.4 Preparation of compound (T037-5)

Compound T037-4 (160 mg, 0.73 mmol), 2,4-dichloropyrimidine (91 mg, 0.615 mmol), DIEA (0.33 mL, 1.845 mmol) and DMF (2 mL) were added to a Biotage microwave tube. Nitrogen was introduced, the tube was sealed, and the reaction mixture was reacted at 140 °C for 2 h under microwave in a temperature mode. The reaction mixture was concentrated under reduced pressure and separated by a preparative silica gel plate (PE/EA = 1:1) to give 140 mg of a yellow solid with a yield of 75%.

### 37.5 Preparation of compound (T037)

Compound T037-5 (120 mg, 0.397 mmol), *trans-*4-aminocyclohexanol (91 mg, 0.795 mmol), DIEA (0.14 mL, 0.795 mmol) and *n*-butanol (3 mL) were added to a Biotage microwave tube. Nitrogen was introduced, the tube was sealed, and the reaction mixture was reacted at 165 °C for 2 h under microwave in a temperature mode. The reaction mixture was concentrated under reduced pressure, dissolved in methanol (2.5 mL), filtered, purified by preparative high performance liquid chromatography (TFA method) and lyophilized to give 25.2 mg of a white solid with a yield of 17%.

¹H NMR (400 MHz, CD₃OD): δ 7.70 (s, 2H), 6.76 (s, 1H), 6.28 (s, 1H), 4.58 (t, J = 6.4 Hz, 2H), 3.93-3.87 (m, 1H), 3.69 (t, J = 6.4 Hz, 2H), 3.63-3.58 (m, 1H), 2.80 (s, 3H), 2.08-2.00 (m, 4H), 1.46-1.37 (m, 4H). LCMS: Rt = 3.311 min, [M+H]⁺ = 381.1.

### Example 38: Preparation of Compound (T038)

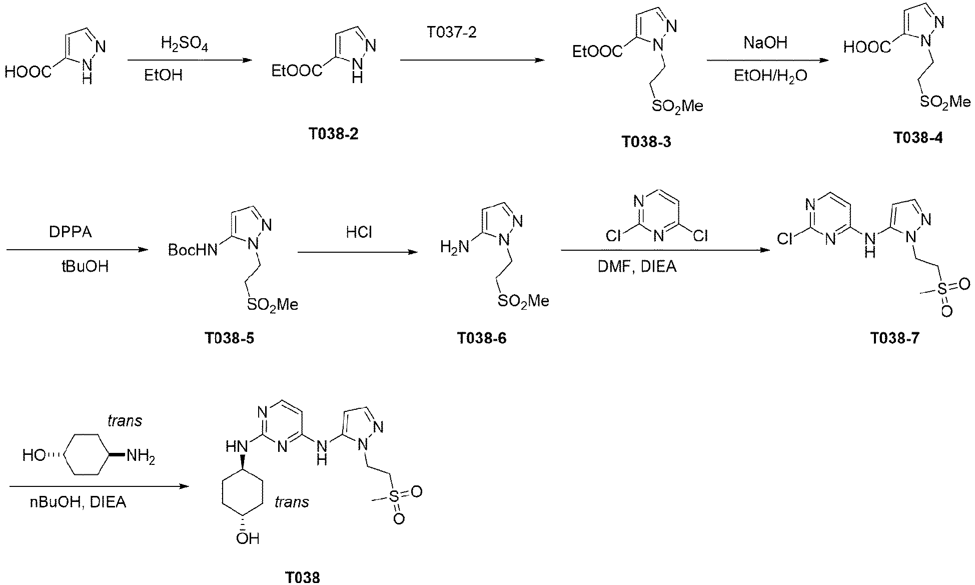

### 38.1 Preparation of compound (T038-2)

A solution of 1*H*-pyrazole-3-carboxylic acid (CAS: 1621-91-6, 2 g, 17.85 mmol) and concentrated sulfuric acid (2.9 mL, 53.56 mmol) in ethanol (80 mL) was heated at reflux for 14 h. The reaction mixture was then cooled to room temperature, subjected to distillation under reduced pressure to remove ethanol, added with ethyl acetate (50 mL) and extracted and washed with saturated aqueous sodium bicarbonate solution, and the organic phase was dried and concentrated to give 1.7 g of a white solid with a yield of 68%.

### 38.2 Preparation of compound (T038-3)

Potassium carbonate (2.06 g, 14.86 mmol) and potassium iodide (190 mg, 1.14 mmol) were added to a solution of compound T038-2 (1.6 g, 11.43 mmol) and compound T037-2 (1.95 g, 13.71 mmol) in DMF (50 mL) at room temperature. The reaction mixture was heated to 120 °C and reacted for 16 h. The reaction mixture was then concentrated under reduced pressure and purified by silica gel column chromatography (PE/EA = 55%) to obtain 2 positional isomers. Based on NOE two-dimensional nuclear magnetic spectrum, it was confirmed that the isomer with smaller polarity and lower conversion was the target product (190 mg, a white solid, a yield of 6.7%).

¹H NMR (400 MHz, CDCl₃): δ 7.52 (d, J = 2.0 Hz, 1H), 6.86 (d, J = 2.0 Hz, 1H), 5.04 (t, J = 6.8 Hz, 2H), 4.40-4.34 (m, 2H), 3.58 (t, J = 6.8 Hz, 2H), 2.86 (s, 3H), 1.40-1.35 (m, 3H).

### 38.3 Preparation of compound (T038-4)

Sodium hydroxide (37 mg, 0.92 mmol) was added to a solution of compound T038-3 (190 mg, 0.77 mmol) in ethanol (5 mL) and water (2 mL) at room temperature. The reaction mixture was stirred at room temperature for 16 h. The reaction mixture was then concentrated to remove ethanol, diluted with water (5 mL) and acidified to pH = 1 with hydrochloric acid (1 M), and a white solid was precipitated out. The mixture was filtered to give the product (140 mg) with a yield of 84%.

### 38.4 Preparation of compound (T038-5)

DPPA (227 mg, 0.82 mmol) and triethylamine (112 mg, 1.1 mmol) were added to a solution of compound T038-4 (120 mg, 0.55 mmol) in *tert-*butanol (anhydrous, 10 mL) at room temperature. The reaction mixture was heated to 90 °C and reacted for 16 h. The reaction mixture was then concentrated under reduced pressure and purified by preparative silica gel plate (DCM/MeOH = 20:1) to give 160 mg of crude product as a brown solid with a yield of 100%.

### 38.5 Preparation of compound (T038-6)

A solution of compound T038-5 (160 mg, 0.55 mmol) in a solution of hydrochloric acid in dioxane (4 M, 10 mL) was stirred at room temperature for 2 h. The reaction mixture was concentrated under reduced pressure to give the crude hydrochloride salt (110 mg, a yellow gum) with a yield of 89%.

### 38.6 Preparation of compound (T038-7)

Compound T038-6 (110 mg, 0.489 mmol), 2,4-dichloropyrimidine (61 mg, 0.407 mmol), DIEA (0.37 mL, 2.035 mmol) and DMF (2 mL) were added to a Biotage microwave tube. Nitrogen was introduced, the tube was sealed, and the reaction mixture was reacted at 145 °C for 3 h under microwave in a temperature mode. The reaction mixture was then cooled to room temperature, diluted with water, extracted with ethyl acetate and concentrated. The residue was purified by a preparative silica gel plate (PE/EA = 40%) to give 30 mg of a yellow solid with a yield of 24%.

### 38.7 Preparation of compound (T038)

Compound T038-7 (30 mg, 0.1 mmol), *trans-*4-aminocyclohexanol (23 mg, 0.2 mmol), DIEA (40 mg, 0.3 mmol) and *n*-butanol (1 mL) were added to a Biotage microwave tube. Nitrogen was introduced, the tube was sealed, and the reaction mixture was reacted at 165 °C for 3 h under microwave in a temperature mode. The reaction mixture was concentrated under reduced pressure, dissolved in methanol (1 mL), filtered, purified by preparative high performance liquid chromatography (ammonium bicarbonate method) and lyophilized to give 3.5 mg of a yellow solid with a yield of 9.2%.

¹H NMR (400 MHz, CD₃OD): δ 7.85-7.83 (m, 1H), 7.52-7.49 (m, 1H), 8.28-8.24 (m, 1H), 6.01.5.99 (m, 1H), 4.49 (t, J = 6.4 Hz, 2H), 3.68-3.64 (m, 2H), 3.60-3.52 (m, 2H), 2.77 (s, 3H), 1.99-1.92 (m, 4H), 1.36-1.26 (m, 4H). LCMS: Rt = 2.349 min, [M+H]⁺= 381.1.

### Example 39: Preparation of Compound (T039)

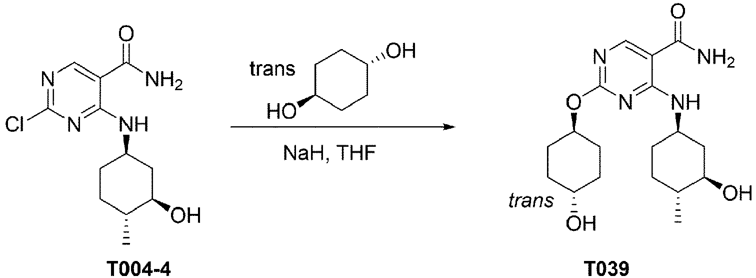

Sodium hydride (60%, 94 mg, 2.46 mmol) was added in portions to a suspension of compound T004-4 (143 mg, 1.232 mmol) in THF (15 mL) under an ice bath. After the addition was completed, *trans*-1,4-cyclohexanediol (CAS: 6995-79-5, 70 mg, 0.246 mmol) was added after the reaction mixture was stirred under an ice bath for 30 min. The reaction mixture was slowly warmed to reflux for 14 h. The reaction mixture was then cooled to room temperature, and the reaction was quenched with methanol and aqueous ammonium chloride solution. The reaction mixture was concentrated under reduced pressure, purified twice by preparative high performance liquid chromatography (TFA method and ammonium bicarbonate method) and lyophilized to give 16 mg of a white solid with a yield of 18%.

¹H NMR (400 MHz, CD₃OD): δ8.43 (s, 1H), 4.84-4.91 (m, 1H), 4.00-3.98 (m, 1H), 3.69-3.66 (m, 1H), 3.19-3.14 (m, 1H), 2.34-2.31 (m, 1H), 2.18-2.15 (m, 2H), 2.03-2.00 (m, 3H), 1.82-1.77 (m, 1H), 1.60-1.55 (m, 2H), 1.47-1.41 (m, 2H), 1.35-1.24 (m, 4H), 1.18-1.09 (m, 1H), 1.04 (d, J = 6.4 Hz, 3H). Rt = 2.576 min, [M+H]⁺ = 365.2.

### Example 40: Preparation of Compound (T040)

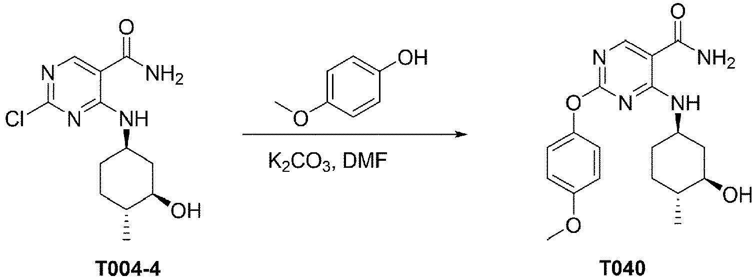

Potassium carbonate (78 mg, 0.564 mmol) was added to a mixed solution of compound T004-4 (80 mg, 0.282 mmol) and p-hydroxyanisole (CAS: 150-76-5, 38 mg, 0.309 mmol) in DMF (5 mL) at room temperature. After purged with nitrogen, the reaction mixture was heated to 80 °C and reacted for 16 h. The reaction mixture was cooled to room temperature and extracted with water and ethyl acetate. The reaction mixture was then purified by preparative high performance liquid chromatography (ammonium bicarbonate method) and lyophilized to give 60 mg of a white solid with a yield of 57%.

¹H NMR (400 MHz, CD₃OD): δ 8.49 (s, 1H), 7.08-7.05 (m, 2H), 6.97-6.93 (m, 2H), 3.80 (s, 3H), 3.61-3.55 (m, 1H), 2.94-2.88 (m, 1H), 2.15-2.11 (m, 1H), 1.83-1.80 (m, 1H), 1.66-1.61 (m, 1H), 1.22-1.08 (m, 3H), 0.97 (d, J = 6.0 Hz, 3H), 0.90-0.78 (m, 1H). Rt = 2.576 min, [M+H]⁺ = 365.2.

### Example 41: Preparation of Compound (T041)

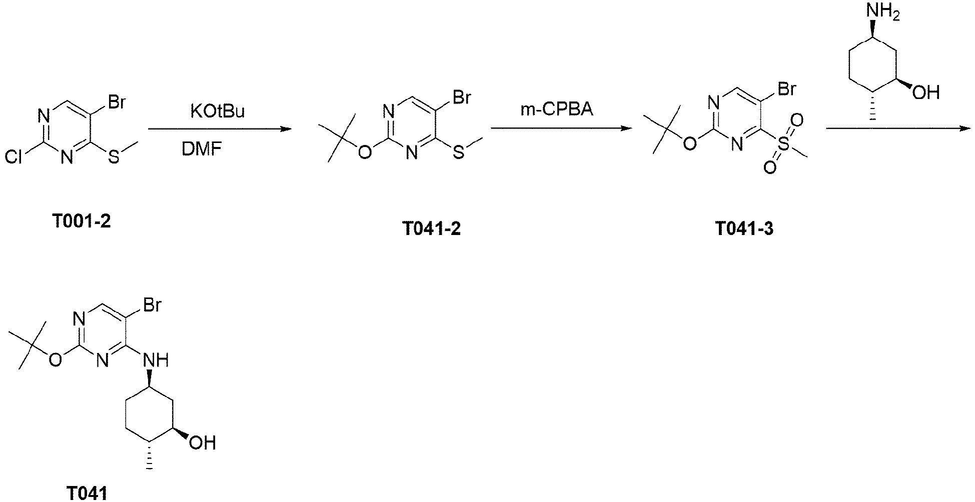

### 41.1 Preparation of compound (T041-2)

Potassium tert-butoxide (56 mg, 0.50 mmol) was added to a solution of compound T001-2 (60 mg, 0.251 mmol) in *N*,*N-*dimethylformamide (2 mL) with stirring under an ice bath. After stirring overnight at room temperature, the reaction was completed. The reaction mixture was added with water and extracted with ethyl acetate, and the organic phase was washed with saturated brine and dried over anhydrous sodium sulfate. After reaction mixture was concentrated under reduced pressure and subjected to column chromatography to give 60 mg of a colorless liquid with a yield 86%.

¹H NMR (400MHz, DMSO-*d₆*) δ8.40 (s, 1H), 2.55 (s, 3H), 1.54 (s, 9H).

### 41.2 Preparation of compound (T041-3)

3-chlorobenzoperoxoic acid (1.9 g, 10.8 mmol) was added to a solution of compound T041-2 (1.5 g, 5.41 mmol) in dichloromethane (20 mL) with stirring under an ice bath. After stirring at room temperature for 7 h, the reaction was completed. The reaction mixture was diluted with dichloromethane and filtered, and the aqueous phase was washed with sodium bicarbonate solution, concentrated under reduced pressure at low temperature and purified by column chromatography to give 700 mg of a white solid with a yield of 42%. The compound was unstable and needed to be used immediately.

### 41.3 Preparation of compound (T041)

(1*R*,3*R*,4*R*)-5-amino-2-methylcyclohexanol hydrochloride (57 mg, 0.342 mmol) and DIEA (132 mg, 1.026 mmol) were added to a solution of compound T041-3 (100 mg, 0.342 mmol) in isopropanol (3 mL) at room temperature with stirring. After stirring at room temperature for 3 h, the reaction was completed. The reaction mixture was added with water and extracted with ethyl acetate, and the organic phase was washed with saturated brine and dried over anhydrous sodium sulfate. The reaction mixture was concentrated under reduced pressure, purified by preparative high performance liquid chromatography (ammonium bicarbonate method) and lyophilized to give 40 mg of a white solid with a yield of 34%. The compound was also unstable due to susceptibility to acid, base, water vapor and the like.

¹H NMR(400MHz, CD₃OD) δ7.97 (s, 1H), 4.09-4.03 (m, 1H), 3.20-3.15 (m, 1H), 2.25-2.21 (m, 1H), 1.96-1.92 (m, 1H), 1.85-1.81 (m, 1H), 1.55 (s, 9H), 1.50-1.32 (m, 3H), 1.17-1.08(m, 1H), 1.04 (d, J = 6.4 Hz, 3H). LCMS: Rt = 4.113 min, [M+H]⁺ = 358.1, 340.1.

### Example 42: Preparation of Compound (T042)

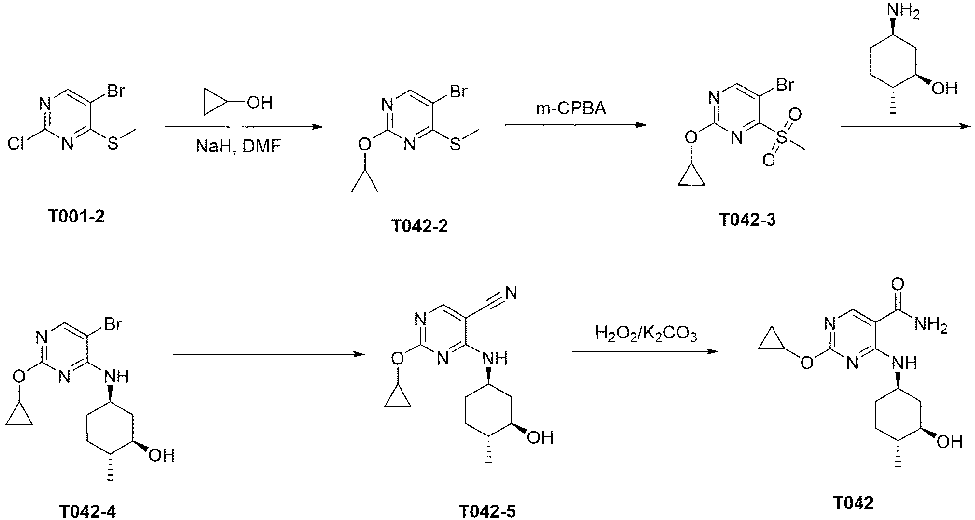

### 42.1 Preparation of compound (T042-2)

Under nitrogen atmosphere, sodium hydride (96 mg, 60%, 2.4 mmol) was added to a solution of cyclopropanol (70 mg, 1.20 mmol) in *N*,*N*-dimethylformamide (3 mL) with stirring under an ice bath. After the reaction mixture was stirred for 1 h under an ice bath, compound T001-2 (288 mg, 1.2 mmol) was added to the reaction mixture, and the resulting reaction mixture was stirred overnight at room temperature. Ammonium chloride solution was added at room temperature to quench the reaction, and the reaction mixture was extracted with ethyl acetate. The organic phase was dried, concentrated and subjected to column chromatography to give 150 mg of a colorless liquid with a yield of 40%.

LCMS: Rt = 1.730 min, [M+H]⁺= 261.0.

### 42.2 Preparation of compound (T042-3)

3-chlorobenzoperoxoic acid (59 mg, 0.343 mmol) was added to a solution of compound T042-2 (30 mg, 0.114 mmol) in dichloromethane (2 mL) with stirring under an ice bath. After stirring at room temperature for 3 h, the reaction was completed. The reaction mixture was diluted with dichloromethane and filtered, and the aqueous phase was washed with sodium bicarbonate solution, concentrated under reduced pressure at low temperature and purified by column chromatography to give 30 mg of a white solid with a yield of 88%.

### 42.3 Preparation of compound (T042-4)

(1*R*,3*R*,4*R*)-5-amino-2-methylcyclohexanol hydrochloride (57 mg, 0.342 mmol) and DIEA (132 mg, 1.026 mmol) were added to a solution of compound T042-3 (100 mg, 0.342 mmol) in isopropanol (3 mL) at room temperature with stirring. After stirring overnight at room temperature, the reaction was completed. The reaction mixture was concentrated under reduced pressure and then subjected to column chromatography to give 50 mg of a white solid with a yield of 43%. LCMS: Rt = 1.523 min, [M+H]⁺ = 341.1, 343.1.

### 42.4 Preparation of compound (T042-5)

Compound T042-4 (40 mg, 0.30 mmol) and cuprous cyanide (104 mg, 1.17 mmol) were dispersed in DMF (2 mL). The reaction mixture was heated to 150 °C and reacted for 5 h, and the reaction was completed as detected by liquid chromatography. The reaction mixture was then cooled to room temperature, added with water and extracted with ethyl acetate. The organic phase was dried over anhydrous sodium sulfate, concentrated under reduced pressure and purified by column chromatography to give 30 mg of a white solid with a yield of 88%.

### 42.5 Preparation of compound (T042)

Commercially available 30% hydrogen peroxide (0.5 mL) and aqueous sodium hydroxide solution (0.2 mL, 1 M) were added dropwise to an aqueous solution (2 mL) of compound T042-5 (40 mg, 0.30 mmol) at room temperature with stirring. After the addition was completed, the reaction mixture was stirred at room temperature for 2 h, and the reaction was completed as detected by liquid chromatography. The reaction mixture was then filtered, and the filtrate was purified by preparative high performance liquid chromatography (ammonium bicarbonate method) and lyophilized to give 10 mg of a white solid with a yield of 27%.

¹H NMR (400MHz, CD₃OD) δ 8.46 (s, 1H), 4.32-4.28 (m, 1H), 4.12-4.06 (m, 1H), 3.20-3.14 (m, 1H), 2.41-2.37 (m, 1H), 2.10-2.07 (m, 1H), 1.82-1.78 (m, 1H), 1.35-1.19 (m, 3H), 1.16-1.12 (m, 1H), 1.04 (d, J = 6.0 Hz, 3H), 0.84-0.79 (m, 4H). LCMS: Rt = 2.722 min, [M+H]⁺ = 307.1.

### Example 43: Preparation of Compound (T043)

Reference was made to the process routes and procedures of Examples 41-42 above to obtain compound T043; [M+H]⁺ = 323.1.

### Example 44: Preparation of Compound (T044)

### 44.1 Preparation of compound (T044-2)

DIEA (300 mg, 2.32 mmol) was added to a suspension of 4,6-dichloronicotinonitrile (CAS: 166526-03-0; 200 mg, 1.16 mmol) and (1*R*,2*R*,5*R*)-5-amino-2-methylcyclohexanol hydrochloride (180 mg, 1.39 mmol) in isopropanol (10 mL) at room temperature, and the reaction mixture was heated to 60 °C and reacted overnight. The reaction mixture was then cooled to room temperature, concentrated under reduced pressure to remove isopropanol, added with ethyl acetate and extracted with water. The organic phase was washed with saturated brine, dried, concentrated and purified by silica gel column chromatography (PE/EA = 1/1) to give a white solid with a yield of 55%.

### 44.2 Preparation of compound (T044)

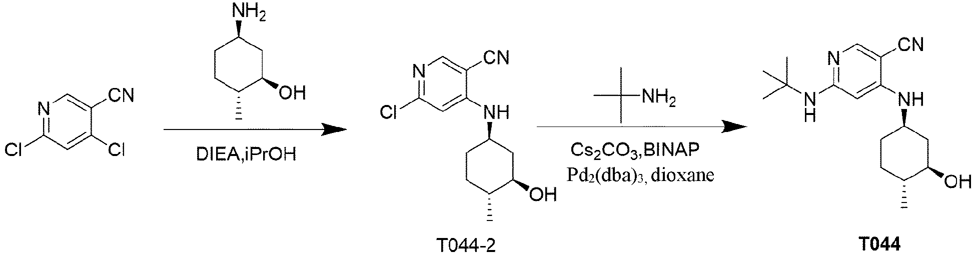

Compound T044-2 (200 mg, 0.75 mmol), CS₂CO₃ (490 mg, 1.5 mmol), Pd₂(dba)₃ (68 mg, 0.075 mmol), BINAP (46 mg, 0.075 mmol), dioxane (3 mL) and *tert*-butylamine (1 mL) were added to a sealed tube at room temperature. After purged with nitrogen for 2 min, the tube was sealed, and the reaction mixture was heated to 100 °C and reacted for 24 h. The reaction mixture was then cooled to room temperature, concentrated under reduced pressure to remove dioxane and added with ethyl acetate and water before extraction. The organic phase was dried, concentrated and purified by thin layer chromatography plate (DCM/MeOH = 10/1) to give 130 mg of a yellow solid (crude product) with a yield of 57%.

¹H NMR (400 MHz, DMSO-*d₆*): δ = 8.00 (s, 1H), 6.87 (d, J = 7.6 Hz, 1H), 5.89 (s, 1H), 4.86 (s, 1H), 4.52 (d, J = 5.6 Hz, 1H), 3.71 (s, 1H), 2.99-2.93 (m, 1H), 2.09-2.07 (m, 1H), 1.83 (d, J = 12.4 Hz, 1H), 1.64-1.60 (m, 1H), 1.36 (s, 9H), 1.17-0.95 (m, 4H), 0.93 (d, J = 6.0 Hz, 3H). Rt = 3.440 min, [M+H]⁺ = 303.1.

### Example 45: Preparation of Compound (T045)

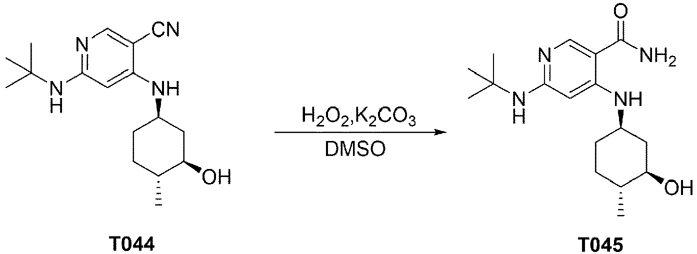

Potassium carbonate (124 mg, 0.9 mmol) and 30% hydrogen peroxide (0.15 mL, about 1.2 mmol) were added to a solution of compound T044 (90 mg, 0.3 mmol) in DMSO (0.5 mL) at room temperature. The reaction mixture was reacted at room temperature for 5 min, and saturated sodium sulfite solution was added to quench the reaction. The reaction mixture was then filtered, and the filtrate was purified by preparative high performance liquid chromatography (ammonium bicarbonate method) and lyophilized to give 18.3 mg of a white solid with a yield of 19%.

¹H NMR (400 MHz, DMSO-*d₆*): δ = 8.65 (s, 1H), 8.17 (s, 1H), 7.50 (br, 1H), 6.78 (br, 1H), 6.35 (d, J = 8.0 Hz, 1H), 5.72 (s, 1H), 4.51 (d, J = 5.2 Hz, 1H), 3.66 (s, 1H), 3.00-2.94 (m, 1H), 2.10-2.07 (m, 1H), 1.85-1.82 (m, 1H), 1.64-1.60 (m, 1H), 1.32 (s, 9H), 1.16-0.99 (m, 4H), 0.93 (d, J = 6.4 Hz, 3H). Rt = 2.918 min; [M+H]⁺ = 321.2.

### Example 46: Preparation of Compound (T046)

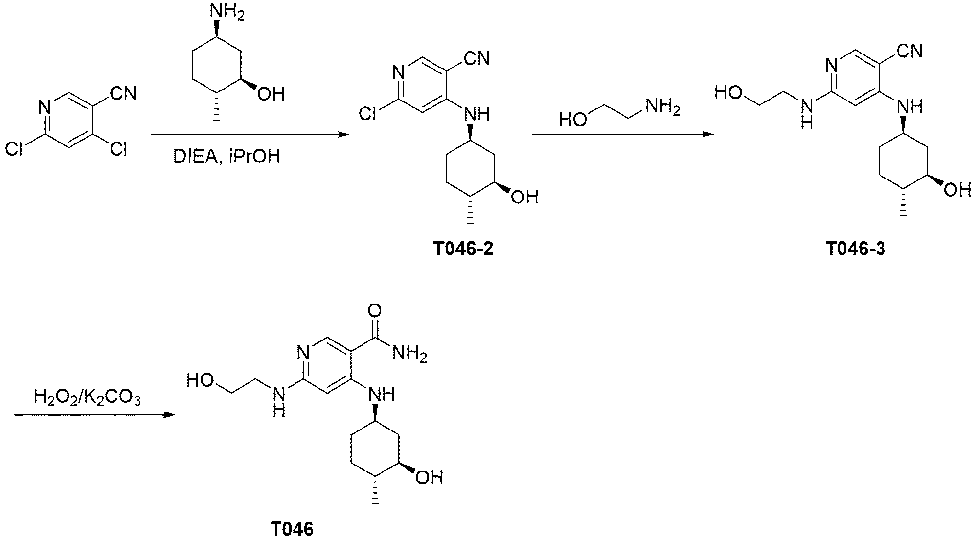

### 46.1 Preparation of compound (T046-2)

Compound 4,6-dichloronicotinonitrile (CAS: 166526-03-0, 100 mg, 0.578 mmol), (1*R*,3*R*,4*R*)-5-amino-2-methylcyclohexanol hydrochloride (95.4 mg, 0.578 mmol) and DIEA (149 mg, 1.156 mmol) were dispersed in isopropanol (5 mL). The reaction mixture was stirred at 60 °C for 2 h, and the reaction was completed as detected by liquid chromatography. The reaction mixture was added with water and ethyl acetate before extraction and liquid separation. The organic phase was dried and concentrated to give 130 mg of a yellow crude product with a yield of 85%.

### 46.2 Preparation of compound (T046-3)

Compound T046-2(100 mg, 0.377 mmol), 2-aminoethanol (1 mL) and DIEA (97 mg, 0.75 mmol) were well mixed. The reaction mixture was heated to 120 °C and stirred for 2 h, and the reaction was completed as detected by liquid chromatography. The reaction mixture was concentrated under reduced pressure and purified by preparative high performance liquid chromatography (ammonium bicarbonate method) to give 20 mg of a white solid with a yield of 18%.

### 46.3 Preparation of compound (T046)

Compound T046-3 (20 mg, 0.075 mmol) and potassium carbonate (20.7 mg, 0.15 mmol) were dispersed in DMSO (1 mL). Commercially available 30% hydrogen peroxide (0.5 mL) was added under an ice bath. The reaction mixture was warmed to room temperature and stirred for 1 h, and the reaction was completed as detected by liquid chromatography. The reaction mixture was then filtered and purified by preparative high performance liquid chromatography (ammonium bicarbonate method) and lyophilized to give 6.0 mg of a white solid with a yield of 26%.

¹H NMR (400MHz, CD₃OD): δ 8.15(s, 1H), 5.62(s, 1H), 3.77-3.74 (m, 2H), 3.70-3.67 (m, 1H), 3.33-3.26(m, 2H), 3.23-3.17(m, 1H), 2.29-2.25(m, 1H), 1.99-1.96 (m, 1H), 1.78-1.74(m, 1H), 1.32-1.13(m, 4H), 1.05(d, J=6.0 Hz, 3H). LCMS: Rt = 2.759 min; [M+H]⁺ = 309.1.

Reference was made to the above process route and procedures of Example 46 to prepare the compounds in Table 3 below:

**Table 3**

| Example | Compound No. | Ms+1 |
|---|---|---|
| 47 | T047 | 307.2 |
| 48 | T048 | 349.1 |
| 49 | T049 | 319.1 |
| 50 | T050 | 335.1 |
| 51 | T051 | 335.1 |
| 52 | T052 | 363.1 |
| 53 | T053 | 363.1 |
| 54 | T054 | 347.2 |
| 55 | T055 | 349.2 |
| 56 | T056 | 333.1 |
| 57 | T057 | 325.1 |
| 58 | T058 | 305.1 |
| 59 | T059 | 321.1 |
| 60 | T060 | 317.2 |

### Example 61: Preparation of Compound (T062)

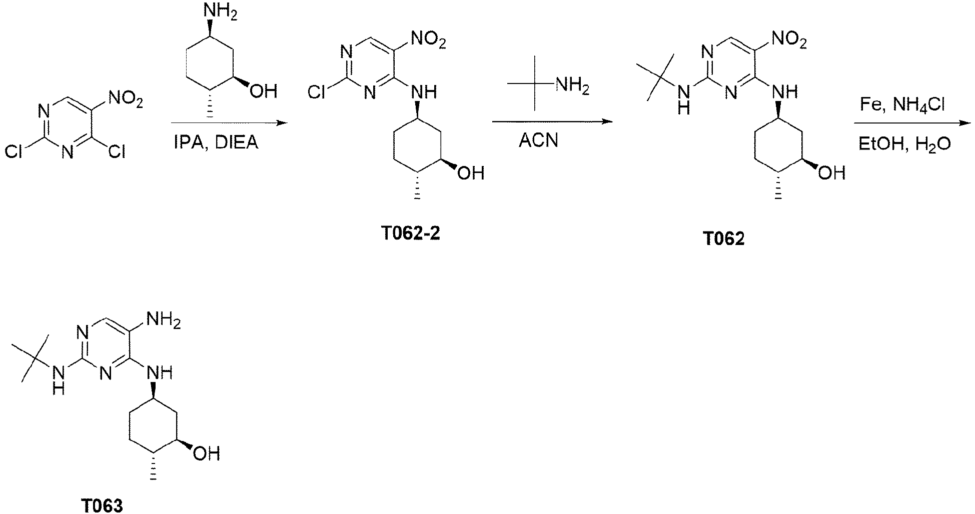

### 61.1 Preparation of compound (T062-2)

DIEA (997 mg, 7.7 mmol) and (1*R*,2*R*,5*R*)-5-amino-2-methylcyclohexanol hydrochloride (467 mg, 2.8 mmol) were added to a solution of 2,4-dichloro-5-fluoropyrimidine (500 mg, 2.5 mmol, CAS: 49845-33-2) in isopropanol (10 mL) at normal temperature. The reaction mixture was reacted for 3 h at room temperature, and the reaction was completed as detected by liquid chromatography. The reaction mixture was concentrated under reduced pressure and purified by silica gel column chromatography (EA/PE = 1/3) to give 510 mg of a yellow solid with a yield of 69.1%.

### 61.2 Preparation of compound (T062)

Compound T062-2 (510 mg, 1.78 mmol), acetonitrile (5 mL) and *tert*-butylamine (2 mL) were added to a sealed tube at normal temperature. After the addition was completed, the tube was sealed and the reaction mixture was heated to 90 °C and reacted for 3 h. The reaction mixture was cooled to room temperature, concentrated under reduced pressure and extracted with water and ethyl acetate, and the organic phase was dried and concentrated to give 420 mg of crude product (a yellow solid) with a yield of 72.7%.

¹H NMR (400 MHz, DMSO-*d₆*): δ = 8.82 (s, 1H), 8.52 (d, J = 7.6 Hz, 1H), 7.96 (s, 1H), 8.52 (d, J = 5.2 Hz, 1H), 4.10-4.06 (m, 1H), 3.06-3.01 (m, 1H), 2.13-2.10 (m, 1H), 1.88-1.85 (m, 1H), 1.74-1.70 (m, 1H), 1.45-1.36 (m, 10H), 1.29-1.23 (m, 2H), 1.03-0.93 (m, 4H). Rt = 4.064 min, [M+H]⁺ = 324.1.

### Example 62: Preparation of Compound (T063)

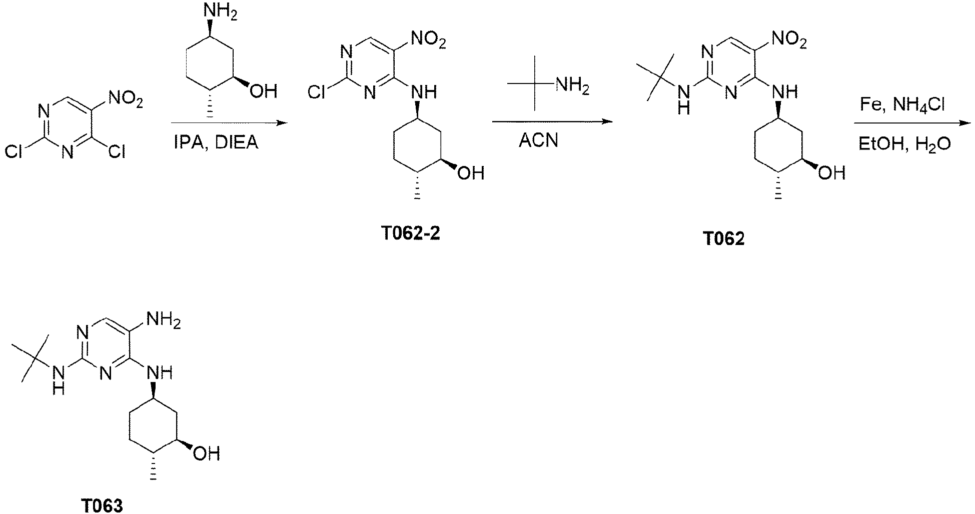

Iron powder (361 mg, 6.46 mmol) and ammonium chloride (348 mg, 6.46 mmol) were added to a mixed solution of compound QR053044A (300 mg, 0.92 mmol) in ethanol (10 mL) and water (2 mL), and the reaction mixture was reacted at 80 °C for 3 h. The reaction mixture filtered, and the filtrate was concentrated under reduced pressure and extracted with water and ethyl acetate. The organic phase was dried and concentrated to give 250 mg of a yellow oil with a yield of 93%. The crude product (50 mg) was purified by preparative high performance liquid chromatography (ammonium bicarbonate method) and lyophilized to give 5.8 mg of a yellow solid.

¹H NMR (400 MHz, CD₃OD): δ = 7.37 (s, 1H), 4.15-4.10 (m, 1H), 3.40-3.36 (m, 1H), 2.43-2.40(m, 1H), 2.12-2.08 (m, 1H), 1.84-1.80 (m, 1H), 1.51 (s, 9H), 1.46-1.34 (m, 3H), 1.25-1.22 (m, 1H), 1.13(d, J = 8.8 Hz, 3H). Rt = 2.362 min, [M+H]⁺ = 294.2.

### Example 63: Preparation of Compound (T064)

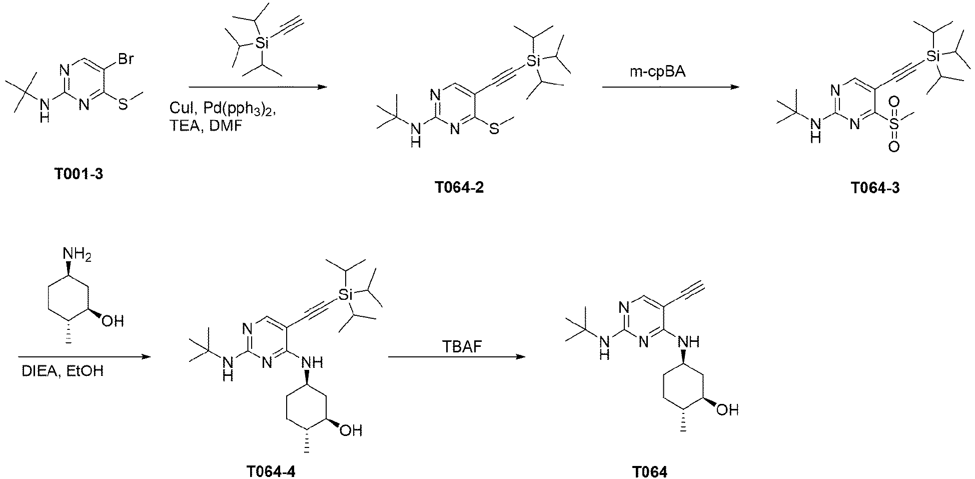

### 63.1 Preparation of compound (T064-2)

Triethylamine (551 mg, 5.4 mmol), bis(diphenylphosphine)palladium(II) dichloride (382 mg, 0.54 mmol), copper(I) iodide (104 mg, 0.54 mmol) and triisopropylsilyl acetylene (497 mg, 2.7 mmol) were added to a solution of compound T001-3 (500 mg, 1.8 mmol) in *N*,*N*-dimethylformamide at normal temperature. After the addition was completed, the reaction mixture was purged with nitrogen, then stirred at 130 °C overnight, and the reaction was completed as detected by liquid chromatography. The reaction mixture was concentrated under reduced pressure and purified by silica gel column chromatography (EA/PE = 1/5) to give 236 mg of a yellow oil with a yield of 34.3%.

### 63.2 Preparation of compound (T064-4)

*m*-chloroperoxybenzoic acid (189 mg, 0.936 mmol) was added in portions to a solution of compound T064-2 (236 mg, 0.624 mmol) in dichloromethane (3 mL) under an ice bath. After the addition was completed, the reaction mixture was stirred overnight at room temperature. After the reaction was completed as detected by liquid chromatography, the reaction mixture was washed with aqueous sodium bicarbonate solution and extracted with dichloromethane (20 mL × 3). The organic phase was collected, dried and concentrated under reduced pressure to give compound T064-3 (crude product).

DIEA (96.7 mg, 0.75 mmol) and (1*R*,2*R*,5*R*)-5-amino-2-methylcyclohexanol hydrochloride (82.5 mg, 0.5 mmol) were added to a solution of compound T064-3 (crude product) in DMF (3 mL) at normal temperature. After the addition was completed, the reaction mixture was stirred at 110 °C overnight. After the reaction was completed as detected by liquid chromatography, the reaction mixture was concentrated to dryness under reduced pressure and purified by column chromatography (EA/PE = 1/3) to give 100 mg of a yellow oil with a two-step yield of 43.8%.

### 63.3 Preparation of compound (T064)

A solution of tetrabutylammonium fluoride in tetrahydrofuran (0.2 mL) was added to a solution of compound T064-4 (95 mg, 0.198 mmol) in THF (3 mL) under an ice bath. After the addition was completed, the reaction mixture was stirred overnight at room temperature, and the reaction was completed as detected by liquid chromatography. The reaction mixture was concentrated under reduced pressure, purified by preparative high performance liquid chromatography (ammonium bicarbonate method) and lyophilized to give 15 mg of a white solid with a yield of 25%.

¹H NMR (400 MHz, CD₃OD): δ = 7.82 (s, 1H), 4.11-4.05 (m, 1H), 3.80 (s, 1H), 3.20-3.14 (m, 1H), 2.30-2.25 (m, 1H), 2.00-1.96 (m, 1H), 1.84-1.78 (m, 1H), 1.46 (s, 9H), 1.43-1.33 (m, 3H), 1.16-1.09 (m, 1H), 1.05 (d, J = 6.4 Hz, 3H). LCMS: Rt = 4.384 min, [M+H]⁺ = 303.2.

### Example 64: Preparation of Compound (T065)

Reference was made to the process route and procedures of Example 64 above, and triisopropylsilyl acetylene was replaced by propyne, thus obtaining compound T065;
[M+H]⁺ = 317.1.

### Example 65: Preparation of Compound (T066)

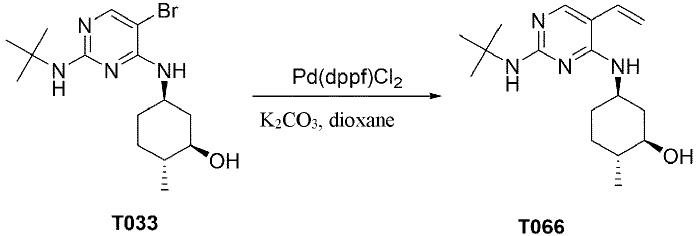

Potassium carbonate (174 mg, 1.26 mmol), [1,1'-bis(diphenylphosphino)ferrocene] dichloropalladium (II) (15.4 mg, 0.02 mmol) and potassium vinyltrifluoroborate (84.9 mg, 0.63 mmol) were added to a solution of compound T033 (150 mg, 0.42 mmol) in dioxane at normal temperature. After the addition was completed, the reaction mixture was purged with nitrogen, then stirred overnight at 105 °C, and the reaction was completed as detected by liquid chromatography. The reaction mixture was concentrated under reduced pressure, purified by preparative high performance liquid chromatography (ammonium bicarbonate method) and lyophilized to give 42 mg of a white solid with a yield of 33%.

¹H NMK (400 MHz, CDCl₃): δ = 7.81 (s, 1H), 6.42-6.35 (m, 1H), 5.40-5.35 (m, 1H), 5.14-5.11 (m, 1H), 4.90 (s, 1H), 4.78 (br, 1H), 4.09-4.01 (m, 1H), 3.34-3.28 (m, 1H), 2.41-2.35 (m, 1H), 2.07-2.04 (m, 1H), 1.82-1.77 (m, 1H), 1.43 (s, 9H), 1.42-1.34 (m, 1H), 1.27-1.08 (m, 3H), 1.06 (d, J = 6.8 Hz, 3H). LCMS: Rt = 3.301 min, [M+H]⁺ = 305.2.

### Example 66: Preparation of Compound (T067)

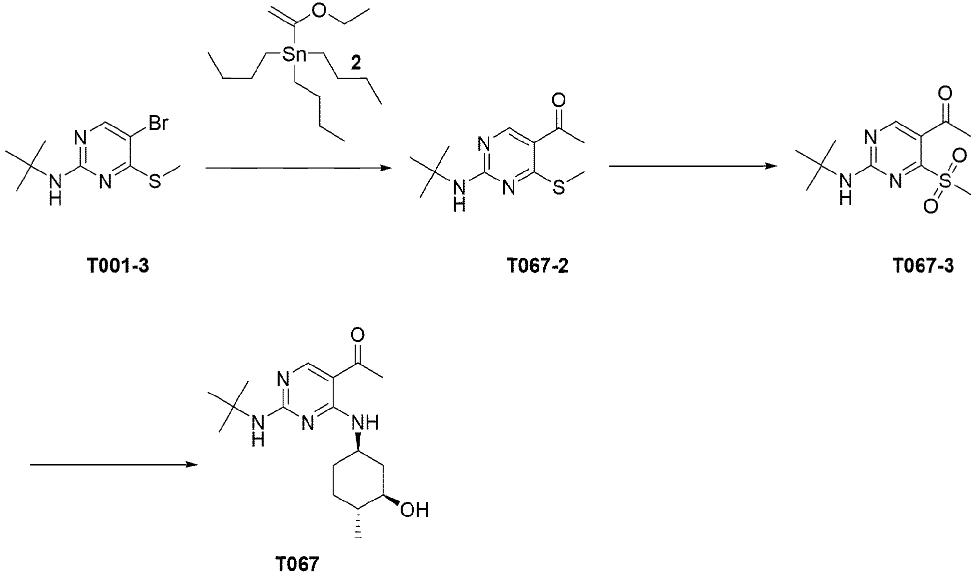

### 66.1 Preparation of compound (T067-2)

Tributyl(1-ethoxyvinyl)tin (1.5 g, 4.3 mmol) and bis(triphenylphosphine)palladium(II) dichloride (1.2 g, 1.8 mmol) were added to a solution of compound T001-3 (1 g, 3.6 mmol) in dioxane (15 mL) at normal temperature. After the addition was completed, the reaction mixture was purged with nitrogen, thenreacted at 80 °C for 16 h. After the reaction was completed as detected by liquid chromatography, the reaction mixture was concentrated under reduced pressure and purified by column chromatography (EA/PE = 1/5) to give 0.8 g of a white solid with a yield of 91.7%. Rt = 1.652 min, [M+H]⁺ = 240.1.

### 66.2 Preparation of compound (T067-3)

*m*-chloroperoxybenzoic acid (0.5 g, 2 mmol) was added in portions to a solution of compound T067-2 (0.25 g, 1 mmol) in dichloromethane (5 mL) under an ice bath. After the addition was completed, the ice bath was removed, the reaction mixture was stirred at room temperature for 4 h, and the reaction was completed as detected by liquid chromatography. Excessive *m*-chloroperoxybenzoic acid was washed away with saturated sodium bicarbonate solution, and the organic phase was washed with water, dried and concentrated under reduced pressure to give 0.2 g of crude product. Rt = 1.43 min, [M+H]⁺ = 272.5.

### 66.3 Preparation of compound (T067)

DIEA (0.3 g, 2.2 mmol) and (1*R*,2*R*,5*R*)-5-amino-2-methylcyclohexanol (0.12 g, 0.7 mmol) were added to a solution of compound T067-3 (0.2 g, 0.7 mmol) in DMF (3 mL) at normal temperature. After the addition was completed, the reaction mixture was heated to 110 °C and reacted for 16 h. After the reaction was completed as detected by liquid chromatography, the reaction mixture was concentrated under reduced pressure, purified by preparative high performance liquid chromatography (ammonium bicarbonate method) and lyophilized to give 50 mg of a white solid with a yield of 21.2%.

¹H NMR (400 MHz, CD₃OD): δ = 9.8.51 (s, 1H), 4.13-4.07 (m, 1H), 3.33-3.12 (m, 1H), 2.42 (s, 3H), 2.35-2.32 (m, 1H), 2.06-2.03 (m, 1H), 1.83-1.79 (m, 1H),1.48 (s, 9H), 1.39-1.25 (m, 3H), 1.16-1.09 (m, 1H), 1.06 (d, J = 6.4 Hz, 3H). Rt = 3.894 min, [M+H]⁺ = 321.2.

Reference was made to the above process route and procedures of Example 66 to prepare the compounds in Table 4 below:

**Table 4**

| Example | Compound No. | Ms+1 |
|---|---|---|
| 67 | T068 | 347.1 |
| 68 | T069 | 335.2 |
| 69 | T070 | 349.2 |
| 70 | T071 | 375.2 |

Reference was made to the above process route and procedures of Example 10 to prepare the compounds in Table 5 below:

**Table 5**

| Example | Compound No. | Ms+1 |
|---|---|---|
| 71 | T072 | 420.1 |
| 72 | T073 | 440.2 |
| 73 | T074 | 386.2 |

### <Biological activity assay>

### Kinase activity screening

JNK1 activity screening. JNK1 activity was detected using a 96-well (Cisbio, 66PL96025) time-resolved fluorometric assay. The JNK1 assay was conducted in the following assay buffer: 10 mM MgCl₂ (Sigma, M1028), 1 mM MnCl₂ (Sigma, M1787), 1 mM DTT (Sigma, D0632) and IX Enzymatic buffer/kinase (Cisbio, 62EZBFDC). By using the assay buffer, 3 µL of 0.1 ng/µL JNK1 kinase (Carna Biosciences, 04-163) was added to a 96-well microplate, and then 4 µL of a compound diluted appropriately (with a DMSO content of 2.5%) was added. The resulting mixture was incubated at room temperature for 0.5 h. To start the reaction, the assay buffer was used to mix 0.003 µM ATP (Aladdin, A7699) and 30 µM substrate ATF2-GST fusion (Cisbio, 64CUS000AFPEB), and 3 µL of the mixed solution was added to the microplate. The resulting mixture was incubated at room temperature for 2.5 h. HTRF detection buffer (Gibco, 62SDBRDD) was used then to prepare a mixture of 5 µg/mL MAb Anti GST-XL665 (Cisbio, 61GSTXLA) and 0.045 µg/mL PAb Anti-phospho ATF2-K (Cisbio, 61P12KAZ), and 10 µL of the mixture was added to the plate to terminate the reaction. After incubation for about 12 h, the plate was read on a Perkin-Elmer Envision reader.

### Cell evaluation method

### 1. RAW264.7 phosphorylated c-jun whole cell assay

RAW264.7 (ATCC TIB-71) cells were purchased from BeNa Culture Collection and maintained in a high-glucose DMEM medium (Gibco) containing 10% fetal bovine serum (Gibco) and 1% penicillin-streptomycin (Gibco). All cells were cultured at 37 °C in 95% air and 5% CO₂. All cells were seeded into 120 µL of cell culture medium in a 96-well plate at a density of 2×10⁵ cells per well. The stock solution of compound (15 mM) was serially diluted in DMSO (Sigma) and further diluted in growth medium, and it was then added at 15 µL per well as a 10× concentrated solution, well mixed with the cells and incubated with the cells for 30 min. Concentration of the compound vehicle (DMSO) in each well was 0.2%. After incubation for 30 min, the cells were activated with 25 ng/mL lipopolysaccharide (Sigma). Lipopolysaccharide was added to the growth medium as a 10× concentrated solution at 15 µL per well. The cell plate was incubated for 1 h, and then all the medium was removed. The level of C-Jun protein with phosphorylation of serine at site 63 was measured using the whole cell lysate kit-phosphorylated C-Jun assay (PathScan^{®} Phospho-c-Jun (Ser63) Sandwich ELISA Kit) (CST, 7145C) according to the manufacturer's instructions.

### 2. Jurkat T-cell IL-2 production assay

Jurkat T cells (clone E6-1) were purchased from China Center for Type Culture Collection and maintained in RPMI1640 (Gibco) containing 10% fetal bovine serum (Gibco) and 1% penicillin-streptomycin (Gibco). All cells were cultured at 37 °C in 95% air and 5% CO₂. All cells were seeded into 120 µL of cell culture medium in a 96-well plate at a density of 1×10⁵ cells per well. The stock solution of compound (15 mM) was serially diluted in DMSO (Sigma) and further diluted in growth medium, and it was then added at 15 µL per well as a 10× concentrated solution, well mixed with the cells and incubated with the cells for 30 min. Concentration of the compound vehicle (DMSO) in each well was 0.2%. After incubation for 30 min, the cells were activated with PMA (phorbol myristate acetate; final concentration: 50 ng/mL) (Sigma) and PHA (phytohemagglutinin; final concentration: 1 µg/mL) (Sigma). PMA and PHA were added to the growth medium as prepared 10 × concentrated solutions at 15 µL per well. The cell plate was incubated for 6 h. The cells were allowed to deposit using a microplate centrifuge (Hunan Xiangyi), and the cell supernatant was collected and stored at -20 °C. The amount of IL-2 in the supernatant was measured using a human IL-2 assay kit (Human IL-2 ValukineTM ELISA Kit) (R&D Systems, VAL110) according to the manufacturer's instructions.

The compounds in Table 6 below were all tested using enzymatic assay and cytological assay in the biochemical test for JNK1, and it was found that the compounds disclosed herein have good activity. In the enzymatic assay, IC₅₀ below 100 nM of a compound was reported as activity level A, IC₅₀ between 100 nM and 500 nM as activity level B, IC₅₀ between 500 nM and 1 µM as activity level C, and IC₅₀ greater than 1 µM as activity level D. In the cytological assay, IC₅₀ below 100 nM of a compound was reported as activity level A, IC₅₀ between 100 nM and 500 nM as activity level B, IC₅₀ between 500 nM and 1 µM as activity level C, and IC₅₀ greater than 1 µM as activity level D.

**Table 6**

| Example | Compound No. | JNK1 IC₅₀ | Raw264.7 C-Jun |
|---|---|---|---|
| 1 | T001 | A | B |
| 1 | T001A | B | B |
| 2 | T002 | A | B |
| 3 | T003 | C | D |
| 4 | T004 | D | D |
| 5 | T005A | D | D |
| 5 | T005 | D | D |
| 6 | T006 | D | D |
| 7 | T007 | D | D |
| 8 | T008 | D | D |
| 9 | T009 | D | D |
| 10 | T010 | A | A |
| 11 | T011 | C | D |
| 12 | T012 | D | D |
| 13 | T013 | C | D |
| 14 | T014 | D | D |
| 15 | T015 | A | C |
| 16 | T016 | A | C |
| 17 | T017 | A | C |
| 18 | T018 | B | C |
| 19 | T019 | B | C |
| 20 | T020 | B | C |
| 21 | T021 | B | D |
| 22 | T022 | C | D |
| 23 | T023 | C | D |
| 24 | T024 | A | B |
| 25 | T025 | A | D |
| 26 | T026 | A | B |
| 27 | T027 | C | D |
| 28 | T028 | D | D |
| 29 | T029 | D | D |
| 30 | T030 | D | D |
| 31 | T031 | D | D |
| 32 | T032 | D | D |
| 33 | T033 | A | C |
| 34 | T034 | D | D |
| 35 | T035 | B | C |
| 36 | T036 | B | C |
| 37 | T037 | D | D |
| 38 | T038 | D | D |
| 39 | T039 | B | D |
| 40 | T040 | B | C |
| 41 | T041 | C | D |
| 42 | T042 | C | D |
| 43 | T043 | C | D |
| 44 | T044 | D | D |
| 45 | T045 | D | D |
| 46 | T046 | C | D |
| 47 | T047 | C | D |
| 48 | T048 | C | D |
| 49 | T049 | C | D |
| 50 | T050 | C | D |
| 51 | T051 | C | D |
| 52 | T052 | C | D |
| 53 | T053 | D | D |
| 54 | T054 | D | D |
| 55 | T055 | D | D |
| 56 | T056 | C | D |
| 57 | T057 | D | D |
| 58 | T058 | D | D |
| 59 | T059 | D | D |
| 60 | T060 | D | D |
| 61 | T062 | D | D |
| 62 | T063 | D | D |
| 63 | T064 | C | D |
| 64 | T065 | D | D |
| 65 | T066 | D | D |
| 66 | T067 | B | B |
| 67 | T068 | B | B |
| 68 | T069 | B | B |
| 69 | T070 | C | D |
| 70 | T071 | C | D |
| 71 | T072 | A | A |
| 72 | T073 | A | B |
| 73 | T074 | A | A |

## Claims

1. A compound of formula (I) and a racemate, a stereoisomer, a tautomer, an isotopically labeled compound, a solvate, a polymorph and a nitrogen oxide thereof, or pharmaceutically acceptable salts thereof: 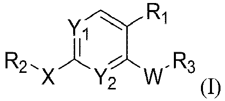 wherein,
Y₁ and Y₂ are each independently CR₄ or N;
X and W are each independently selected from CR₅R₆, O and NR₇;
R₁ is selected from H, hydroxy, halogen, cyano, nitro, NR₁₁R₁₂, C(O)NR₁₁R₁₂, C(=S)NR₁₁R₁₂, S(O)₂NR₁₁R₁₂, C(=NR₁₃)NR₁₁R₁₂, NHC(O)NR₁₁R₁₂, P(O)₂NR₁₁R₁₂, P(O)R₁₃NR₁₁R₁₂, C(O)R₁₄, NHC(O)R₁₄, C(O)OR₁₅, and the following groups unsubstituted or optionally substituted with one, two or more Rₐ: C₁₋₄₀ alkyl, C₂₋₄₀ alkenyl, C₂₋₄₀ alkynyl, C₁₋₄₀ alkoxy, C₃₋₂₀ cycloalkyl, 3-20 membered heterocyclyl, C₆₋₂₀ aryl and 5-20 membered heteroaryl;
R₂ is selected from H and the following groups unsubstituted or optionally substituted with one, two or more R_{b}: C₁₋₄₀ alkyl, C₂₋₄₀ alkenyl, C₂₋₄₀ alkynyl, C₁₋₄₀ alkoxy, C₃₋₂₀ cycloalkyl, 3-20 membered heterocyclyl, C₆₋₂₀ aryl, C₆₋₂₀ aryl-C₁₋₄₀ alkyl, 5-20 membered heteroaryl and 5-20 membered heteroaryl-C₁₋₄₀ alkyl; or
R₂, together with X, forms the following groups unsubstituted or optionally substituted with one, two or more R_{b}: NHC(O)NR₁₁R₁₂, C₃₋₂₀ cycloalkyl, 3-20 membered heterocyclyl, C₆₋₂₀ aryl and 5-20 membered heteroaryl;
R₃ is selected from H and the following groups unsubstituted or optionally substituted with one, two or more R_{c}: NR₁₁R₁₂, C(O)NR₁₁R₁₂, C(O)R₁₄, C₁₋₄₀ alkyl, C₂₋₄₀ alkenyl, C₂₋₄₀ alkynyl, C₁₋₄₀ alkoxy, C₃₋₂₀ cycloalkyl, 3-20 membered heterocyclyl, C₆₋₂₀ aryl and 5-20 membered heteroaryl; or
R₃, together with W, forms the following groups unsubstituted or optionally substituted with one, two or more R_{c}: NHC(O)NR₁₁R₁₂, C₃₋₂₀ cycloalkyl, 3-20 membered heterocyclyl, C₆₋₂₀ aryl and 5-20 membered heteroaryl;
R₄ is selected from H, hydroxy, halogen, cyano, nitro, amino, and the following groups unsubstituted or optionally substituted with one, two or more R_{d}: C₁₋₄₀ alkyl, C₂₋₄₀ alkenyl, C₂₋₄₀ alkynyl and C₁₋₄₀ alkoxy;
R₅ and R₆ are each independently selected from H, hydroxy, halogen, cyano, and the following groups unsubstituted or optionally substituted with one, two or more R_{d}: C₁₋₄₀ alkyl, C₂₋₄₀ alkenyl, C₂₋₄₀ alkynyl and C₁₋₄₀ alkoxy, with the proviso that R₅ and R₆ are not both hydroxy, cyano or C₁₋₄₀ alkoxy simultaneously; or, R₅ and R₆, together with the carbon atom attached thereto, form carbonyl;
R₇ is selected from H and the following groups unsubstituted or optionally substituted with one, two or more Rₑ: C₁₋₄₀ alkyl, C₂₋₄₀ alkenyl, C₂₋₄₀ alkynyl, C₁₋₄₀ alkoxy, C₃₋₂₀ cycloalkyl, 3-20 membered heterocyclyl, C₆₋₂₀ aryl and 5-20 membered heteroaryl;
R₁₁ and R₁₂ are each independently selected from H and the following groups unsubstituted or optionally substituted with one, two or more Rₑ: C(O)R₁₄, C₁₋₄₀ alkyl, C₂₋₄₀ alkenyl, C₂₋₄₀ alkynyl, C₁₋₄₀ alkoxy, C₃₋₂₀ cycloalkyl, 3-20 membered heterocyclyl, C₆₋₂₀ aryl and 5-20 membered heteroaryl; or
R₁₁ and R₁₂, together with the nitrogen atom attached thereto, form the following groups unsubstituted or optionally substituted with one, two or more Rₑ: 3-20 membered heterocyclyl and 5-20 membered heteroaryl;
R₁₃ is selected from H, hydroxy, cyano, and the following groups unsubstituted or optionally substituted with one, two or more Rₑ: C₁₋₄₀ alkyl and C₁₋₄₀ alkoxy;
R₁₄ is selected from H, hydroxy, halogen, and the following groups unsubstituted or optionally substituted with one, two or more R_{f}: C₁₋₄₀ alkyl, C₂₋₄₀ alkenyl, C₂₋₄₀ alkynyl, C₁₋₄₀ alkoxy, C₃₋₂₀ cycloalkyl, 3-20 membered heterocyclyl, C₆₋₂₀ aryl and 5-20 membered heteroaryl;
R₁₅ is selected from H and the following groups unsubstituted or optionally substituted with one, two or more R_{f}: C₁₋₄₀ alkyl, C₂₋₄₀ alkenyl, C₂₋₄₀ alkynyl, C₃₋₂₀ cycloalkyl, 3-20 membered heterocyclyl, C₆₋₂₀ aryl and 5-20 membered heteroaryl;
each Rₐ is independently selected from CN, halogen, OH, NH₂, oxo, and the following groups unsubstituted or optionally substituted with one, two or more R_{f}: C₁₋₄₀ alkyl and C₁₋₄₀ alkoxy;
each R_{b} is independently selected from CN, halogen, OH, NH₂, COOH, NO₂, oxo, S(O)₂CH₃, C(O)NHCH₂CH₃, and the following groups unsubstituted or optionally substituted with one, two or more R_{f}: C₁₋₄₀ alkyl, C₁₋₄₀ alkoxy, C₃₋₂₀ cycloalkyl, 3-20 membered heterocyclyl, C₆₋₂₀ aryl and 5-20 membered heteroaryl;
each R_{c} is independently selected from CN, halogen, OH, NH₂, COOH, NO₂, C(O)NH₂, C(O)NHOH, C(O)N(OH)CH₃, oxo, C(O)CH₂COOH, C(O)CH₂CN, C(O)CH₂Cl, C(O)CH₂F, C(O)CH₂Br, and the following groups unsubstituted or optionally substituted with one, two or more R_{f}: C₁₋₄₀ alkyl, C₁₋₄₀ alkoxy, C₃₋₂₀ cycloalkyl, 3-20 membered heterocyclyl, C₆₋₂₀ aryl and 5-20 membered heteroaryl;
R_{d}, Rₑ and R_{f} are each the same or different, and are each independently selected from CN, halogen, OH, NH₂, oxo, S(O)₂CH₃, C₁₋₄₀ alkyl, C₁₋₄₀ haloalkyl, C₁₋₄₀ alkoxy and C₁₋₄₀ haloalkoxy.

2. The compound and the racemate, the stereoisomer, the tautomer, the isotopically labeled compound, the solvate, the polymorph and the nitrogen oxide thereof or the pharmaceutically acceptable salts thereof according to claim 1, wherein,
Y₁ and Y₂ are independently CR₄ or N;
X and W are independently selected from CR₅R₅, O and NR₇;
R₁ is selected from H, hydroxy, halogen, cyano, nitro, NR₁₁R₁₂, C(O)NR₁₁R₁₂, C(=S)NR₁₁R₁₂, S(O)₂NR₁₁R₁₂, C(=NR₁₃)NR₁₁R₁₂, NHC(O)NR₁₁R₁₂, P(O)₂NR₁₁R₁₂, P(O)R₁₃NR₁₁R₁₂, C(O)R₁₄, NHC(O)R₁₄, C(O)OR₁₅, and the following groups unsubstituted or optionally substituted with one, two or more Rₐ: C₁₋₁₀ alkyl, C₂₋₁₀ alkenyl, C₂₋₁₀ alkynyl, C₁₋₁₀ alkoxy, C₃₋₁₀ cycloalkyl, 3-10 membered heterocyclyl, C₆₋₁₄ aryl and 5-14 membered heteroaryl;
R₂ is selected from H and the following groups unsubstituted or optionally substituted with one, two or more R_{b}: C₁₋₁₀ alkyl, C₂₋₁₀ alkenyl, C₂₋₁₀ alkynyl, C₁₋₁₀ alkoxy, C₃₋₁₀ cycloalkyl, 3-10 membered heterocyclyl, C₆₋₁₄ aryl, C₆₋₁₄ aryl-C₁₋₁₀ alkyl, 5-14 membered heteroaryl and 5-14 membered heteroaryl-Ci-io alkyl; or
R₂, together with X, forms the following groups unsubstituted or optionally substituted with one, two or more R_{b}: NHC(O)NR₁₁R₁₂, C₃₋₁₀ cycloalkyl, 3-10 membered heterocyclyl, C₆₋₁₄ aryl and 5-14 membered heteroaryl;
R₃ is selected from H and the following groups unsubstituted or optionally substituted with one, two or more R_{c}: NR₁₁R₁₂, C(O)NR₁₁R₁₂, C(O)R₁₄, C₁₋₁₀ alkyl, C₂₋₁₀ alkenyl, C₂₋₁₀ alkynyl, C₁₋₁₀ alkoxy, C₃₋₁₀ cycloalkyl, 3-10 membered heterocyclyl, C₆₋₁₄ aryl and 5-14 membered heteroaryl; or
R₃, together with W, forms the following groups unsubstituted or optionally substituted with one, two or more R_{c}: NHC(O)NR₁₁R₁₂, C₃₋₁₀ cycloalkyl, 3-10 membered heterocyclyl, C₆₋₁₄ aryl and 5-14 membered heteroaryl;
R₄ is selected from H, hydroxy, halogen, cyano, nitro, amino, and the following groups unsubstituted or optionally substituted with one, two or more R_{d}: C₁₋₁₀ alkyl, C₂₋₁₀ alkenyl, C₂₋₁₀ alkynyl and C₁₋₁₀ alkoxy;
R₅ and R₆ are each independently selected from H, hydroxy, halogen, cyano, and the following groups unsubstituted or optionally substituted with one, two or more R_{d}: C₁₋₁₀ alkyl, C₂₋₁₀ alkenyl, C₂₋₁₀ alkynyl and C₁₋₁₀ alkoxy, with the proviso that R₅ and R₆ are not both hydroxy, cyano or C₁₋₁₀ alkoxy simultaneously; or, R₅ and R₆, together with the carbon atom attached thereto, form carbonyl, i.e., CR₅R₆ is C(=O);
R₇ is selected from H and the following groups unsubstituted or optionally substituted with one, two or more Rₑ: C₁₋₁₀ alkyl, C₂₋₁₀ alkenyl, C₂₋₁₀ alkynyl, C₁₋₁₀ alkoxy, C₃₋₁₀ cycloalkyl, 3-10 membered heterocyclyl, C₆₋₁₄ aryl and 5-14 membered heteroaryl;
R₁₁ and R₁₂ are each independently selected from H and the following groups unsubstituted or optionally substituted with one, two or more Rₑ: C(O)R₁₄, C₁₋₁₀ alkyl, C₂₋₁₀ alkenyl, C₂₋₁₀ alkynyl, C₁₋₁₀ alkoxy, C₃₋₁₀ cycloalkyl, 3-10 membered heterocyclyl, C₆₋₁₄ aryl and 5-14 membered heteroaryl; or
R₁₁ and R₁₂, together with the nitrogen atom attached thereto, form the following groups unsubstituted or optionally substituted with one, two or more Rₑ: 3-10 membered heterocyclyl and 5-14 membered heteroaryl;
R₁₃ is selected from H, hydroxy, cyano, and the following groups unsubstituted or optionally substituted with one, two or more Rₑ: C₁₋₁₀ alkyl and C₁₋₁₀ alkoxy;
R₁₄ is selected from H, hydroxy, halogen, and the following groups unsubstituted or optionally substituted with one, two or more R_{f}: C₁₋₁₀ alkyl, C₂₋₁₀ alkenyl, C₂₋₁₀ alkynyl, C₁₋₁₀ alkoxy, C₃₋₁₀ cycloalkyl, 3-10 membered heterocyclyl, C₆₋₁₄ aryl and 5-14 membered heteroaryl;
R₁₅ is selected from H and the following groups unsubstituted or optionally substituted with one, two or more R_{f}: C₁₋₁₀ alkyl, C₂₋₁₀ alkenyl, C₂₋₁₀ alkynyl, C₃₋₁₀ cycloalkyl, 3-10 membered heterocyclyl, C₆₋₁₄ aryl and 5-14 membered heteroaryl;
each Rₐ is independently selected from CN, halogen, OH, NH₂, oxo, and the following groups unsubstituted or optionally substituted with one, two or more R_{f}: C₁₋₁₀ alkyl and C₁₋₁₀ alkoxy;
each R_{b} is independently selected from CN, halogen, OH, NH₂, COOH, NO₂, oxo, S(O)₂CH₃, C(O)NHCH₂CH₃, and the following groups unsubstituted or optionally substituted with one, two or more R_{f}: C₁₋₁₀ alkyl, C₁₋₁₀ alkoxy, C₃₋₁₀ cycloalkyl, 3-10 membered heterocyclyl, C₆₋₁₄ aryl and 5-14 membered heteroaryl;
each R_{c} is independently selected from CN, halogen, OH, NH₂, COOH, NO₂, C(O)NH₂, C(O)NHOH, C(O)N(OH)CH₃, oxo, C(O)CH₂COOH, C(O)CH₂CN, C(O)CH₂Cl, C(O)CH₂F, C(O)CH₂Br, and the following groups unsubstituted or optionally substituted with one, two or more R_{f}: C₁₋₁₀ alkyl, C₁₋₁₀ alkoxy, C₃₋₁₀ cycloalkyl, 3-10 membered heterocyclyl, C₆₋₁₄ aryl and 5-14 membered heteroaryl;
R_{d}, Rₑ and R_{f} are each the same or different, and are each independently selected from CN, halogen, OH, NH₂, oxo, S(O)₂CH₃, C₁₋₁₀ alkyl, C₁₋₁₀ haloalkyl, C₁₋₁₀ alkoxy and C₁₋₁₀ haloalkoxy.

3. The compound and the racemate, the stereoisomer, the tautomer, the isotopically labeled compound, the solvate, the polymorph and the nitrogen oxide thereof or the pharmaceutically acceptable salts thereof according to claim 1 or 2, wherein,
Y₁ and Y₂ are independently CR₄ or N;
X and W are independently selected from CR₅R₆, O and NR₇;
R₁ is selected from H, hydroxy, halogen, cyano, nitro, NR₁₁R₁₂, C(O)NR₁₁R₁₂, C(=S)NR₁₁R₁₂, S(O)₂NR₁₁R₁₂, C(=NR₁₃)NR₁₁R₁₂, NHC(O)NR₁₁R₁₂, P(O)₂NR₁₁R₁₂, P(O)R₁₃NR₁₁R₁₂, C(O)R₁₄, NHC(O)R₁₄, C(O)OR₁₅, and the following groups unsubstituted or optionally substituted with one, two or more Rₐ: C₁₋₆ alkyl, C₂₋₆ alkenyl, C₂₋₆ alkynyl, C₁₋₆ alkoxy, C₃₋₁₀ cycloalkyl, 3-10 membered heterocyclyl, C₆₋₁₄ aryl and 5-14 membered heteroaryl;
R₂ is selected from H and the following groups unsubstituted or optionally substituted with one, two or more R_{b}: C₁₋₆ alkyl, C₂₋₆ alkenyl, C₂₋₆ alkynyl, C₁₋₆ alkoxy, C₃₋₁₀ cycloalkyl, 3-10 membered heterocyclyl, C₆₋₁₄ aryl, C₆₋₁₄ aryl-C₁₋₆ alkyl, 5-14 membered heteroaryl and 5-14 membered heteroaryl-C₁₋₆ alkyl; or
R₂, together with X, forms the following groups unsubstituted or optionally substituted with one, two or more R_{b}: NHC(O)NR₁₁R₁₂, C₃₋₁₀ cycloalkyl, 3-10 membered heterocyclyl, C₆₋₁₄ aryl and 5-14 membered heteroaryl;
R₃ is selected from H and the following groups unsubstituted or optionally substituted with one, two or more R_{c}: NR₁₁R₁₂, C(O)NR₁₁R₁₂, C(O)R₁₄, C₁₋₆ alkyl, C₂₋₆ alkenyl, C₂₋₆ alkynyl, C₁₋₆ alkoxy, C₃₋₁₀ cycloalkyl, 3-10 membered heterocyclyl, C₆₋₁₄ aryl and 5-14 membered heteroaryl; or
R₃, together with W, forms the following groups unsubstituted or optionally substituted with one, two or more R_{c}: NHC(O)NR₁₁R₁₂, C₃₋₁₀ cycloalkyl, 3-10 membered heterocyclyl, C₆₋₁₄ aryl and 5-14 membered heteroaryl;
R₄ is selected from H, hydroxy, halogen, cyano, nitro, amino, and the following groups unsubstituted or optionally substituted with one, two or more R_{d}: C₁₋₆ alkyl, C₂₋₆ alkenyl, C₂₋₆ alkynyl and C₁₋₆ alkoxy;
R₅ and R₆ are each independently selected from H, hydroxy, halogen, cyano, and the following groups unsubstituted or optionally substituted with one, two or more R_{d}: C₁₋₆ alkyl, C₂₋₆ alkenyl, C₂₋₆ alkynyl and C₁₋₆ alkoxy, with the proviso that R₅ and R₆ are not both hydroxy, cyano or C₁₋₆ alkoxy simultaneously; or, R₅ and R₆, together with the carbon atom attached thereto, form carbonyl, i.e., CR₅R₆ is C(=O);
R₇ is selected from H and the following groups unsubstituted or optionally substituted with one, two or more Rₑ: C₁₋₆ alkyl, C₂₋₆ alkenyl, C₂₋₆ alkynyl, C₁₋₆ alkoxy, C₃₋₁₀ cycloalkyl, 3-10 membered heterocyclyl, C₆₋₁₄ aryl and 5-14 membered heteroaryl;
R₁₁ and R₁₂ are each independently selected from H and the following groups unsubstituted or optionally substituted with one, two or more Rₑ: C(O)R₁₄, C₁₋₆ alkyl, C₂₋₆ alkenyl, C₂₋₆ alkynyl, C₁₋₆ alkoxy, C₃₋₁₀ cycloalkyl, 3-10 membered heterocyclyl, C₆₋₁₄ aryl and 5-14 membered heteroaryl; or
R₁₁ and R₁₂, together with the nitrogen atom attached thereto, form the following groups unsubstituted or optionally substituted with one, two or more Rₑ: 3-10 membered heterocyclyl and 5-14 membered heteroaryl;
R₁₃ is selected from H, hydroxy, cyano, and the following groups unsubstituted or optionally substituted with one, two or more Rₑ: C₁₋₆ alkyl and C₁₋₆ alkoxy;
R₁₄ is selected from H, hydroxy, halogen, and the following groups unsubstituted or optionally substituted with one, two or more R_{f}: C₁₋₆ alkyl, C₂₋₆ alkenyl, C₂₋₆ alkynyl, C₁₋₆ alkoxy, C₃₋₁₀ cycloalkyl, 3-10 membered heterocyclyl, C₆₋₁₄ aryl and 5-14 membered heteroaryl;
R₁₅ is selected from H and the following groups unsubstituted or optionally substituted with one, two or more R_{f}: C₁₋₆ alkyl, C₂₋₆ alkenyl, C₂₋₆ alkynyl, C₃₋₁₀ cycloalkyl, 3-10 membered heterocyclyl, C₆₋₁₄ aryl and 5-14 membered heteroaryl;
each Rₐ is independently selected from CN, halogen, OH, NH₂, oxo, and the following groups unsubstituted or optionally substituted with one, two or more R_{f}: C₁₋₆ alkyl and C₁₋₆ alkoxy;
each R_{b} is independently selected from CN, halogen, OH, NH₂, COOH, NO₂, oxo, S(O)₂CH₃, C(O)NHCH₂CH₃, and the following groups unsubstituted or optionally substituted with one, two or more R_{f}: C₁₋₆ alkyl, C₁₋₆ alkoxy, C₃₋₁₀ cycloalkyl, 3-10 membered heterocyclyl, C₆₋₁₄ aryl and 5-14 membered heteroaryl;
each R_{c} is independently selected from CN, halogen, OH, NH₂, COOH, NO₂, C(O)NH₂, C(O)NHOH, C(O)N(OH)CH₃, oxo, C(O)CH₂COOH, C(O)CH₂CN, C(O)CH₂Cl, C(O)CH₂F, C(O)CH₂Br, and the following groups unsubstituted or optionally substituted with one, two or more R_{f}: C₁₋₆ alkyl, C₁₋₆ alkoxy, C₃₋₁₀ cycloalkyl, 3-10 membered heterocyclyl, C₆₋₁₄ aryl and 5-14 membered heteroaryl;
R_{d}, Rₑ and R_{f} are each the same or different, and are each independently selected from CN, halogen, OH, NH₂, oxo, S(O)₂CH₃, C₁₋₆ alkyl, C₁₋₆ haloalkyl, C₁₋₆ alkoxy and C₁₋₆ haloalkoxy.

4. The compound and the racemate, the stereoisomer, the tautomer, the isotopically labeled compound, the solvate, the polymorph and the nitrogen oxide thereof or the pharmaceutically acceptable salts thereof according to any of claims 1-3, wherein,
Y₁ and Y₂ are independently CR₄ or N;
X and W are independently O or NR₇;
R₁ is selected from H, hydroxy, halogen, cyano, nitro, NR₁₁R₁₂, C(O)NR₁₁R₁₂, C(=S)NR₁₁R₁₂, S(O)₂NR₁₁R₁₂, C(=NR₁₃)NR₁₁R₁₂, NHC(O)NR₁₁R₁₂, P(O)₂NR₁₁R₁₂, P(O)R₁₃NR₁₁R₁₂, C(O)R₁₄, NHC(O)R₁₄, C(O)OR₁₅, and the following groups unsubstituted or optionally substituted with one, two or more Rₐ: C₁₋₆ alkyl, C₂₋₆ alkenyl, C₂₋₆ alkynyl, C₁₋₆ alkoxy, C₃₋₆ cycloalkyl, 3-7 membered heterocyclyl and 5-6 membered heteroaryl;
R₂ is selected from H and the following groups unsubstituted or optionally substituted with one, two or more R_{b}: C₁₋₆ alkyl, C₂₋₆ alkenyl, C₂₋₆ alkynyl, C₁₋₆ alkoxy, C₃₋₆ cycloalkyl, 3-7 membered heterocyclyl, phenyl, benzyl, 5-6 membered heteroaryl and 5-6 membered heteroaryl-C₁₋₆ alkyl; or
R₂, together with X, forms the following groups unsubstituted or optionally substituted with one, two or more R_{b}: NHC(O)NR₁₁R₁₂, 3-7 membered heterocyclyl and 5-6 membered heteroaryl;
R₃ is selected from H and the following groups unsubstituted or optionally substituted with one, two or more R_{c}: NR₁₁R₁₂, C(O)NR₁₁R₁₂, C(O)R₁₄, C₁₋₆ alkyl, C₃₋₆ cycloalkyl, 3-7 membered heterocyclyl, phenyl and 5-6 membered heteroaryl; or
R₃, together with W, forms the following groups unsubstituted or optionally substituted with one, two or more R_{c}: NHC(O)NR₁₁R₁₂, 3-7 membered heterocyclyl and 5-6 membered heteroaryl;
R₄ is selected from H, hydroxy and halogen;
R₇ is H;
R₁₁ and R₁₂ are each independently selected from H and the following groups unsubstituted or optionally substituted with one, two or more Rₑ: C₁₋₆ alkyl, C₂₋₆ alkenyl and C₂₋₆ alkynyl; or
R₁₁ and R₁₂, together with the nitrogen atom attached thereto, form the following groups unsubstituted or optionally substituted with one, two or more Rₑ: 3-7 membered heterocyclyl and 5-6 membered heteroaryl;
R₁₃ is selected from H, cyano, methyl and hydroxy;
R₁₄ is selected from H, hydroxy, halogen, and the following groups unsubstituted or optionally substituted with one, two or more R_{f}: C₁₋₆ alkyl, C₂₋₆ alkenyl, C₂₋₆ alkynyl, C₁₋₆ alkoxy and C₃₋₆ cycloalkyl;
R₁₅ is selected from H and C₁₋₆ alkyl unsubstituted or optionally substituted with one, two or more R_{f};
each Rₐ is independently selected from CN, halogen, OH, NH₂, oxo, and the following groups unsubstituted or optionally substituted with one, two or more R_{f}: C₁₋₆ alkyl and C₁₋₆ alkoxy;
each R_{b} is independently selected from CN, halogen, OH, NH₂, COOH, NO₂, oxo, S(O)₂CH₃, C(O)NHCH₂CH₃, and the following groups unsubstituted or optionally substituted with one, two or more R_{f}: C₁₋₆ alkyl, C₁₋₆ alkoxy, C₃₋₆ cycloalkyl, 3-7 membered heterocyclyl, phenyl and 5-6 membered heteroaryl;
each R_{c} is independently selected from CN, halogen, OH, NH₂, COOH, NO₂, C(O)NH₂, C(O)NHOH, C(O)N(OH)CH₃, oxo, C(O)CH₂COOH, C(O)CH₂CN, C(O)CH₂Cl, C(O)CH₂F, C(O)CH₂Br, and the following groups unsubstituted or optionally substituted with one, two or more R_{f}: C₁₋₆ alkyl, C₁₋₆ alkoxy, 3-7 membered heterocyclyl and 5-6 membered heteroaryl;
Rₑ and R_{f} are each the same or different, and are each independently selected from CN, halogen, OH, NH₂, oxo, S(O)₂CH₃, C₁₋₆ alkyl, C₁₋₆ haloalkyl, C₁₋₆ alkoxy and C₁₋₆ haloalkoxy.

5. The compound and the racemate, the stereoisomer, the tautomer, the isotopically labeled compound, the solvate, the polymorph and the nitrogen oxide thereof or the pharmaceutically acceptable salts thereof according to any of claims 1-4, wherein the compound of formula (I) is a compound of formula (II): 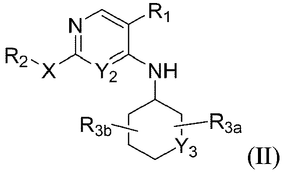 wherein,
Y₂, X, R₁ and R₂ are defined as in any of claims 1-4;
Y₃ is CR₈R₉ or NR₁₀;
R₈ and R₉ are the same or different, and are each independently selected from H, hydroxy, halogen, cyano, and the following groups unsubstituted or optionally substituted with one, two or more hydroxy or halogen: C₁₋₆ alkyl, C₁₋₆ alkoxy, C₁₋₆ alkenyl and C₁₋₆ alkynyl; or R₈ and R₉, together with the carbon atom attached thereto, form carbonyl, i.e., CR₈R₉ is C(=O);
R₁₀ is selected from H, C(O)NH₂, C(O)CH₂CN, C(O)CH₂COOH, and the following groups unsubstituted or optionally substituted with one, two or more hydroxy or halogen: C₁₋₆ alkyl, C₁₋₆ alkoxy, C₁₋₆ alkenyl and C₁₋₆ alkynyl;
R₃ₐ and R_{3b} are the same or different, and are each independently selected from H, hydroxy, halogen, cyano, oxo, and the following groups unsubstituted or optionally substituted with one, two or more hydroxy or halogen: C₁₋₆ alkyl, C₁₋₆ alkoxy, C₁₋₆ alkenyl and C₁₋₆ alkynyl.

6. The compound and the racemate, the stereoisomer, the tautomer, the isotopically labeled compound, the solvate, the polymorph and the nitrogen oxide thereof or the pharmaceutically acceptable salts thereof according to any of claims 1-5, wherein the compound of formula (I) is selected from a compound of formula (II-1): 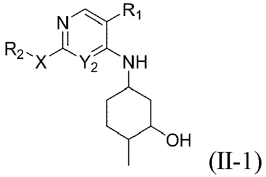 wherein,
Y₂, X, R₁ and R₂ are defined as in any of claims 1-5.

7. A method for preparing the compound and the racemate, the stereoisomer, the tautomer, the isotopically labeled compound, the solvate, the polymorph and the nitrogen oxide thereof or the pharmaceutically acceptable salts thereof according to any of claims 1-6, being one of the following schemes 1-3: 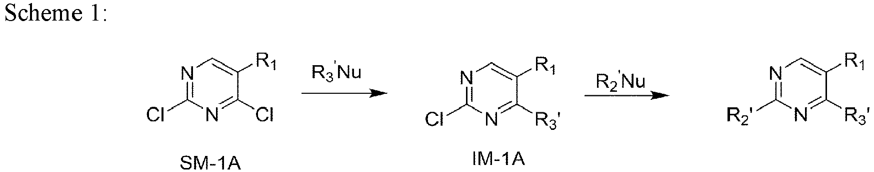 in this scheme, R₂' is R₂-X, R₃' is R₃-W, Nu is a nucleophilic group, and R₁, R₂, R₃, X, and W are defined as in any of claims 1-6;
scheme 1 comprises the following steps 1a and 1b:
(1a) subjecting SM-1A to a reaction with a nucleophilic reagent containing R₃' in the presence of a Lewis base to give IM-1A; and
(1b) subjecting IM-1A to a reaction with a nucleophilic reagent containing R₂ in the presence of a Lewis base to give a corresponding product or intermediate; 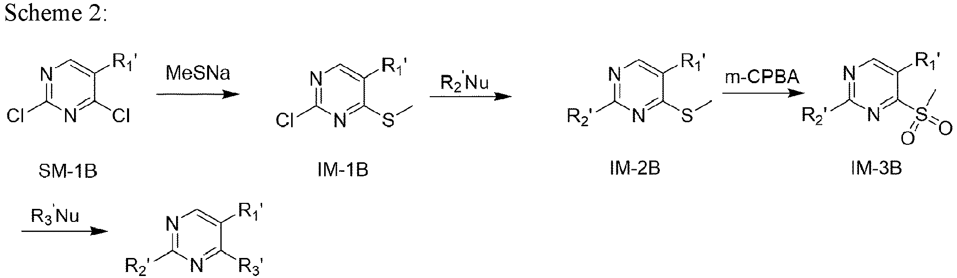 in this scheme, R₁' is R₁ or a group that can derive into R₁, R₂' is R₂-X, R₃' is R₃-W, Nu is a nucleophilic group, and R₁, R₂, R₃, X and W are defined as in any of claims 1-6;
scheme 2 comprises the following steps 2a-2d:
(2a) subjecting SM-1B to a reaction with sodium thiomethoxide to give IM-1B, and optionally, carrying out derivatization reaction on R₁' in the IM-1B before proceeding to subsequent steps;
(2b) subjecting IM-1B to a reaction with a nucleophilic reagent containing R₂ in the presence of a Lewis base to give IM-2B;
(2c) subjecting IM-2B to a reaction with *m*-chloroperoxybenzoic acid to give IM-3B; and
(2d) subjecting IM-3B to a reaction with a nucleophilic reagent containing R₃' in the presence of a Lewis base to give a corresponding product or intermediate, and optionally, further carrying out a derivatization reaction; 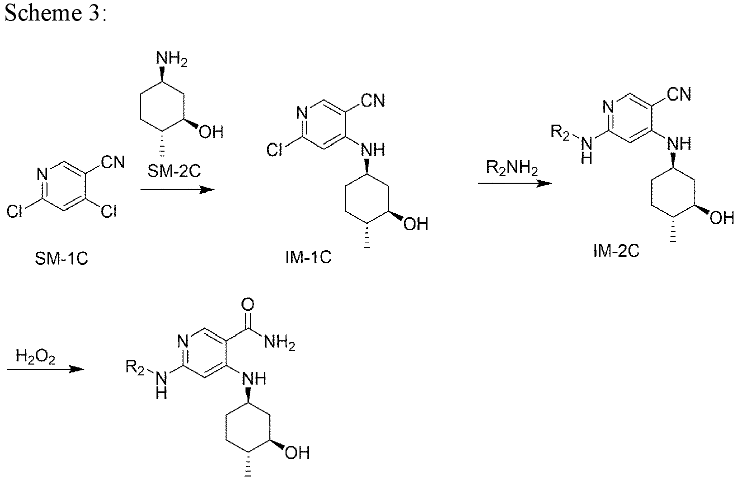 in this scheme, R₂ is defined as in any of claims 1-6;
scheme 3 comprises the following steps 3a-3c:
(3a) subjecting SM-1C to a reaction with SM-2C in the presence of a Lewis base to give IM-1C;
(3b) subjecting IM-1C to a reaction with R₂NH₂ in the presence of a Lewis base to give a corresponding product or intermediate; and
(3c) subjecting IM-2C to a reaction with H₂O₂ in the presence of a Lewis base to give a corresponding product.

8. A pharmaceutical composition, comprising a therapeutically effective amount of the compound and the racemate, the stereoisomer, the tautomer, the isotopically labeled compound, the solvate, the polymorph and the nitrogen oxide thereof or the pharmaceutically acceptable salts thereof according to any of claims 1-6 and one or more pharmaceutically acceptable auxiliary materials, wherein the pharmaceutically acceptable auxiliary material can be one or more selected from a disintegrant, a glidant, a lubricant, a diluent, a filler, an adhesive and a colorant.

9. Use of the compound and the racemate, the stereoisomer, the tautomer, the isotopically labeled compound, the solvate, the polymorph and the nitrogen oxide thereof or the pharmaceutically acceptable salts thereof according to any of claims 1-6 in preparing a kinase inhibitor, in particular an inhibitor of kinases JNK1 and/or JNK2.

10. Use of the compound and the racemate, the stereoisomer, the tautomer, the isotopically labeled compound, the solvate, the polymorph or the nitrogen oxide thereof or the pharmaceutically acceptable salts thereof according to any of claims 1-6 in preparing a medicament for preventing and/or treating following diseases or disorders: rheumatoid arthritis; rheumatoid spondylitis; osteoarthritis; asthma; bronchitis; allergic rhinitis; chronic obstructive pulmonary disease; cystic fibrosis; inflammatory bowel disease; irritable bowel syndrome; mucous colitis; ulcerative colitis; Crohn's disease; Huntington's disease; hepatitis; pancreatitis; nephritis; multiple sclerosis; lupus erythematosus; type II diabetes; obesity; atherosclerosis; post-angioplasty restenosis; left ventricular hypertrophy; myocardial infarction; stroke; ischemic injury of the heart, lung, intestine, kidney, liver, pancreas, spleen and brain; acute or chronic organ transplant rejection; preservation of organs for transplantation; organ failure or loss of limb (e.g., including but not limited to those resulting from ischemia-reperfusion injury, trauma, gross bodily injury, car accident, crush injury or transplantation failure); graft versus host disease; endotoxic shock; multiple organ failure; psoriasis; burns caused by exposure to fire, chemicals or radiation; eczema; dermatitis; skin grafting; ischemia; ischemic disorders associated with surgery or traumatic injury (e.g., vehicular accident, gunshot wound or limb crush); epilepsy; Alzheimer's disease; Parkinson's disease; immune response to bacterial or viral infection; cachexia; angiogenic and proliferative diseases; solid tumor; and cancers of various tissues, such as colon, rectum, prostate, liver, lung, bronchus, pancreas, brain, head, neck, stomach, skin, kidney, cervix, blood, larynx, esophagus, mouth, pharynx, bladder, ovary or uterus,
in particular, the medicament is used for preventing and/or treating the following diseases or disorders: hepatic and pulmonary fibrosis diseases, such as non-alcoholic steatohepatitis, steatosis, cirrhosis, primary sclerosing cholangitis, primary biliary cirrhosis, hepatitis, hepatocellular carcinoma, hepatic fibrosis accompanied by long-term or repeated alcohol intake, accompanied by an infection, accompanied by liver transplantation or accompanied by drug-induced liver injury, and idiopathic pulmonary fibrosis; systemic sclerosis, scleroderma, chronic allograft nephropathy, antibody-mediated rejection, or lupus, such as lupus erythematosus or systemic lupus.
